# EUROPEAN PATENT APPLICATION

(11) **EP 4 527 839 A1**
(43) Date of publication of application: **26.03.2025**
(21) Application number: 23807529.5
(22) Date of filing: 11.05.2023
(51) Int. Cl.: C07D 519/00, C07D 207/452, C07D 307/42, C08G 59/14, C08G 59/62

(54) **PHENOLIC HYDROXY GROUP-CONTAINING COMPOUND, CURABLE RESIN COMPOSITION, CURED PRODUCT, AND LAMINATE**

(30) Priority: 17.05.2022 JP 2022080769
(71) Applicant: DIC Corporation, Tokyo 174-8520 (JP)
(72) Inventor: OTSU, Masato, Sakura-shi, Chiba 285-8668 (JP); ARITA, Kazuo, Sakura-shi, Chiba 285-8668 (JP)
(74) Representative: TBK
(86) International application number: PCT/JP2023/017676
(87) International publication number: WO 2023/223925

(57) **Abstract**

There is provided a compound which, while being a curable resin, can easily exhibit repairability, easy disassembly, and remoldability when being in the form of a cured product, a curable resin composition obtained using the same; and a cured product thereof. Specifically, there is provided a phenolic hydroxyl group-containing compound in which a structural unit A having one or more phenolic hydroxyl groups and a structural unit B different from the A are linked in A-B-A, and the structural unit A and the structural unit B are bonded by a reversible bond by a Diels-Alder reaction between a furan structure and a maleimide structure.

## Description

### TECHNICAL FIELD

The present invention relates to a phenolic hydroxyl group-containing compound having a specific structure, a curable resin composition containing the same, a cured product, and a laminate containing a layer consisting of the cured product.

### BACKGROUND ART

Cured products obtained from epoxy resins have excellent heat resistance, mechanical strength, electrical properties, adhesive properties, and the like, and are essential materials in various fields such as electrical and electronics, paints, and adhesives.

On the other hand, cured products using thermosetting resins such as epoxy resins have low long-term reliability. For example, when a cured product of an epoxy resin deteriorates due to oxidation, cracks may occur.

In addition, the cured product obtained by curing a thermosetting resin such as an epoxy resin is not soluble in solvents (insoluble) and do not dissolve even at high temperatures (insoluble), thus not recyclable or reusable, and the cured product becomes waste after use, making it a challenge to reduce waste and reduce the burden on the environment.

Therefore, a cured product using an epoxy resin or the like is required to solve the problems of long life and waste reduction, and it is considered effective to add easy disassembly, and repairability and remoldability to the cured product to solve these problems.

Against this background, a method has been disclosed in which a compound having thermal degradability is blended in advance with a reactive adhesive component, and then, after use, the adhesive strength is reduced by a certain amount of heating to enable disassembly (see, for example, Patent Document 1).

Further, a method is disclosed in which even if cracks or delamination occurs in an encapsulant using an epoxy resin or the like, use of microcapsule particles encapsulating a first thermosetting resin and a second thermosetting resin precursor material imparts self-repairablity to the encapsulant (see, for example, Patent Document 2).

In addition to the above, there has been much research on the use of reversible bonds such as dynamic covalent bonding and supramolecular bonding in a cured product to impart repairability and remoldability.

### CITATION LIST

### PATENT LITERATURE

Patent Document 1:JP-A-2013-256557
Patent Document 2:JP-A-2017-041496

### SUMMARY OF INVENTION

### TECHNICAL PROBLEM

In the technology provided in Patent Document 1, the adhesive is disposed of after disassembly, and although the base material to be adhered to is recyclable, the overall recyclability of the adhesive is insufficient, which is a problem. Further, the technology disclosed in Patent Document 2 has a certain degree of self-repairing property, but is not a solution from the viewpoint of reuse, and the problem of waste when the adhesive is no longer needed remains. In addition, since it is necessary to ensure the molecular mobility of the raw materials to be used involved in the reversible bond, there is a problem that the raw materials to be used are limited to gel-like substances with poor mechanical strength, and improvements are currently required in all of these areas. Therefore, the object of the present invention is to provide a compound that can easily achieve easy disassembly, repairability, and remoldability in a cured product while being a curable resin, and a curable resin composition using the compound, and a cured product made from the compound.

### SOLUTION TO PROBLEM

As a result of their diligent study, the inventors found that the above problems can be solved by using a phenolic hydroxyl group-containing compound having a specific structure and using the compound as a curable resin composition, and completed the invention.

That is, the present invention includes the following aspects.
[1] A phenolic hydroxyl group-containing compound in which a structural unit A having one or more phenolic hydroxyl groups and a structural unit B different from the structural unit A are linked in A-B-A, in which
   the structural unit A and the structural unit B are bonded by a reversible bond by a Diels-Alder reaction between a furan structure and a maleimide structure.
[2] The phenolic hydroxyl group-containing compound according to [1], in which
   the structural unit B has an alkylene chain or an alkylene ether chain.
[3] The phenolic hydroxyl group-containing compound according to [2], in which
   the alkylene chain has 4 to 16 carbon atoms.
[4] The hydroxyl group-containing compound according to any one of [1] to [4], in which
   the structural unit B further has a reversible bond by the Diels-Alder reaction between the furan structure and the maleimide structure.
[5] A phenolic hydroxyl group-containing compound represented by the following general formula: 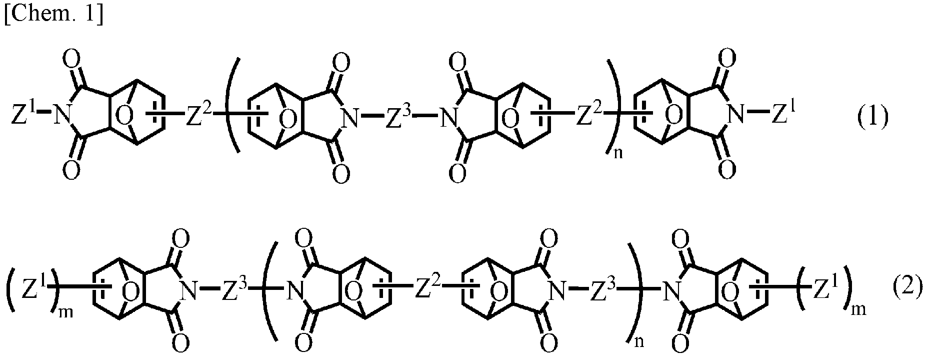 [the furan-derived structure in the formulas (1) and (2) may include a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group, as a substituent, in the formulas, m is an integer of 1 to 4, and n is an average value of repetition number of 0 to 10, Z¹ is any of the structures represented by the following formula (3), Z² is any of the structures represented by the following formula (4), Z³ is any of the structures represented by by the following formula (5), and each of the multiple structures present in one molecule may be identical or different from each other, 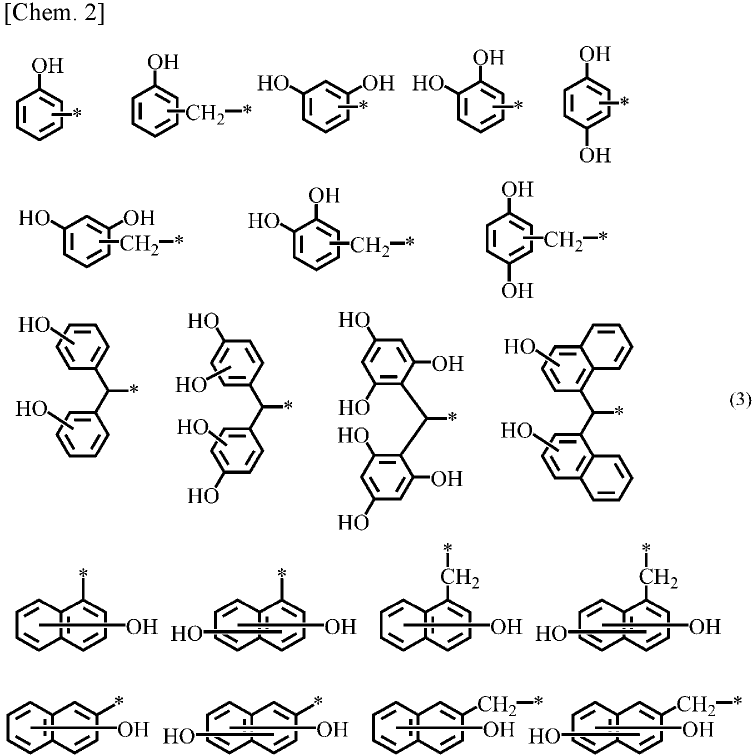 [the aromatic ring in the formula (3) may be substituted or unsubstituted, and * denotes a binding point, the hydroxyl group on the naphthalene ring in the formula indicates that the hydroxyl group may be bonded at any point], 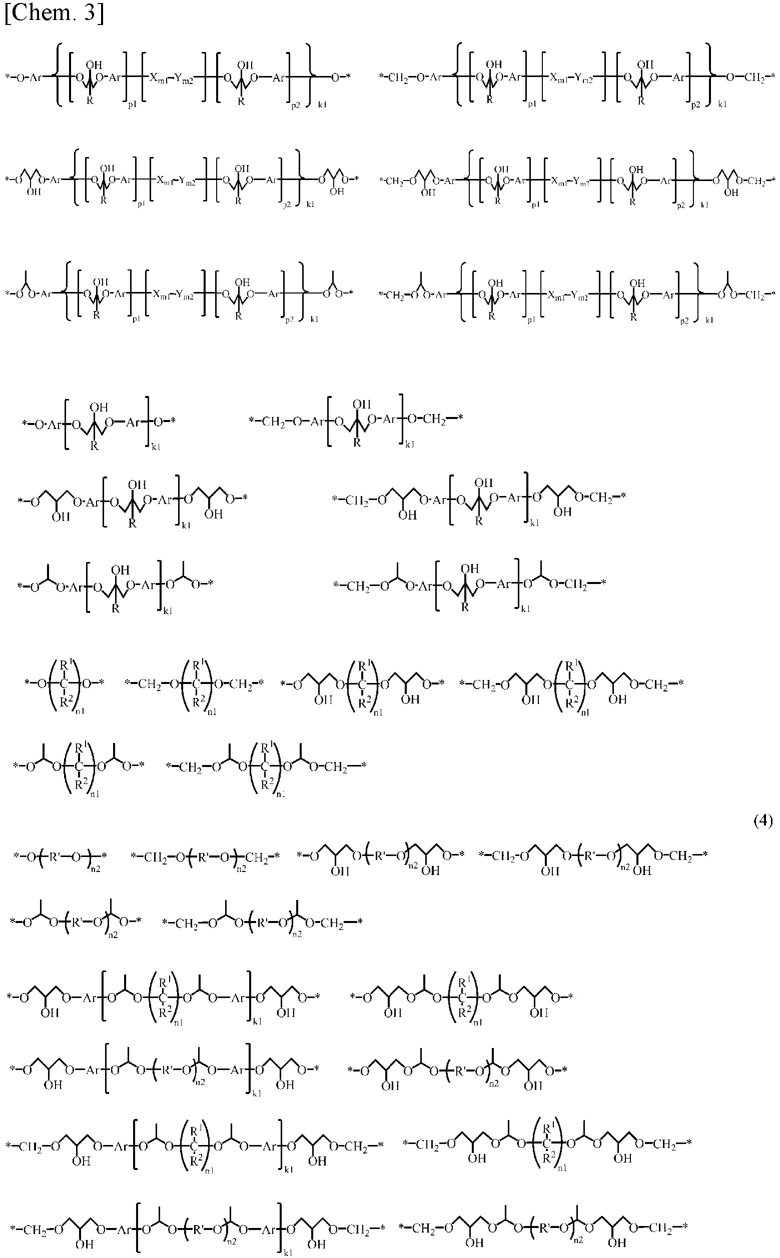 [in the formula (4),
   Ar's are each independently a structure containing an unsubstituted aromatic ring or an aromatic ring having a substituent,
   R¹ and R² are each independently a hydrogen atom, a methyl group or an ethyl group,
   R is a hydrogen atom or a methyl group,
   R' is a divalent hydrocarbon group having 2 to 12 carbon atoms,
   n1 is an integer of 2 to 16, and n2 is an average value of repeating units of 2 to 30,
   k1 is an average value of repetition number, in a range of 0.5 to 10,
   p1 and p2 are each independently 0 to 5, and
   X is a structural unit represented by the following formula (4-1), and Y is a structural unit represented by the following formula (4-2), 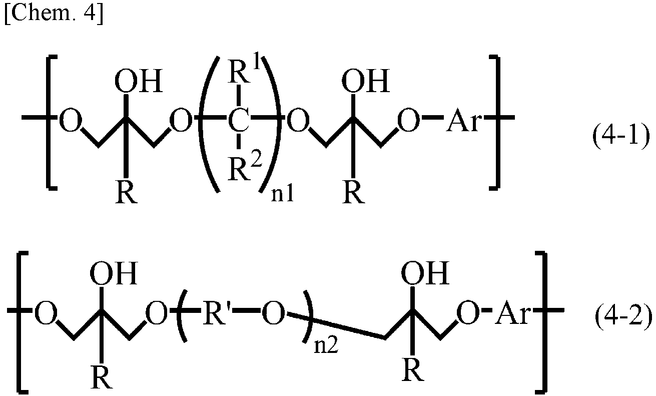
   [in the formulas (4-1) and (4-2), Ar, R, R¹, R², R', n1, and n2 are the same as above],
   m1 and m2 are an average value of repetition number, each independently 0 to 25, and m1 + m2 ≥ 1,
   provided that a bond between the structural unit X represented by the formula (4-1) and the structural unit Y represented by the formula (4-2) may be random or blocked, and total numbers of respective structural units X and Y present in one molecule are m1 and m2, respectively], 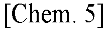 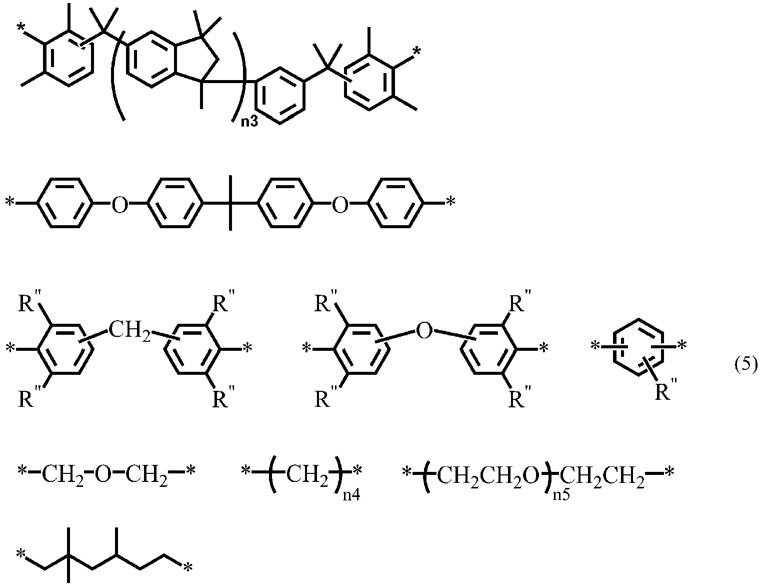
   [in the formula (5), n3 and n5 are an average value of repetition number and are 0.5 to 10, respectively,
   n4 is an integer of 1 to 16, and
   R"'s are each independently a hydrogen atom, a methyl group or an ethyl group].
[6] A curable resin composition including, as essential components:
   the phenolic hydroxyl group-containing compound according to any one of [1] to [5]; and
   a compound (I) having reactivity with a phenolic hydroxyl group-containing compound.
[7] The curable resin composition according to [6], in which
   a concentration of the reversible bond in the phenolic hydroxyl group-containing compound to a total mass of curable components in the curable resin composition is 0.10 mmol/g or more.
[8] The curable resin composition according to [6] or [7], in which
   the compound (I) having reactivity with the phenolic hydroxyl group-containing compound is an epoxy resin.
[9] The curable resin composition according to [8], further including:
   a curing agent for an epoxy resin other than the phenolic hydroxyl group-containing compound.
[10] The curable resin composition according to [8] or [9], in which
   the epoxy resin is represented by the following formula (6) and has an epoxy equivalent weight of 500 to 10000 g/eq: 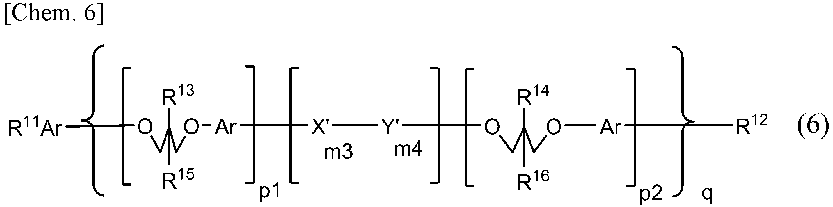
   [in the formula (6), Ar's are each independently a structure containing an unsubstituted aromatic ring or an aromatic ring having a substituent,
   X' is a structural unit represented by the following general formula (6-1), and Y' is a structural unit represented by the following general formula (6-2), 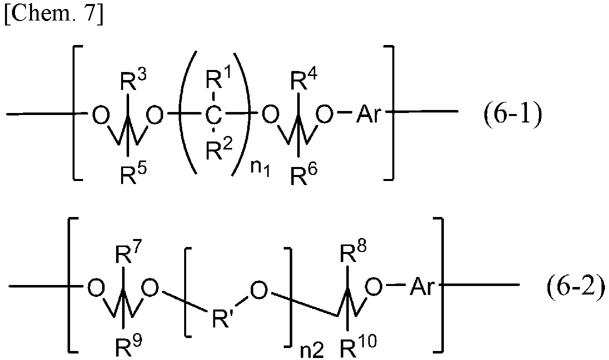
   [in the formulas (6-1) and (6-2), Ar is the same as above,
   R¹ and R² are each independently a hydrogen atom, a methyl group or an ethyl group,
   R' is a divalent hydrocarbon group having 2 to 12 carbon atoms,
   R³, R⁴, R⁷, and R⁸ are each independently a hydroxyl group, a glycidyl ether group, or a 2-methylglycidyl ether group,
   R⁵, R⁶, R⁹, and R¹⁰ are each independently a hydrogen atom or a methyl group,
   n1 is an integer of 4 to 16, and
   n2 is an average value of repeating units of 2 to 30],
   R¹¹ and R¹² are each independently a glycidyl ether group or a 2-methylglycidyl ether group,
   R¹³ and R¹⁴ are each independently a hydroxyl group, a glycidyl ether group or a 2-methylglycidyl ether group,
   R¹⁵ and R¹⁶ are hydrogen atoms or methyl groups,
   m3, m4, p1, p2, and q are an average value of repetition number,
   m3 and m4 are each independently 0 to 25 and m3 + m4 ≥ 1,
   p1 and p2 are each independently 0 to 5, and
   q is 0.5 to 5,
   provided that the bond between X' represented by the general formula (6-2) and Y' represented by the general formula (6-3) may be random or blocked, and total numbers of respective structural units X' and Y' present in one molecule are m3 and m4, respectively].
[11] The curable resin composition according to any one of [6] to [10], in which
   the curable resin composition is any of a self-repairing composition, an easily disassembled composition, or a composition for a remolding material.
[12] A cured product obtained by curing the curable resin composition according to any one of [6] to [10].
[13] A laminate containing:
   a base material; and
   a layer containing the cured product according to [12].
[14] A heat-resistant member containing:
   the cured product according to [12].
[15] An intermediate of a conjugated diene or a parent diene intermediate represented by the following general formulas (1)' and (2)': 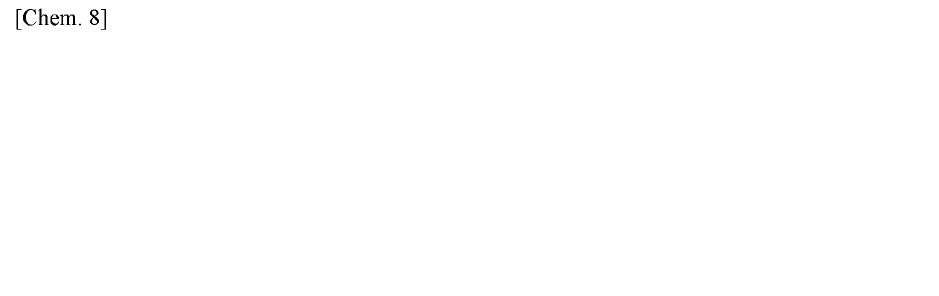 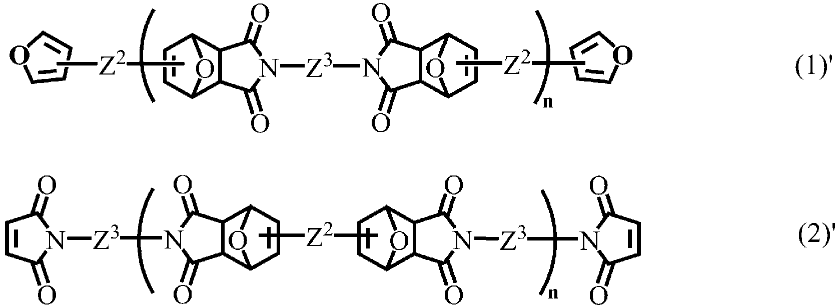 [in the formulas, n, Z², and Z³ are the same as above].
[16] A method for producing a phenolic hydroxyl group-containing compound, the method including:
   synthesizing a phenolic hydroxyl group-containing compound represented by the formulas (1) and (2) in situ during a process of curing thereof together with a compound (I) having reactivity with the phenolic hydroxyl group-containing compound, by using an intermediate of a conjugated diene or a parent diene intermediate represented by the general formulas (1)' and (2)'.
[17] A cured product obtained by curing and reacting the formula (1)', a maleimide compound having a phenolic hydroxyl group, and a compound (I) having reactivity with the phenolic hydroxyl group-containing compound as essential raw materials.
[18] A cured product obtained by curing and reacting the formula (2)', a furan compound having a phenolic hydroxyl group, and a compound (I) having reactivity with the phenolic hydroxyl group-containing compound as essential raw materials.

### ADVANTAGEOUS EFFECTS OF INVENTION

According to the present invention, a cured product made of a curable resin composition can be given easy disassembly, repairability and remoldability, which can contribute to extending the service life of the cured product itself and reducing waste.

### DESCRIPTION OF EMBODIMENTS

Hereinafter, embodiments of the present invention will be described in detail. It should be understood that the present invention is not limited to the following embodiments, and design changes, improvements, and the like are appropriately added based on ordinary knowledge of those skilled in the art without departing from the gist of the present invention.

The phenolic hydroxyl group-containing compound as an embodiment of the present invention is a phenolic hydroxyl group-containing compound in which a structural unit A having one or more phenolic hydroxyl groups and a structural unit B different from the A are linked in A-B-A, and the structural unit A and the structural unit B are bonded by a reversible bond by a Diels-Alder reaction between a furan structure and a maleimide structure.

With such a configuration, the phenolic hydroxyl group-containing compound is incorporated into the crosslinked structure by a curing reaction based on the phenolic hydroxyl group thereof. In addition, since the phenolic hydroxyl group is more reactive than an alcoholic hydroxyl group, the curing reaction proceeds in a shorter time. On the other hand, by being reversible even after becoming a cured product, the structural unit B in particular can be present apart from the crosslinked structure, and thus has high molecular mobility even in the cured product. For this reason, when the cured product is subjected to impact such that the cured product cracks or is pulverized, the reversible bonds are likely to break, resulting in easy disassembly. On the other hand, the reversible bonds can be reversibly remolded even in low temperature regions, including room temperature, and can exhibit functions such as repairability and remoldability. The structural unit B is present away from the crosslinked structure and thus exhibits particularly high molecular mobility, indicating low-temperature repairability and low-temperature remolding. For example, even when the cured product obtained by using the phenolic hydroxyl group-containing compound of the present invention is pulverized, it is possible to easily repair the cured product based on the reversible bond by placing the cured product at low temperatures, including room temperature, or in a warm or heated state, and it is also possible to remold the cured product after having the cured product pulverized.

The method using furan having a reactive functional group on the ring and maleimide having a reactive functional group is preferable for introducing the furan-type addition-type structure by the Diels-Alder reaction into the compound, because the production process is simple. A specific reversible bond partial structure can be represented by the following chemical formula. A reversible bond can be introduced into the compound by bonding with other structural units based on the R portion in the following formula in the maleimide-derived structure or various reactive functional groups on the ring in the furan-derived structure.

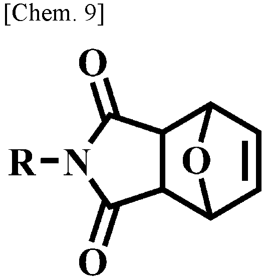

The Diels-Alder reaction is an addition reaction between a conjugated diene and a parent diene to form a 6-membered ring. Since the Diels-Alder reaction is an equilibrium reaction, a Retro-Diels-Alder reaction occurs at a predetermined temperature, resulting in dissociation (decrosslinking). When the obtained cured product is subjected to scratches or mechanical energy such as an external force, the C-C bond of the Diels-Alder reaction unit has lower bonding energy than that of a normal covalent bond, and thus the C-C bond of the Diels-Alder reaction unit will be preferentially broken. Therefore, the cured product exhibits easy disassembly. In addition, in the temperature range lower than the dissociation temperature, it is believed that the equilibrium of the C-C bond of the Diels-Alder reaction unit moves toward the binding direction, thus forming the adduct (Diels-Alder reaction unit) again and allowing damage repair and remolding.

In the reversible bond by the Diels-Alder reaction, for example, the Diels-Alder reaction unit consisting of an anthracene structure and a maleimide structure has a high dissociation temperature of 250°C or higher and maintains the cross-linked structure without dissociation at least at about 200°C. On the other hand, the reversible bond by the Diels-Alder reaction with the furan structure and the maleimide structure undergo a Retro-Diels-Alder reaction at around 120°C, causing dissociation (decrosslinking). Therefore, the heating temperature required for the cured product to exhibit easy disassembly can be reduced, and the cured product has excellent easy disassembly for applications where high-temperature heating is not suitable.

Although the the reversible bond described above will be present in at least two sites in the target phenolic hydroxyl group-containing compound, it is preferable to have the multiple reversible bonds in the structural unit B as well from the viewpoint of obtaining a structure with higher molecular mobility and facilitating adjustment of properties such as mechanical strength of the cured product.

In addition, for the same reason as above, the molecular weight as the structural unit B is preferably have a certain size or more, for example, the average molecular weight (Mw) thereof is preferably 28 or more. When there are reversible bonds in the structural unit B, the molecular weight between the reversible bonds is preferably 28 or more. A crosslinkable functional group similar to the phenolic hydroxyl group in the structural unit A may be present in the structural unit B, but from the viewpoint of more easily expressing the effect of the present invention, it is preferable to have no crosslinkable (curable) functional group.

The structural unit B preferably contains an alkylene chain or an alkylene ether chain, from the viewpoint of enabling the cured product to exhibit greater flexibility or better conformability to a base material when the phenolic hydroxyl group-containing compound of the present invention is used, for example, as a structural adhesive, and in this case, the alkylene chain more preferably contains 2 to 30 carbon atoms, and most preferably contains 4 to 16 carbon atoms. The alkylene ether chain is not particularly limited, but is preferably an alkylene ether chain having 2 to 12 carbon atoms, and the average value of repetition number is preferably in the range of 2 to 30.

The number of phenolic hydroxyl groups in the structural unit A is not particularly limited, but from the viewpoints of industrial availability of raw materials and ease of adjustment of crosslinking density when a cured product is obtained, the number of hydroxyl groups is preferably in the range of 1 to 3, and more preferably 1 to 2.

The average molecular weight (Mw) of the phenolic hydroxyl group-containing compound is not particularly limited, but is preferably 500 or more, and preferably 50,000 or less, from the viewpoint of achieving mechanical strength, flexibility, and easy disassembly, as well as repairability, and remoldability when used as a cured product. In addition, when there are multiple reversible bonds other than between A and B, for example in the structural unit B, it is more preferable that the molecular weight per reversible bond be in the range of 300 to 10000 from the viewpoint of the easy disassembly and remoldability of the cured product.

The phenolic hydroxyl group-containing compound according to an embodiment of the present invention is a compound represented by the following general formula.

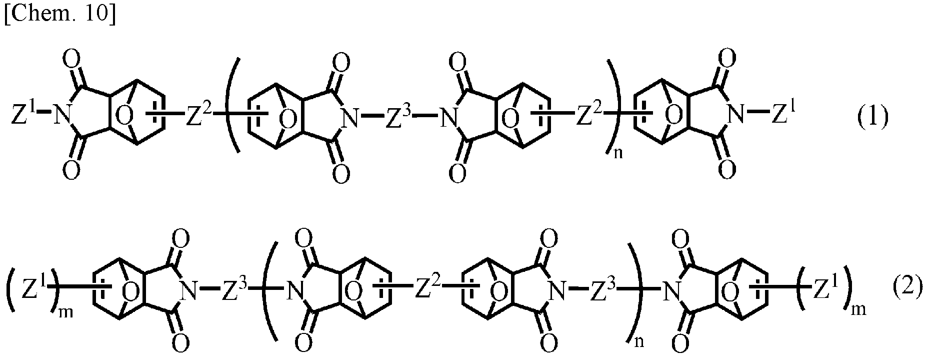

The furan-derived structure in the formulas (1) and (2) may include a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group, as a substituent. In the formulas, m is an integer of 1 to 4, and n is an average value of repetition number of 0 to 10. Z¹ is any of the structures represented by the following formula (3), Z² is any of the structures represented by the following formula (4), Z³ is any of the structures represented by by the following formula (5), and each of the multiple structures present in one molecule may be identical or different from each other.

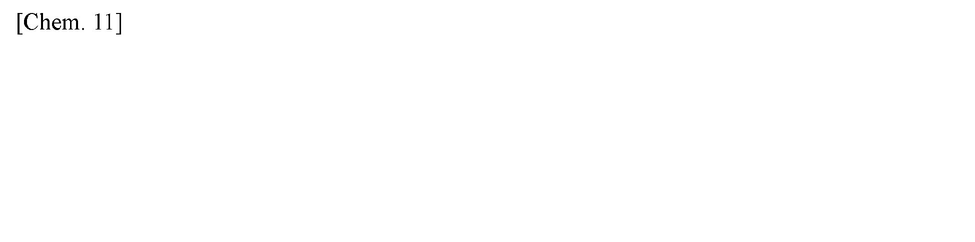

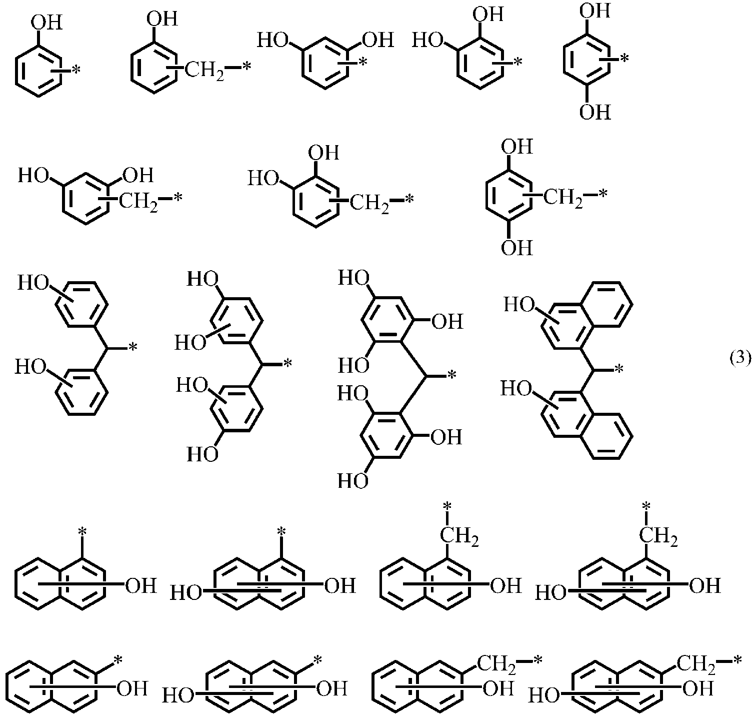

The aromatic ring in the formula (3) may be substituted or unsubstituted, and * denotes a binding point. The hydroxyl group on the naphthalene ring in the formula indicates that the hydroxyl group may be bonded at any site.

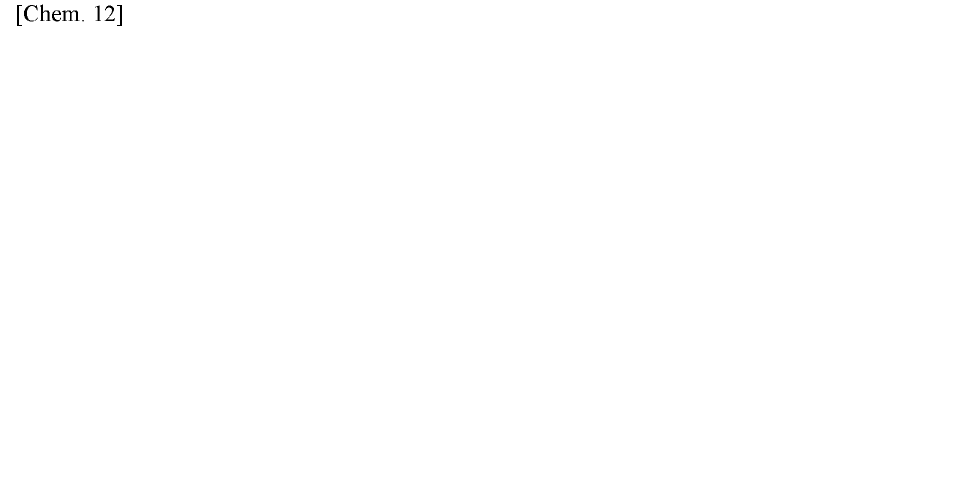

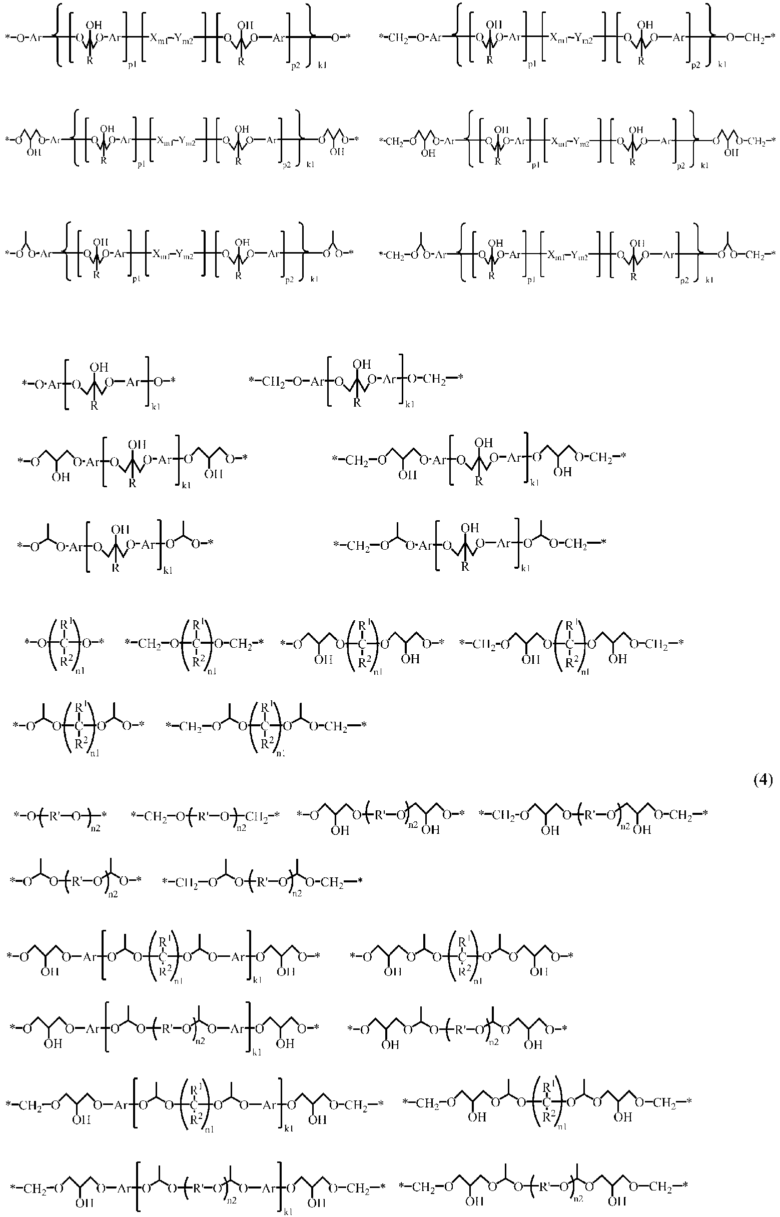

In the formula (4),
Ar's are each independently a structure containing an unsubstituted aromatic ring or an aromatic ring having a substituent,
R¹ and R² are each independently a hydrogen atom, a methyl group or an ethyl group,
R is a hydrogen atom or a methyl group,
R' is a divalent hydrocarbon group having 2 to 12 carbon atoms,
n1 is an integer of 2 to 16, and n2 is an average value of repeating units of 2 to 30,
k1 is an average value of repetition number, in a range of 0.5 to 10,
p1 and p2 are each independently 0 to 5, and
X is a structural unit represented by the following formula (4-1) and Y is a structural unit represented by the following formula (4-2), 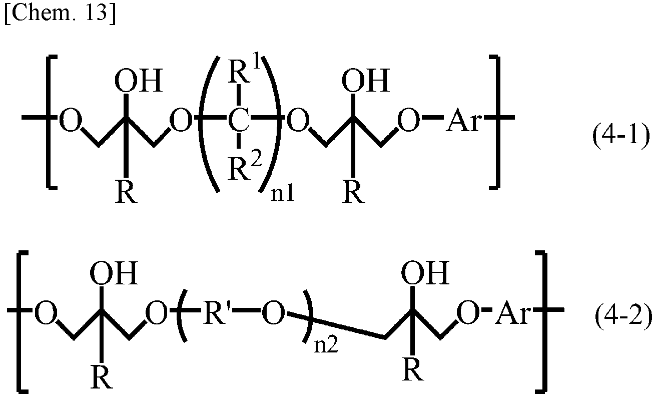
in the formulas (4-1) and (4-2), Ar, R, R¹, R², R', n1, and n2 are the same as above.
m1 and m2 are an average value of repetition number, each independently 0 to 25, and m1 + m2 ≥ 1,
provided that a bond between the structural unit X represented by the formula (4-1) and the structural unit Y represented by the formula (4-2) may be random or blocked, and total numbers of respective structural units X and Y present in one molecule are m1 and m2, respectively.

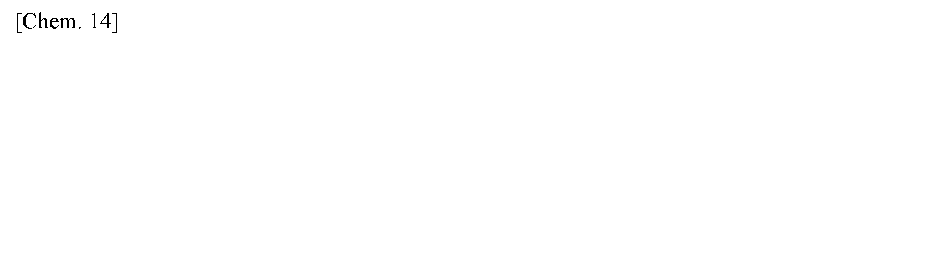

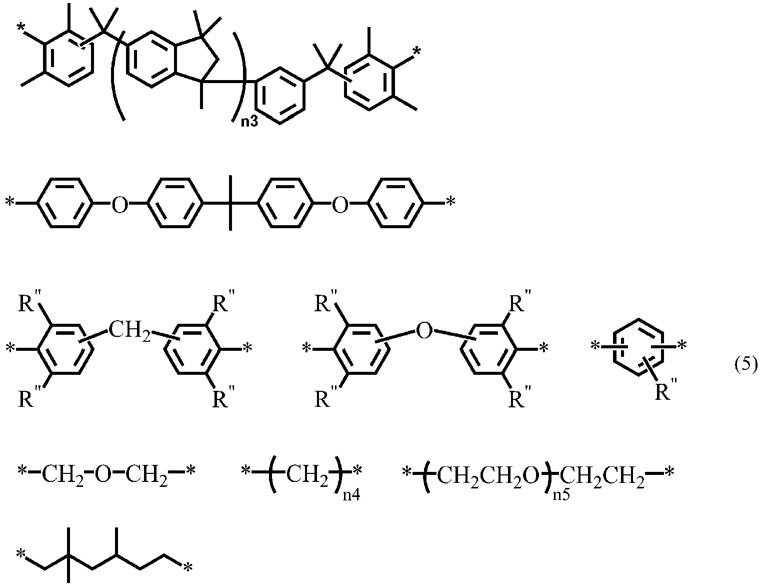

In the formula (5), n3 and n5 are an average value of repetition number and are 0.5 to 10, respectively,
n4 is an integer of 1 to 16, and
R"'s are each independently a hydrogen atom, a methyl group or an ethyl group.

The general formulas (1) and (2) have a reversible bond formed by a furan structure and a maleimide structure at the terminal within the molecule. The terminal maleimide structure in the general formula (1) and the terminal furan structure in the general formula (2) each have one or more Z1 which is any structure represented by the general formula (3), and this phenolic hydroxyl group contributes to the curing reaction in the curable resin composition described later. m is the number of Z1 in the furan-derived structure and is an integer of 1 to 4, but is preferably in the range of 1 to 2, and more preferably 1, from the viewpoints of industrial availability of raw materials, ease of control of the curing reaction, and the like.

In the formula, Z1 is one having the phenolic hydroxyl group represented by the general formula (3), but among these, from the viewpoint of availability of raw materials and reactivity, those represented by the following structural formulas are preferable.

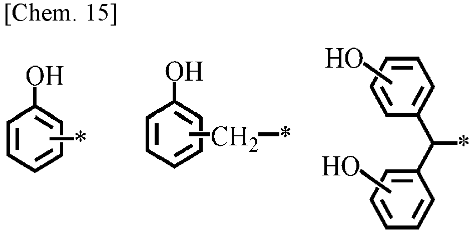

In the formula, Z2 is represented by the general formula (4), but among these, from the viewpoint of availability of raw materials, reactivity, and the balance between toughness and flexibility of the obtained compound, those represented by the following structural formulas are preferable. 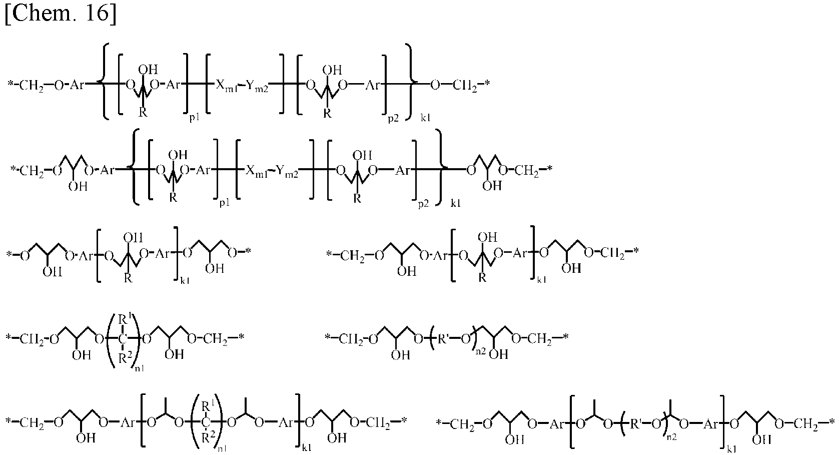 [Ar, R, m1, m2, n1, n2, k1, p1, p2, X, and Y in the formulas are the same as above]

In the above general formulas (1) and (2), the site linking the furan-derived structure is Z2, and the site linking the maleimide-derived structure is Z3, which are any of the structures represented by the above general formulas (4) and (5), respectively.

n in the general formulas (1) and (2) is the average value of repetition number and is 0 to 10, preferably in the range of 0 to 5.

Ar in these structural formulas is an aromatic ring that may have a substituent, and is not particularly limited. Examples of the aromatic ring include a benzene ring, a naphthalene ring, an anthracene ring, a phenanthrene ring, and a fluorene ring. Examples of the substituent include a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an arakyl group, and an aryl group. It is preferable that the substituent on Ar does not cause a curing reaction when used as the curable resin composition described later, since the effect of the present invention is more easily exhibited.

Among these, Ar is preferably any structure represented by the following structural formulas. 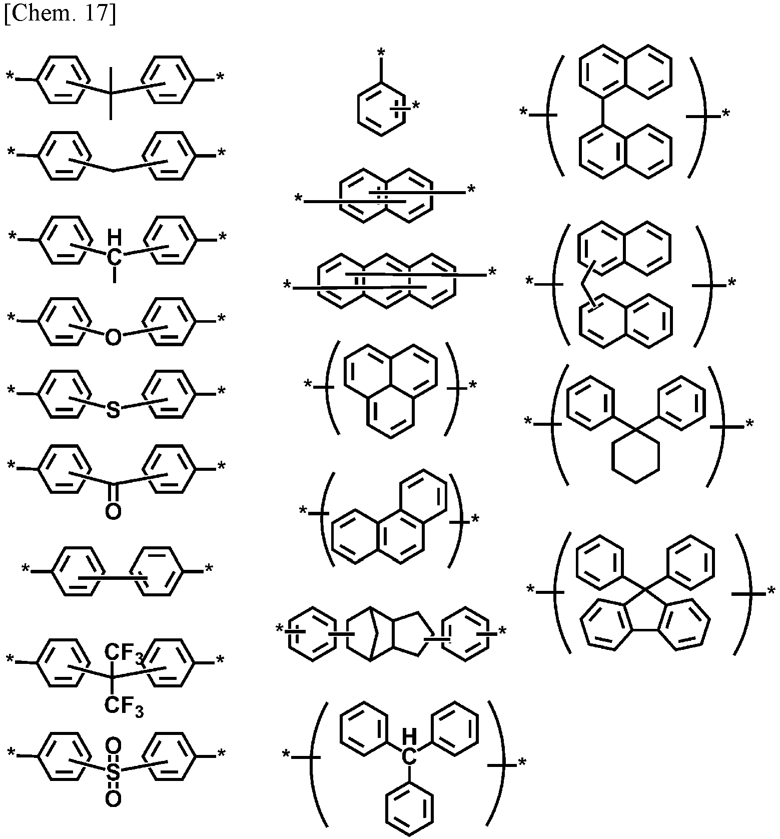 [The aromatic ring in the formulas may be substituted or unsubstituted, and * denotes a binding point.]

Further, the examples of Ar include structures represented by the following formulas. 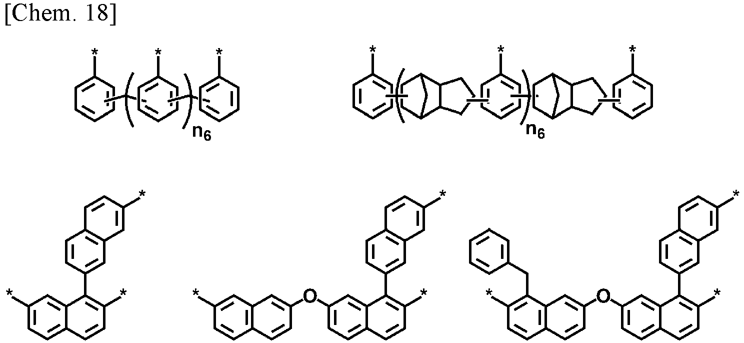 (In the formulas, the aromatic ring may be substituted or unsubstituted, n₆ = 1 to 4, and * denotes a binding point.)

The following structures of Ar are particularly preferable. * denotes a binding point.

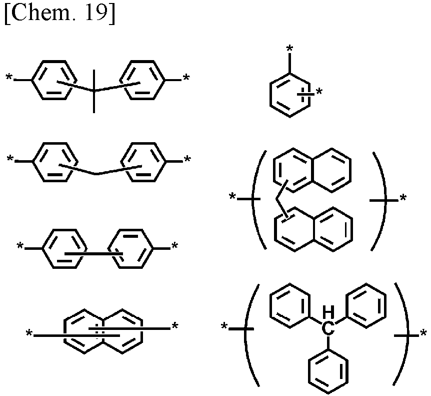

The repeating unit n₁ in the general formulas (4) and (4-1) is an integer of 2 to 16. When n₁ is 4 or more, the deformation mode when a cured product is obtained is likely to be elastic deformation. In addition, n₁ is less than or equal to 16, which prevents a decrease in crosslinking density. n₁ is preferably 4 to 15, more preferably 6 to 12.

In the general formulas (4) and (4-1), R¹ and R² are each independently a hydrogen atom, a methyl group or an ethyl group, and R's are each independently a hydrogen atom or a methyl group, respectively. Among these, a hydrogen atom is preferable.

In the general formulas (4) and (4-2), n₂ is an average value of the repeating units of 2 to 30. In this range, the balance between the viscosity of the phenolic hydroxyl group-containing compound and the crosslinking density of the obtained cured product becomes favorable, and thus it is preferable. The range is preferably 2 to 25, more preferably 4 to 20.

In the general formulas (4) and (4-2), R' is a divalent hydrocarbon group having 2 to 12 carbon atoms. In this range, adhesive strength is improved and the deformation mode of the cured product is more likely to be elastic deformation. Preferably, R' is a divalent hydrocarbon group having 2 to 6 carbon atoms.

Examples of the divalent hydrocarbon group are not particularly limited, but include a linear or branched-chain alkylene group, an alkenylene group, an alkynylene group, a cycloalkylene group, an arylene group, and an aralkylene group (a divalent group having an alkylene group and an arylene group).

Examples of the alkylene group include a methylene group, an ethylene group, a propylene group, a butylene group, a pentylene group, a hexylene group, a trimethylene group, a tetramethylene group, a pentamethylene group, and a hexamethylene group. Examples of the alkenylene group include a vinylene group, a 1-methylvinylene group, a propenylene group, a butenylene group, and a pentenylene group. Examples of the alkynylene group include an ethynylene group, a propynylene group, a butynylene group, a pentynylene group, and a hexynylene group. Examples of the cycloalkylene group include a cyclopropylene group, a cyclobutylene group, a cyclopentylene group, and a cyclohexylene group. Examples of the arylene group include a phenylene group, a tolylene group, a xylylene group, and a naphthylene group.

Among these, an ethylene group, a propylene group, and a tetramethylene group are preferable from the viewpoint of a balance between the availability of raw materials, the viscosity of the obtained phenolic hydroxyl group-containing compound, and the flexibility when a cured product is obtained.

In the general formulas (4) and (4-2), R's are each independently a hydrogen atom or a methyl group. Among these, a hydrogen atom is preferable.

m1 and m2 in the general formula (4) are an average value of repetition number of the structural unit X and the structural unit Y described above, respectively, and are each independently 0 to 25, and m1 + m2 ≥ 1. Preferably, m1 and m2 are in the range of 0.5 to 10, respectively.

In addition, k1 in the general formula (4) is the average of the repetitions and is in the range of 0.5 to 5, preferably in the range of 0.5 to 2.

In the general formula (5), n3 and n5 are an average value of repetition number and are 0.5 to 10, respectively, n4 is an integer of 1 to 16, and R"'s are each independently a hydrogen atom, a methyl group or an ethyl group. Among these, from the viewpoint of availability of raw materials and mechanical properties of the obtained cured product, n3 is preferably in the range of 0.5 to 10, n5 is preferably in the range of 2 to 3, n4 is preferably an integer of 1 to 8, and R" is preferably a hydrogen atom.

Examples of the phenolic hydroxyl group-containing compound of the present invention include, but are not limited to, those represented below. 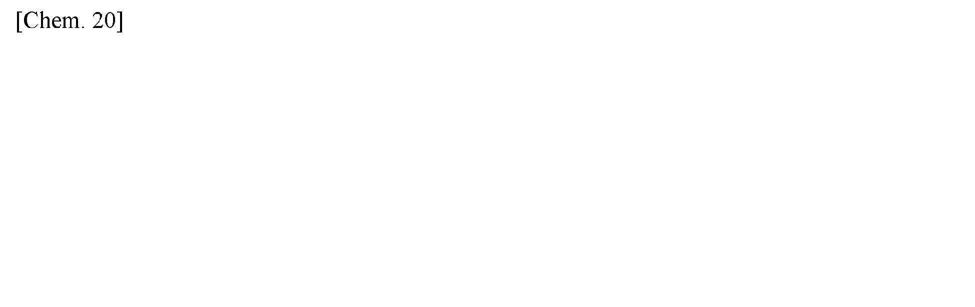 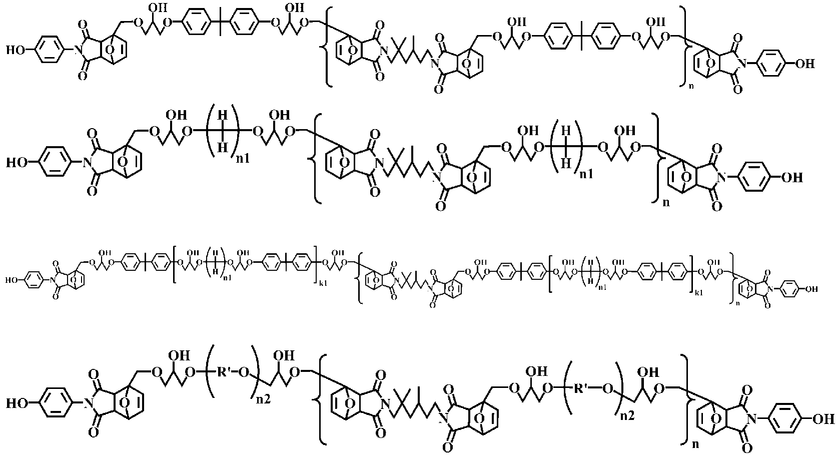 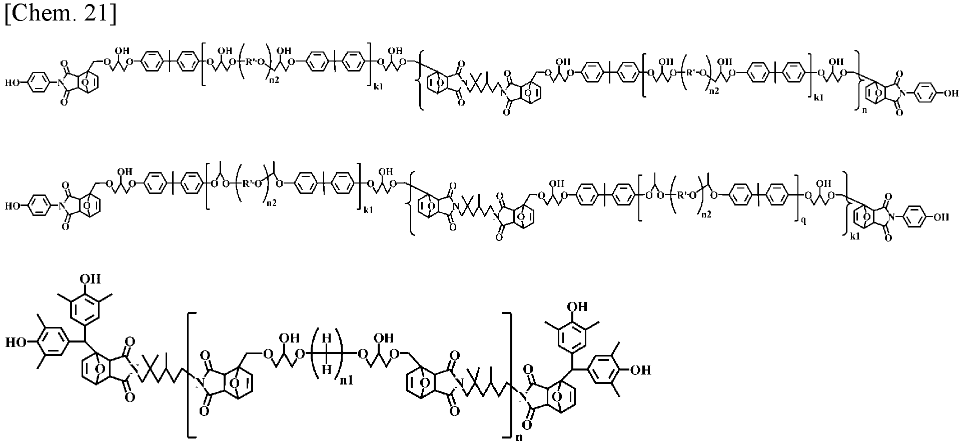 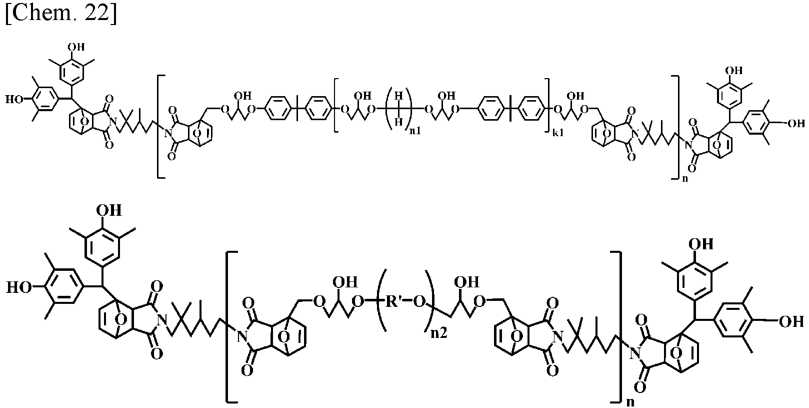 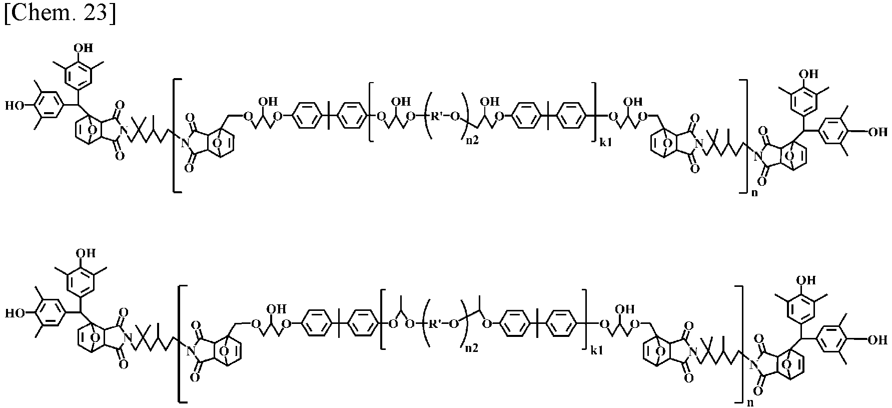 [R' in the formulas is a divalent hydrocarbon group having 2-12 carbon atoms, n is an average number of repeating units of 0 to 10, n1 is an integer of 4 to 16, n2 is an average value of repeating units of 2 to 30, and k1 is 0.5 to 5.]

A method for producing the phenolic hydroxyl group-containing compound according to one embodiment of the present invention is not particularly limited, and the compound may be produced stepwise by using known reactions depending on the target structure, and the compound may be obtained by appropriately combining commercially available raw materials. Hereinafter, a representative synthesis method will be described.

The general formulas (1) and (2) each have, as a reversible bond, two Diels-Alder reaction units, which are addition reaction units formed by a Diels-Alder reaction consisting of a furan structure and a maleimide structure, within the molecule, and can be obtained by using a maleimide compound having the structure Z1 in the general formula (1), and by using a furan compound having the structure Z1 in the general formula (2).

It is widely known that the so-called Diels-Alder reaction in which a conjugated diene such as a furan structure and a parent diene such as a maleimide structure undergo an addition reaction to form a 6-membered ring is an equilibrium reaction, and that at a temperature higher than the temperature at which the addition reaction proceeds, a retro-Diels-Alder reaction proceeds, that is a reverse reaction in which the addition reaction unit dissociates to return to the original conjugated diene and parent diene.

Examples of the maleimide compound having the structure of Z1 include any of the compounds listed in the following formulas. Among these, hydroxyphenylmaleimide is preferable from the viewpoint of curability, and monohydroxyphenylmaleimide is particularly preferable from the viewpoint of a balance between reactivity, physical properties of the cured product, and easy disassembly, repairability, and remoldability. Among monohydroxyphenylmaleimides, para-hydroxyphenylmaleimide is particularly preferable from the viewpoint of heat resistance.

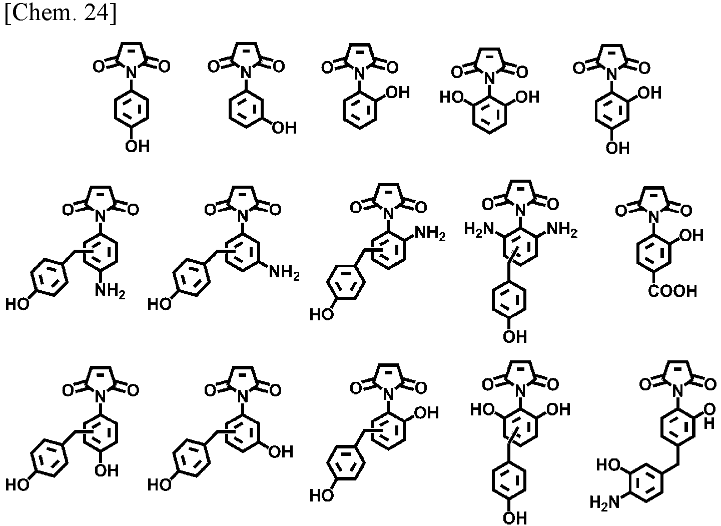

Examples of the furan compound having the structure of Z1 include any of the compounds listed in the following formulas.

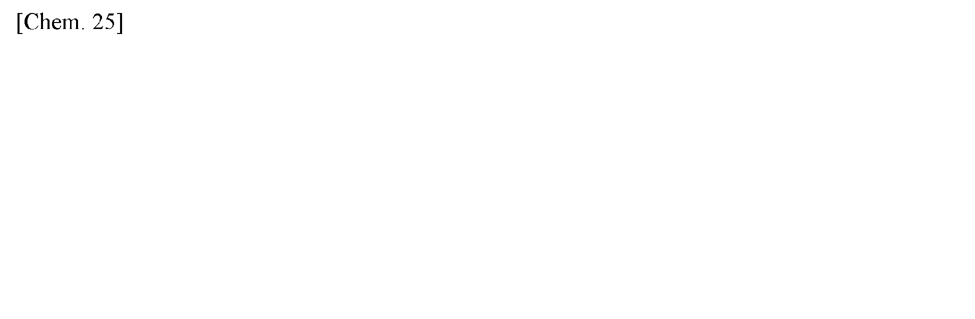

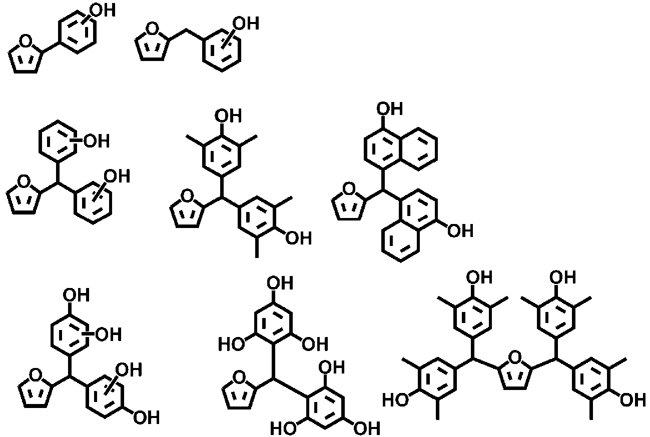

Among the above formulas, the following compounds are particularly preferable from the viewpoint of a balance between reactivity, physical properties of the cured product, and easy disassembly, repairability, and remoldability.

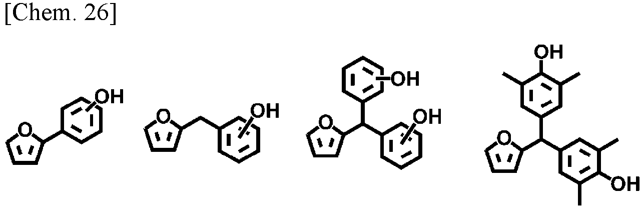

The structures of the maleimide compound and the furan compound each include those having, independently of each other, a hydrogen atom, a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group or an aryl group as a substituent. **In** addition, in the structures of the compounds listed in the above formulas, the alkoxy group, the aralkyloxy group, the aryloxy group, the carboxy group, the alkyloxycarbonyl group, the aryloxycarbonyl group, the alkyl group, the cycloalkyl group, the aralkyl group and the aryl group also include those having various substituents further bonded to the carbon atoms thereof.

The Diels-Alder reaction can be performed by any known method. For example, a conjugated diene compound and a parent diene compound are mixed in equimolar amounts, or in some cases, one of the components is used in excess, and the mixture is melted by heating or dissolved in a solvent and stirred at room temperature to 110°C for 1 to 24 hours. The resulting product can be obtained by filtration or solvent distillation without purification, or by a commonly used isolation and purification method such as recrystallization, reprecipitation, or chromatography.

The sites other than the reversible bond can be synthesized by known methods. For example, a diglycidyl ether of an aliphatic dihydroxy compound or an aliphatic divinyl ether is reacted with an aromatic hydroxy compound to obtain a compound having a hydroxy group at the terminal, which is then reacted with furfuryl glycidyl ether or the like to introduce a furan structure at the terminal, and further, a Diels-Alder reaction is performed with a maleimide compound having a phenolic hydroxyl group as described above to obtain the compound represented by the general formula (1).

Alternatively, a compound having a hydroxy group at the terminal is obtained, and then epoxidized to convert the terminal into a glycidyl ether group. Thereafter, the compound is reacted with furfuryl alcohol or the like to introduce a furan structure at the terminal. Furthermore, the compound represented by the general formula (1) can be obtained by performing a Diels-Alder reaction with a maleimide compound having a phenolic hydroxyl group as described above.

Alternatively, an aromatic dihydroxy compound is reacted with a dihalogenated alkyl compound or a dihalogenated aralkyl compound to obtain a compound having a halogenated alkyl group at the terminal, and then the compound is reacted with furfuryl alcohol or the like to introduce a furan structure at the terminal. Furthermore, the compound is subjected to a Diels-Alder reaction with a maleimide compound having the phenolic hydroxyl group as described above to obtain the compound represented by the general formula (1).

Examples of the diglycidyl ether of the aliphatic dihydroxy compound are not particularly limited, but include 1,11-undecanediol diglycidyl ether, 1,12-dodecanediol diglycidyl ether, 1,13-tridecanediol, 1,14-tetradecanediol diglycidyl ether, 1,15-pentadecanediol diglycidyl ether, 1,16-hexadecanediol diglycidyl ether, 2-methyl-1,11-undecanediol diglycidyl ether, 3-methyl-1,11-undecanediol diglycidyl ether, and 2,6,10-trimethyl-1,11-undecanediol diglycidyl ether, which may be used alone or in combination of two or more kinds thereof.

Among these, a compound having a structure in which glycidyl groups are linked via an ether group to both terminals of an alkylene chain having 12 to 14 carbon atoms are preferable from the viewpoint of an excellent balance between flexibility and heat resistance of the obtained cured product, and it is most preferable to use 1,12-dodecanediol diglycidyl ether, 1,13-tridecanediol, or 1,14-tetradecanediol diglycidyl ether.

Examples of the aliphatic divinyl ether is not particularly limited, but include divinyl ethers of linear alkylene groups, such as polyethylene glycol divinyl ether, polypropylene glycol divinyl ether, polytetramethylene glycol divinyl ether, 1,3-butylene glycol divinyl ether, 1,4-butanediol divinyl ether, 1,6-hexanediol divinyl ether, 1,9-nonanediol divinyl ether, and 1,10-decanediol divinyl ether, divinyl ethers of a branched alkylene group such as neopentyl glycol divinyl ether, divinyl ethers containing a cycloalkane structure, such as 1,4-cyclohexanediol divinyl ether, 1,4-cyclohexanedimethanol divinyl ether, tricyclodecanediol divinyl ether, tricyclodecane dimethanol divinyl ether, pentacyclopentadecanedimethanol divinyl ether, and pentacyclopentadecanediol divinyl ether, bisphenol A divinyl ether, bisphenol F divinyl ether, and hydroquinone divinyl ether, which may be used alone or in combination of two or more kinds thereof.

Among these, from the viewpoint of an excellent balance between flexibility and toughness of the obtained cured product, a divinyl ether having a polyether structure or a linear alkylene chain having 9 to 10 carbon atoms is preferable, and it is most preferable to use polyethylene glycol divinyl ether, polypropylene glycol divinyl ether, polytetramethylene glycol divinyl ether, 1,9-nonanediol divinyl ether, and 1,10-decanediol divinyl ether.

Examples of the aromatic hydroxy compound are not particularly limited, but include dihydroxybenzenes such as hydroquinone, resorcin, and catechol; trihydroxybenzenes such as pyrogallol, 1,2,4-trihydroxybenzene, and 1,3,5-trihydroxybenzene; triphenylmethane type phenols such as 4,4',4"-trihydroxytriphenylmethane; dihydroxynaphthalenes such as 1,6-dihydroxynaphthalene, 2,7-dihydroxynaphthalene, 1,4-dihydroxynaphthalene, 1,5-dihydroxynaphthalene, 2,3-dihydroxynaphthalene, and 2,6-dihydroxynaphthalene; tetrafunctional phenols such as 1,1'-methylenebis-(2,7-naphthalenediol), 1,1'-binaphthalene-2,2',7,7'-tetraol, and 1,1'-oxybis-(2,7-naphthalenediol) obtained by coupling reaction of dihydroxynaphthalenes; bisphenols such as bis(4-hydroxyphenyl)methane, 2,2-bis(4-hydroxyphenyl)propane, 2,2-bis(3-methyl-4-hydroxyphenyl)propane, 1,1-bis(4-hydroxyphenyl)cyclohexane, and 1,1-bis(4-hydroxyphenyl)-1-phenylethane, and bis(4-hydroxyphenyl)sulfone; biphenols such as 2,2'-biphenol, 4,4'-biphenol, (1,1'-biphenyl)-3,4-diol, 3,3'-dimethyl-(1,1'-biphenyl)-4,4'-diol, 3-methyl-(1,1'-biphenyl)-4,4'-diol, 3,3',5,5'-tetramethylbiphenyl-2,2'-diol, 3,3',5,5'-tetramethylbiphenyl-4,4'-diol, 5-methyl-(1,1'-biphenyl)-3,4'diol, 3'-methyl-(1,1'-biphenyl)-3,4'diol, and 4'-methyl-(1,1'-biphenyl)-3,4'diol; alicyclic structure-containing phenols such as polyaddition products of phenol and dicyclopentadiene, and polyaddition products of phenol and terpene compounds; naphthols such as bis(2-hydroxy-1-naphthyl)methane and bis(2-hydroxy-1-naphthyl)propane; and the so-called zylock phenolic resin that is a condensation reaction product of phenol and phenylene dimethyl chloride or biphenylene dimethyl chloride, which may be used alone or in combination of two or more kinds thereof. Further, examples thereof include a bifunctional phenol compound having a structure in which there is a substitution with a methyl group, a t-butyl group, or a halogen atom as a substituent at the aromatic nucleus of each compound. The alicyclic structure-containing phenols and the zylock phenolic resin may contain not only bifunctional components but also trifunctional or higher functional components at the same time. The compounds may be used as they are, or may be subjected to a purification process such as a column purification process to extract only the bifunctional components.

Among these, in terms of excellent balance between flexibility and toughness when a cured product is obtained, bisphenols are preferable, and especially from the point of view of the remarkable performance in imparting toughness, bis(4-hydroxyphenyl)methane and 2,2-bis(4-hydroxyphenyl)propane are preferable. Further, when moisture resistance of the cured product is important, phenols containing an alicyclic structure are preferably used.

Regarding the reaction ratio of the diglycidyl ether of the aliphatic dihydroxy compound to the aromatic hydroxy compound, the reaction is preferably performed in the range of 1/1.01 to 1/5.0 (molar ratio) of the former/the latter, and in terms of providing a well-balanced combination of flexibility and heat resistance of the obtained cured product, it is preferable that (a1)/(a2) is 1/1.02 to 1/3.0 (molar ratio).

The reaction between the diglycidyl ether of the aliphatic dihydroxy compound and the aromatic hydroxy compound is preferably performed in the presence of a catalyst. As the catalyst, various catalysts can be used, and examples thereof include alkaline (earth) metal hydroxides such as sodium hydroxide, potassium hydroxide, lithium hydroxide, and calcium hydroxide; alkali metal carbonates such as sodium carbonate and potassium carbonate, phosphorus compounds such as triphenylphosphine, DMP-30, DMAP, chlorides, bromides, and iodides of tetramethylammonium, tetraethylammonium, tetrabutylammonium, benzyltributylammonium, and the like, quaternary ammonium salts such as chlorides, bromides, and iodides of tetramethylphosphonium, tetraethylphosphonium, tetrabutylphosphonium, benzyltributylphosphonium, and the like; tertiary amines such as triethylamine, N,N-dimethylbenzylamine, 1,8-diazabicyclo[5.4.0]undecene, and 1,4-diazabicyclo[2.2.2]octane; and imidazoles such as 2-ethyl-4-methylimidazole and 2-phenylimidazole. Two or more kinds of catalysts may be used in combination. Among these, sodium hydroxide, potassium hydroxide, triphenylphosphine, and DMP-30 are preferable because the reaction proceeds quickly and the effect of reducing the amount of impurities is high. The amount of these catalysts used is not particularly limited, but it is preferable to use 0.0001 to 0.01 mole per mole of the phenolic hydroxyl group of the aromatic hydroxy compound. The form of these catalysts is not particularly limited, and the catalysts may be used in the form of an aqueous solution or in the form of a solid.

The reaction between the diglycidyl ether of the aliphatic dihydroxy compound and the aromatic hydroxy compound can be performed in the absence of a solvent or in the presence of an organic solvent. Examples of the organic solvent that can be used include methyl cellosolve, ethyl cellosolve, toluene, xylene, methyl isobutyl ketone, dimethyl sulfoxide, propyl alcohol, and butyl alcohol. The amount of the organic solvent used is usually 50 to 300% by mass, preferably 100 to 250% by mass, based on the total mass of the raw materials charged. These organic solvents can be used alone or two or more kinds thereof can be mixed and used. In order to perform the reaction quickly, it is preferable to use no solvent, while the use of dimethyl sulfoxide is preferable in terms of reducing impurities in the final product.

The reaction temperature when performing the above reaction is usually 50 to 180°C, and the reaction time is usually 1 to 10 hours. The reaction temperature is preferably 100 to 160°C in terms of reducing impurities in the final product. In addition, when the obtained compound is significantly colored, an antioxidant or a reducing agent may be added to suppress the coloring. Examples of the antioxidant are not particularly limited, but include hindered phenol compounds such as 2,6-dialkylphenol derivatives, divalent sulfur compounds, and phosphite ester compounds containing a trivalent phosphorus atom. Examples of the reducing agent are not particularly limited, but include hypophosphorous acid, phosphorous acid, thiosulfuric acid, sulfurous acid, hydro sulfite, and salts thereof.

After the reaction is completed, the reaction mixture may be neutralized or washed until the pH value of the reaction mixture reaches 3 to 7, preferably 5 to 7. The neutralization and washing treatments may be performed in the usual manner. For example, when a basic catalyst is used, an acidic substance such as hydrochloric acid, sodium dihydrogen phosphate monobasic, p-toluenesulfonic acid, or oxalic acid can be used as a neutralizing agent. After neutralization or washing with water, the compound can be obtained by, if necessary, distilling off the solvent under reduced pressure and heating, and concentrating the product.

The reaction ratio of the aliphatic divinyl ether and the aromatic hydroxy compound is preferably reacted in the range of 1/1.01 to 1/5.0 (molar ratio) of the former/the latter, and in terms of providing a well-balanced combination of flexibility and heat resistance of the obtained cured product, it is preferable that (a1)/(a2) is in the range of 1/1.02 to 1/3.0 (molar ratio).

The reaction between the diglycidyl ether of the aliphatic dihydroxy compound and the aromatic hydroxy compound proceeds sufficiently without the use of a catalyst, but a catalyst can be used as appropriate from the viewpoint of the selection of raw materials and increasing the reaction rate. Examples of the catalyst that can be used herein include inorganic acids such as sulfuric acid, hydrochloric acid, nitric acid, and phosphoric acid; organic acids such as toluenesulfonic acid, methanesulfonic acid, xylenesulfonic acid, trifluoromethanesulfonic acid, oxalic acid, formic acid, trichloroacetic acid, and trifluoroacetic acid; and Lewis acids such as aluminum chloride, iron chloride, tin chloride, gallium chloride, titanium chloride, aluminum bromide, gallium bromide, boron trifluoride ether complex, and boron trifluoride phenol complex. The amount of the catalyst used is usually in the range of 10 ppm to 1% by weight based on the mass of the divinyl ether compound. In this case, it is preferable to select the type and amount used so as not to cause a nuclear addition reaction of the vinyl group to the aromatic ring.

In addition, the reaction between the aliphatic divinyl ether and the aromatic hydroxy compound can be performed in the absence of a solvent or in the presence of an organic solvent. Examples of the organic solvent include aromatic organic solvents such as benzene, toluene, and xylene; ketone-based organic solvents such as acetone, methyl ethyl ketone, methyl isobutyl ketone, and cyclohexanone; and alcohol-based organic solvents such as methanol, ethanol, isopropyl alcohol, and normal butanol. The amount of the organic solvent used is usually 50 to 300% by mass, preferably 100 to 250% by mass, based on the total mass of the raw materials charged. These organic solvents can be used alone or two or more kinds thereof can be mixed and used.

The reaction temperature when performing the above reaction is usually 50 to 150°C, and the reaction time is usually 0.5 to 10 hours. In this case, in order to prevent self-polymerization of the vinyl ether group, the reaction is preferably performed in an oxygen atmosphere.

After completion of the reaction, when an organic solvent was used, the organic solvent is removed under reduced pressure and heating, and when a catalyst was used, the catalyst is deactivated with a deactivator or the like as necessary, and then removed by washing with water or filtration, whereby the compound can be obtained.

The compound having a hydroxyl group at the terminal thus obtained is reacted with furfuryl glycidyl ether or the like. In this case, sodium hydroxide, potassium hydroxide, potassium carbonate, or the like can be used as a catalyst, and toluene, acetone, methyl ethyl ketone (MEK), methyl isobutyl ketone, acetonitrile, dimethylformamide, or the like can be used as a solvent. The reaction temperature is from room temperature to 200°C, and the reaction time is 1 to 24 hours. Thereafter, the catalyst is then removed by filtration or the like, and the target compound can be obtained by extraction, solvent removal, or the like. The Diels-Alder reaction for this compound is as described above.

Examples of the aliphatic hydroxy compound are not particularly limited, but include 1,4-butanediol, 1,5-pentanediol, 1,6-hexanediol, 1,7-heptanediol, 1,8-octanediol, 1,9-nonanediol, 1,10-decanediol, 1,11-undecanediol, 1,12-dodecanediol, 1,13-tridecanediol, 1,14-tetradecanediol, 1,15-pentadecanediol, 1,16-hexadecanediol, 2-methyl-1,11-undecanediol, 3-methyl-1,11-undecanediol, 2,6,10-trimethyl-1,11-undecanediol, polyethylene glycol, polypropylene glycol, polytetramethylene glycol, polypentamethylene glycol diglycidyl ether, polyhexamethylene glycol diglycidyl ether, polyheptamethylene glycol diglycidyl ether, which may be used alone or in combination of two or more kinds thereof.

Among these, from the viewpoint of an excellent balance between flexibility and heat resistance of the obtained cured product, it is preferable to use a dihydroxy compound having a polyether structure or a linear alkylene chain having 12 to 14 carbon atoms, and it is most preferable to use polyethylene glycol, polypropylene glycol, polytetramethylene glycol, 1,12-dodecanediol, 1,13-tridecanediol, or 1,14-tetradecanediol.

Examples of the dihalogenated alkyl compound are not particularly limited, but include 1,4-dichlorobutane, 1,5-dichloropentane, 1,6-dichlorohexane, 1,7-dichloroheptane, 1,8-dichlorooctane, 1,9-dichlorononane, 1,10-dichlorodecane, 1,11-dichloroundecane, 1,12-dichlorododecane, 1,4-dibromobutane, 1,5-dibromopentane, 1,6-dibromohexane, 1,7-dibromoheptane, 1,8-dibromooctane, 1,9-dibromononane, 1,10-dibromodecane, 1,11-dibromoundecane, 1,12-dibromododecane, which may be used alone or in combination of two or more kinds thereof.

Examples of the dihalogenated aralkyl compound are not particularly limited, but include dichloroxylene, dichloromethylbiphenyl, dibromoxylene, dibromomethylbiphenyl, which may be used alone or in combination of two or more kinds thereof.

Regarding the reaction ratio of the aromatic dihydroxy compound to the dihalogenated alkyl compound or dihalogenated aralkyl compound, the reaction is preferably performed in the range of 1/1.01 to 1/5.0 (molar ratio) of the former/the latter, and from the viewpoint of providing a well-balanced combination of flexibility and heat resistance of the obtained cured product, it is preferable that (a1)/(a2) is in the range of 1/1.02 to 1/3.0 (molar ratio).

The reaction between the aromatic dihydroxy compound and the dihalogenated alkyl compound or dihalogenated aralkyl compound is preferably performed in the presence of a catalyst. Various catalysts can be used as the catalyst, and examples thereof include alkali (earth) metal hydroxides such as sodium hydroxide, potassium hydroxide, lithium hydroxide, and calcium hydroxide, and alkali metal carbonates such as sodium carbonate and potassium carbonate. Two or more kinds of catalysts may be used in combination. Among these, sodium hydroxide, potassium hydroxide and potassium carbonate are preferable because the reaction proceeds quickly and the effect of reducing the amount of impurities is high. The amount of these catalysts used is not particularly limited, but it is preferable to use 0.0001 to 10 moles per mole of the phenolic hydroxyl group of the aromatic hydroxy compound. The form of these catalysts is not particularly limited, and the catalysts may be used in the form of an aqueous solution or in the form of a solid.

Further, the reaction between the aromatic dihydroxy compound and the dihalogenated alkyl compound or the dihalogenated aralkyl compound can be performed in the absence of a solvent or in the presence of an organic solvent. Examples of the organic solvent that can be used include toluene, acetone, methyl ethyl ketone (MEK), methyl isobutyl ketone, acetonitrile, and dimethylformamide. The amount of organic solvents used is usually 50 to 300% by mass, preferably 100 to 1000 % by mass, based on the total mass of the charged raw material. These organic solvents can be used alone or two or more kinds thereof can be mixed and used.

The reaction temperature when performing the reaction is usually from the room temperature to 150°C, and the reaction time is usually 1 to 24 hours. The reaction temperature is preferably at room temperature to 100°C because the impurities of the final product can be reduced.

Furfuryl alcohol or the like is reacted to a compound having a halogenated alkyl group at the terminal obtained in this way. In this case, sodium hydroxide, potassium hydroxide, potassium carbonate, or the like can be used as a catalyst, and toluene, acetone, methyl ethyl ketone (MEK), methyl isobutyl ketone, acetonitrile, dimethylformamide, or the like can be used as a solvent. The reaction temperature is from room temperature to 200°C, and the reaction time is 1 to 24 hours. Thereafter, the catalyst is then removed by filtration or the like, and the target compound can be obtained by extraction, solvent removal, or the like. The Diels-Alder reaction for this compound is as described above.

The intermediate of the conjugated diene or parent diene intermediate before the Diels-Alder reaction can be represented by the following general formulas (1)' and (2)'. 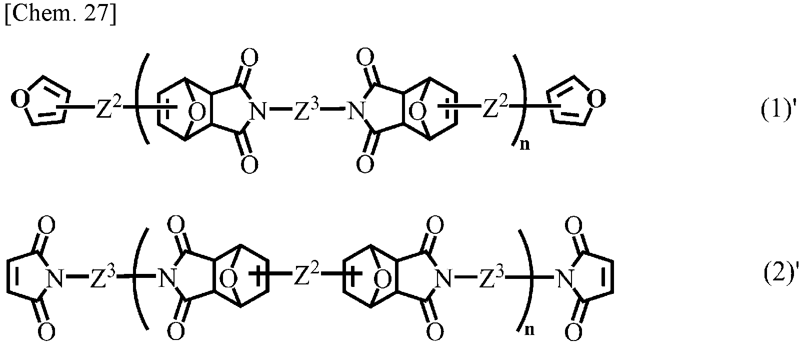 [in the formulas, n, Z², and Z³ are the same as above.]

The phenolic hydroxyl group-containing compound of the present invention can be a curable resin composition by combining a compound (I) having a reactivity with a phenolic hydroxyl group-containing compound. The curable resin composition can be suitably used for various electrical and electronic components such as an adhesive, a coating material, a photo resist, a printed wiring board, a semiconductor encapsulation material, and the like.

Examples of the compound (I) having reactivity with the phenolic hydroxyl group-containing compound include a melamine compound substituted with at least one group selected from a methylol group, an alkoxymethyl group, and an acyloxymethyl group, a guanamine compound, a glycol uril compound, a urea compound, a resol resin, an epoxy resin, an isocyanate compound, an azide compound, a compound containing double bonds such as an alkenyl ether group, acid anhydride, hexamethylenetetramine and the modified product thereof, an oxazoline compound.

Examples of the melamine compound include hexamethylol melamine, hexamethoxymethylmelamine, and compounds obtained by methoxymethylation of 1 to 6 methylol groups of hexamethylol melamine, , hexamethoxyethyl melamine, hexaacyloxymethyl melamine, and compounds obtained by acyloxymethylation of 1 to 6 methylol groups of hexamethylol melamine.

Examples of the guanamine compound include tetramethylol guanamine, tetramethoxymethyl guanamine, tetramethoxymethyl benzoguanamine, compounds obtained by methoxymethylation of 1 to 4 methylol groups of tetramethylol guanamine, tetramethoxyethyl guanamine, tetraacyloxyguanamine, and compounds obtained by acyloxymethylation of 1 to 4 methylol groupsof tetramethylol guanamine.

Examples of the glycol uril compound include 1,3,4,6-tetrakis(methoxymethyl)glycoluril, 1,3,4,6-tetrakis(butoxymethyl)glycoluril, 1,3,4,6-tetrakis(hydroxymethyl)glycoluril.

Example of the urea compound include 1,3-bis(hydroxymethyl) urea, 1,1,3,3-tetrakis(butoxymethyl) urea and 1,1,3,3-tetrakis(methoxymethyl) urea.

Examples of the resol resin include a polymer obtained by reacting a compound containing a phenolic hydroxyl group such as phenol, alkyl phenols such as cresol and xylenol, phenylphenol, resorcinol, biphenyl, bisphenol such as bisphenol A and bisphenol F, naphthol and dihydroxynaphthalene, with an aldehyde compound under alkaline catalytic conditions.

Examples of the epoxy resin include liquid epoxy resins such as a bisphenol A epoxy resin, a bisphenol F epoxy resin, a bisphenol S epoxy resin, a bisphenol AD epoxy resin, a polyhydroxybenzene epoxy resin, a polyhydroxynaphthalene epoxy resin, a biphenyl epoxy resin, and tetramethyl biphenyl epoxy resin, brominated epoxy resins such as brominated phenolic novolac epoxy resin, solid bisphenol A epoxy resin, phenolic novolac epoxy resin, and cresol novolac epoxy resin, a triphenylmethane epoxy resin, a tetraphenylethane epoxy resin, a dicyclopentadiene-phenol addition reaction epoxy resin, a phenolic aralkyl epoxy resins, a phenylene ether epoxy resin, a naphthylene ether epoxy resin, a naphthol novolac epoxy resin, a naphthol aralkyl epoxy resin, a naphthol-phenol co-condensed novolac epoxy resin, a naphthol-cresol co-condensed novolac epoxy resin, an aromatic hydrocarbon formaldehyde resin-modified phenolic resin epoxy resin, and a biphenyl-modified novolac epoxy resin, which may be used alone or in combination of two or more kinds thereof, and it is preferable to select various kinds depending on the intended application and physical properties of the cured product.

Examples of the isocyanate compound include tolylene diisocyanate, diphenylmethane diisocyanate, hexamethylene diisocyanate, and cyclohexane diisocyanate.

Examples of the azide compound include 1,1'-biphenyl-4,4'-bisazide, 4,4'-methylidenebisazide, 4,4'-oxybisazide.

Examples of the compound containing a double bond such as the alkenyl ether group include ethylene glycol divinyl ether, triethylene glycol divinyl ether, 1,2-propanediol divinyl ether, 1,4-butanediol divinyl ether, tetramethylene glycol divinyl ether, neopentyl glycol divinyl ether, trimethylolpropane trivinyl ether, hexanediol divinyl ether, 1,4-cyclohexanediol divinyl ether, pentaerythritol trivinyl ether, pentaerythritol tetravinyl ether, sorbitol tetravinyl ether, sorbitol pentavinyl ether, and trimethylol propane trivinyl ether.

Examples of the acid anhydride include aromatic anhydrides such as phthalic anhydride, trimellitic anhydride, pyromellitic anhydride, 3,3',4,4'-benzophenone tetracarboxylic dianhydride, biphenyl tetracarboxylic dianhydride, 4,4'-(isopropylidene)diphthalic anhydride, 4,4'-(hexafluoroisopropylidene)diphthalic anhydride; and alicyclic carboxylic anhydrides such as anhydrous tetrahydrophthalic acid, anhydrous methyl tetrahydrophthalic acid, anhydrous hexahydrophthalic acid, anhydrous methyl hexahydrophthalic acid, anhydrous endomethylenetetrahydrophthalic acid anhydrous dodecenyl succinic acid, anhydrous trialkyltetrahydrophthalic acid.

The concentration of the reversible bond in the curable resin composition of the present invention is preferably 0.10 mmol/g or higher based on the total mass of the curable components in the curable resin composition. According to such a configuration, easy disassembly, and repairability and remoldability of the cured product obtained from the curable resin composition are further improved. The concentration of the reversible bond described above is more preferably between 0.10 and 3.00 mmol/g, and even more preferably between 0.15 and 2.00 mmol/g. The concentration of reversible bond in the present invention can be appropriately selected according to the glass transition temperature or the like of the target cured product as defined by the tan δ peak top of the dynamic viscoelasticity analyzer (DMA). For example, if the glass transition temperature of the cured product is used as a guide, sufficient repairability and remoldability functions are likely to be exhibited even at the low end of the preferred range if the glass transition temperature of the cured product is near room temperature. On the other hand, if the glass transition temperature of the target cured product is above 100°C as a guide, the function is more likely to be exhibited at the high end of the preferred range. However, in the temperature range above the glass transition temperature measured by DMA, molecular mobility is generally high and sufficient repairability and remoldability functions are easily exhibited even when the concentration of the phenolic hydroxyl group-containing compound is low. Therefore, the effect of the repairability and remoldability functions exhibited can also be adjusted by, for example, adjusting the aging temperature for repair and the heating temperature for remolding in a timely manner. Thus, the relationship between the glass transition temperature of the cured product and the concentration of reversible bond is not limited to these factors.

As the compound (I) having reactivity with the phenolic hydroxyl group-containing compound, an epoxy resin is preferably used because the curable resin composition excellent in curability, mechanical strength in a cured product, heat resistance, and the like is obtained.

As the epoxy resin, an epoxy resin represented by the following formula (6) and having an epoxy equivalent weight of 500 to 10000 g/eq may be used. 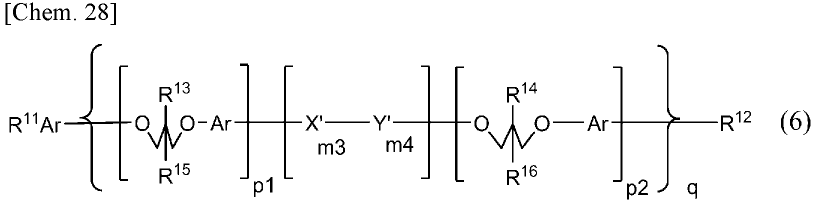 [In the formula (6), Ar's are each independently a structure containing an unsubstituted aromatic ring or an aromatic ring having a substituent,

X' is a structural unit represented by the following general formula (6-1), and Y' is a structural unit represented by the following general formula (6-2), 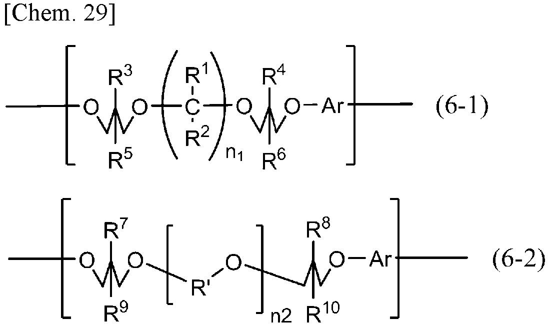 [in the formulas (6-1) and (6-2), Ar is the same as above,
R¹ and R² are each independently a hydrogen atom, a methyl group or an ethyl group,
R' is a divalent hydrocarbon group having 2 to 12 carbon atoms,
R³, R⁴, R⁷, and R⁸ are each independently a hydroxyl group, a glycidyl ether group, or a 2-methylglycidyl ether group,
R⁵, R⁶, R⁹, and R¹⁰ are each independently a hydrogen atom or a methyl group,
n1 is an integer of 2 to 16, and
n2 is an average value of repeating units of 2 to 30.]
R¹¹ and R¹² are each independently a glycidyl ether group or a 2-methylglycidyl ether group,
R¹³ and R¹⁴ are each independently a hydroxyl group, a glycidyl ether group or a 2-methylglycidyl ether group,
R¹⁵ and R¹⁶ are hydrogen atoms or methyl groups,
m3, m4, p1, p2, and q are an average value of repetition number,
m3 and m4 are each independently 0 to 25 and m3 + m4 ≥ 1,
p1 and p2 are each independently 0 to 5, and
q is 0.5 to 5.

Provided that a bond between X' represented by the general formula (6-2) and Y' represented by the general formula (8-3) may be random or blocked, and total numbers of respective structural units X' and Y' present in one molecule are m3 and m4, respectively.]

The epoxy resin represented by the general formula (6) may be alone combined with the phenolic hydroxyl group-containing compound of the present invention to obtain a curable resin, but from the viewpoint of adding more flexibility to the cured product and making it easier to exhibit easy disassembly, it is also preferable to use an epoxy resin with an epoxy equivalent weight of 100 to 300 g/eq in combination.

The epoxy resin that can be used in combination is only required to have an epoxy equivalent weight in the range of 100 to 300 g/eq, and is not limited in the structure thereof. Examples of the epoxy resin include liquid epoxy resins such as a bisphenol A epoxy resin, a bisphenol F epoxy resin, a bisphenol S epoxy resin, a bisphenol AD epoxy resin, a polyhydroxybenzene epoxy resin, a polyhydroxynaphthalene epoxy resin, a biphenyl epoxy resin, and tetramethyl biphenyl epoxy resin, brominated epoxy resins such as brominated phenolic novolac epoxy resin, solid bisphenol A epoxy resin, phenolic novolac epoxy resin, cresol novolac epoxy resin, a triphenylmethane epoxy resin, a tetraphenylethane epoxy resin, a dicyclopentadiene-phenol addition reaction epoxy resin, a phenolic aralkyl epoxy resins, a phenylene ether epoxy resin, a naphthylene ether epoxy resin, a naphthol novolac epoxy resin, a naphthol aralkyl epoxy resin, a naphthol-phenol co-condensed novolac epoxy resin, a naphthol-cresol co-condensed novolac epoxy resin, an aromatic hydrocarbon formaldehyde resin-modified phenolic resin epoxy resin, and a biphenyl-modified novolac epoxy resin, which may be used alone or in combination of two or more kinds thereof, and it is preferable to select various kinds depending on the intended application and physical properties of the cured product.

Among these, it is preferable to use an epoxy resin having an epoxy equivalent weight of 100 to 300 g/eq among liquid epoxy resins such as bisphenol A epoxy resin, bisphenol F epoxy resin, bisphenol S epoxy resin, bisphenol AD epoxy resin, polyhydroxybenzene epoxy resin, polyhydroxynaphthalene epoxy resin, biphenyl epoxy resin, and tetramethylbiphenyl epoxy resin, and it is particularly preferable to use an epoxy resin having an epoxy equivalent weight of 100 to 300 g/eq among bisphenol A epoxy resin, bisphenol F epoxy resin, bisphenol S epoxy resin, and bisphenol AD epoxy resin.

The use ratio of the epoxy resin represented by the general formula (6) and the epoxy resin having the epoxy equivalent weight of 100 to 300 g/eq is not particularly limited, but from the viewpoint of easy phase separation in the cured product, the mass ratio of the former to the latter is 97:3 to 3:97, preferably 10:90 to 90: 10, and particularly preferably 80:20 to 20:80. Phase separation in the cured product results in a sea-island structure, which has both adhesion and stress relaxation properties in the cured product, exhibits especially high adhesion over a wide temperature range, and has the effect of reducing mold shrinkage before and after heat curing of the resin composition.

Furthermore, when the phenolic hydroxyl group-containing compound of the present invention is combined with an epoxy resin to make a curable resin composition, a curing agent for an epoxy resin may be blended.

Examples of the curing agent that can be used here include various known curing agents for epoxy resins, such as an amine compound, an acid anhydride, an amide compound, a phenolic hydroxyl group-containing compound, a carboxylic acid compound, and a thiol compound.

Examples of the amine compound include aliphatic amine compounds such as trimethylenediamine, ethylenediamine, N,N,N',N'-tetramethylethylenediamine, pentamethyldiethylenetriamine, triethylenediamine, dipropylenediamine, N,N,N',N'-tetramethylpropylenediamine, tetramethylenediamine, pentanediamine, hexamethylenediamine, trimethylhexamethylenediamine, N,N,N',N'-tetramethylhexamethylenediamine, N,N-dimethylcyclohexylamine, diethylenetriamine, triethylenetetramine, tetraethylenepentamine, dimethylaminopropylamine, diethylaminopropylamine, dibutylaminopropylamine, 1,4-diazabicyclo(2,2,2)octane (triethylenediamine), polyoxyethylenediamine, polyoxypropylenediamine, bis(2-dimethylaminoethyl) ether, dimethylaminoethoxyethanol, triethanolamine, dimethylaminohexanol, and benzylmethylamine, dimethylbenzylamine, m-xylenediamine, alpha-methylbenzylmethylamine;

Alicyclic and heterocyclic amine compounds such as piperidine, piperazine, mentandiamine, isophoronediamine, methylmorpholine, ethylmorpholine, N,N',N"-tris(dimethylaminopropyl)hexahydro-s-triazine, 3,9-bis(3-aminopropyl)-2,4,8,10-tetraoxyspiro(5,5)undecane adduct, N-aminoethylpiperazine, trimethylaminoethylpiperazine, bis(4-aminocyclohexyl)methane, and N,N'-dimethylpiperazine, 1,8-diazabicyclo-[5.4.0]-undecene (DBU);

Aromatic amine compounds such as o-phenylenediamine, m-phenylenediamine, p-phenylenediamine, diaminodiphenylmethane, diaminodiphenylsulfone, pyridine, picoline;

Modified amine compounds such as an epoxy compound addition polyamine, a Michael addition polyamine, a Mannich addition polyamine, a thiourea addition polyamine, a ketone blocked polyamine, dicyandiamide, guanidine, an organic acid hydrazide, diaminomaleonitrile, an amine imide, a boron trifluoride/piperidine complex, and a boron trifluoride-monoethylamine complex.

Specific examples of the acid anhydride include phthalic anhydride, trimellitic anhydride, pyromellitic anhydride, maleic anhydride, maleic anhydride polypropylene glycol, terahydrophthalic anhydride, methyl tetrahydro phthalic anhydride, methylnadic anhydride, hexahydrophthalic anhydride, and methyl hexahydro phthalic anhydride.

Examples of the phenolic hydroxyl group-containing compound include bisphenols such as bis(4-hydroxyphenyl)methane, 2,2-bis(4-hydroxyphenyl)propane, 2,2-bis(3-methyl-4-hydroxyphenyl)propane, 1,1-bis(4-hydroxyphenyl)cyclohexane, and 1,1-bis(4-hydroxyphenyl)-1-phenylethane, and bis(4-hydroxyphenyl) sulfone, a phenolic novolac resin, a cresol novolac resin, an aromatic hydrocarbon formaldehyde resin modified phenolic resin, a dicyclopentadiene phenol addition resin, a phenol aralkyl resin (zylock resin), a naphthol aralkyl resin, a trimethylol methane resin, a tetraphenylol ethane resin, a naphthol novolac resin, a naphthol-phenol co-condensed novolac resin, a naphthol-cresol co-condensed novolac resin, a biphenyl-modified phenol resin (polyvalent phenol compound with phenol core linked by bis-methylene groups), a biphenyl-modified naphthol resin (polyvalent naphthol compound with phenol nucleus linked by bismethylene groups), an aminotriazine-modified phenolic resin (polyvalent phenol compound with phenol nucleus linked by melamine, benzoguanamine, and the like), and an alkoxy group-containing aromatic ring modified novolac resin (polyvalent phenolic compound in which the phenol nucleus and alkoxy group-containing aromatic ring are linked by formaldehyde).

Examples of the amide compound include dicyandiamide and polyamide amine. Examples of the polyamide amine include those obtained by reacting aliphatic dicarboxylic acids such as succinic acid, glutaric acid, adipic acid, pimelic acid, suberic acid, and azelaic acid, or carboxylic acid compounds such as fatty acids and dimer acids with aliphatic polyamines or polyamines with polyoxyalkylene chains.

Examples of the carboxylic acid compound include a carboxylic acid polymer such as a carboxylic acid terminal polyester, a polyacrylic acid, and a maleic acid-modified polypropylene glycol.

The thiol compound is preferably one that contains two or more thiol groups in one molecule. Examples of the thiol compound include 3,3'-dithiodipropionic acid, trimethylolpropane tris(thioglycolate), pentaerythritol tetrakis(thioglycolate), ethylene glycol dithioglycolate, 1,4-bis(3-mercaptobutyryloxy)butane, tris[(3-mercaptopropionyloxy)-ethyl] - isocyanurate, trimethylolpropane tris(3-mercaptopropionate), pentaerythritol tetrakis(3-mercaptopropionate), pentaerythritol tetrakis(3-mercaptobutyrate), dipentaerythritol hexakis(3-mercaptopropionate), 1,3,4,6-tetrakis(2-mercaptoethyl)glycoluril, 4-butanedithiol, 1,6-hexanedithiol, and 1,10-decanedithiol.

When using these curing agents, only one type of curing agent may be used, or two or more types may be mixed. For applications such as underfill materials and general coating applications, it is preferable to use the amine, carboxylic acid, and or acid anhydride compounds. In addition, for adhesive and flexible wiring board applications, amine compounds, especially dicyandiamide, are preferable from the viewpoint of workability, curability, and long-term stability. Further, for semiconductor encapsulation material applications, solid-type phenolic compounds are preferable from the viewpoint of heat resistance of the cured product. In addition, for battery applications, aliphatic amines and thiol compounds are preferable from the viewpoint of low-temperature curing.

The amount of the epoxy resin used and the amount of the curing agent used are not particularly limited, but from the viewpoint of good mechanical properties of the resulting cured product, and the like, the amount of active groups that can react with the epoxy groups is preferably 0.4 to 1.5 equivalents per 1 equivalent of total of epoxy groups in the resin composition.

In addition, when an epoxy resin is used, a curing accelerator may be contained. Various types of curing accelerators can be used, and examples thereof include urea compounds, phosphorus compounds, tertiary amines, imidazoles, imidazolines, organic acid metal salts, Lewis acids, and amine complex salts. When used for adhesive applications, from the viewpoint of excellent workability and low-temperature curing properties, a urea compound, particularly 3-(3,4-dichlorophenyl)-1,1-dimethylurea (DCMU), is preferable. For use in semiconductor encapsulation material applications, from the viewpoint of excellent curability, heat resistance, electrical properties, moisture resistance reliability, and the like, triphenylphosphine is preferable among phosphorus compounds, and 1,8-diazabicyclo-[5.4.0]-undecene is preferable among tertiary amines.

Examples of the phosphorus compound include alkyl phosphine such as ethyl phosphine and butyl phosphine; primary phosphine such as phenyl phosphine; dialkyl phosphine such as dimethyl phosphine and dipropyl phosphine; secondary phosphine such as diphenyl phosphine and methyl ethyl phosphine; and tertiary phosphine such as trimethyl phosphine, triethyl phosphine, and triphenyl phosphine.

Examples of the imidazole include imidazole, 1-methylimidazole, 2-methylimidazole, 3-methylimidazole, 4-methylimidazole, 5-methylimidazole, 1-ethylimidazole, 2-ethylimidazole, 3-ethylimidazole, 4-ethylimidazole, 5-ethylimidazole, 1-n-propylimidazole, 2-n-propylimidazole, 1-isopropylimidazole, 2-isopropylimidazole, 1-n-butylimidazole, 2-n-butylimidazole, 1-isobutyl imidazole, 2-isobutyl-imidazole, 2-undecyl-1H-imidazole, 2-heptadecyl-1H-imidazole, 1,2-dimethylimidazole, 1,3-dimethylimidazole, 2,4-dimethylimidazole, 2-ethyl-4-methylimidazole, 1-phenyl imidazole, 2-phenyl-1H-imidazole, 4-methyl-2-phenyl-1H-imidazole, 2-phenyl-4-methylimidazole, 1-benzyl-2-methylimidazole, 1-benzyl-2-phenyl imidazole, 1-cyanoethyl-2-methylimidazole, 1-cyanoethyl-2-ethyl-4-methylimidazole, 1-cyanoethyl-2-undecylimidazole, 1-cyanoethyl-2-phenyl-imidazole, 2-phenyl-imidazole isocyanuric acid adduct, 2-methylimidazole isocyanuric acid adduct, 2-phenyl-4,5-dihydroxymethylimidazole, 2-phenyl-4-methyl-5-hydroxymethylimidazole, 1-cyanoethyl-2-phenyl-4,5-di (2-cyanoethoxy) methylimidazole, 1-dodecyl-2-methyl-3-benzylimidazolium chloride, and 1-benzyl-2-phenyl imidazole hydrochloride.

Examples of the imidazoline compound include 2-methylimidazoline and 2-phenylimidazoline.

Examples of the urea compound include p-chlorophenyl-N,N-dimethyl urea, 3-phenyl-1,1-dimethyl urea, 3-(3,4-dichlorophenyl)-N,N-dimethyl urea, and N-(3-chloro-4-methylphenyl)-N',N'-dimethyl urea.

In the curable resin composition of the present invention, other thermosetting resins and thermoplastic resins may be used in combination as long as the effects of the present invention are not impaired.

Examples of other thermosetting resins include cyanate ester resins, resins having a benzoxazine structure, active ester resins, vinyl benzyl compounds, acrylic compounds, and copolymers of styrene and maleic anhydride. When the above-described other thermosetting resins are used in combination, the amount thereof is not particularly limited as long as the effects of the present invention are not impaired, but is preferably in the range of 1 to 50 parts by mass in 100 parts by mass of the curable resin composition.

Examples of the cyanate ester resin include bisphenol A type cyanate ester resin, bisphenol F type cyanate ester resin, bisphenol E type cyanate ester resin, bisphenol S type cyanate ester resin, bisphenol sulfide-type cyanate ester resin, phenylene ether-type cyanate ester resin, naphthylene ether type cyanate ester resin, biphenyl type cyanate ester resin, tetramethylbiphenyl type cyanate ester resin, polyhydroxynaphthalene type cyanate ester resin, phenolic novolac type cyanate ester resin, cresol novolac type cyanate ester resin, triphenylmethane type cyanate ester resin, tetraphenylethane type cyanate ester resin, dicyclopentadiene-phenol addition-reaction type cyanate ester resin, phenol-aralkyl type cyanate ester resin, naphthol novolac type cyanate ester resin, naphthol aralkyl type cyanate ester resin, naphthol-phenol co-condensed novolac type cyanate ester resin, naphthol-cresol co-condensed novolac type cyanate ester resin, aromatic hydrocarbon formaldehyde resin modified phenolic resin type cyanate ester resin, biphenyl-modified novolac type cyanate ester resin, and anthracene type cyanate ester resin. Each of these resins may be used alone, or two or more kinds thereof may be used in combination.

Among these cyanate ester resins, particularly from the viewpoint of obtaining a cured product with excellent heat resistance, it is preferable to use a bisphenol A cyanate ester resin, a bisphenol F cyanate ester resin, a bisphenol E cyanate ester resin, a polyhydroxynaphthalene cyanate ester resin, a naphthylene ether cyanate ester resin, and a novolac cyanate ester resin, and from the viewpoint of obtaining a cured product with excellent dielectric properties, a dicyclopentadiene-phenol addition-reaction type cyanate ester resin is preferable.

The resin having a benzoxazine structure is not particularly limited, and examples thereof include reaction products of bisphenol F, formalin, and aniline (F-a type benzoxazine resin), reaction products of diaminodiphenylmethane and formalin and phenol (P-d type benzoxazine resin), reaction products of bisphenol A, formalin, and aniline, reaction products of dihydroxydiphenyl ether, formalin, and aniline, reaction products of diaminodiphenyl ether, formalin, and phenol, reaction products of dicyclopentadiene-phenol addition-type resin, formalin, and aniline, reaction products of phenolphthalein, formalin, and aniline, reaction products of diphenyl sulfide, formalin, and aniline. Each of these resins may be used alone, or two or more kinds thereof may be used in combination.

The active ester resin is not particularly limited, but in general, compounds having, in one molecule, two or more ester groups with high reactivity, such as phenol esters, thiophenol esters, N-hydroxyamine esters, and esters of heterocyclic hydroxy compounds are preferably used. The active ester resin is preferably that obtained by condensation reaction of a carboxylic acid compound and/or thiocarboxylic acid compound with a hydroxy compound and/or thiol compound. In particular, from the viewpoint of improving heat resistance, active ester resins obtained from carboxylic acid compounds or their halides and hydroxy compounds are preferable, and active ester resins obtained from carboxylic acid compounds or their halides and phenolic and/or naphthol compounds are more preferable. Examples of the carboxylic acid compound include benzoic acid, acetic acid, succinic acid, maleic acid, itaconic acid, phthalic acid, isophthalic acid, terephthalic acid, pyromellitic acid, or halides thereof. Examples of the phenol compound or the naphthol compound include hydroquinine, resorcin, bisphenol A, bisphenol F, bisphenol S, dihydroxydiphenyl ether, phenolphthalein, methylated bisphenol A, methylated bisphenol F, methylated bisphenol S, phenol, o-cresol, m-cresol, p-cresol, catechol, α-naphthol, β-naphthol, 1,5-dihydroxynaphthalene, 1,6-dihydroxynaphthalene, 2,6-dihydroxynaphthalene, dihydroxybenzophenone, trihydroxybenzophenone, tetrahydroxybenzophenone, phloroglucin, benzene triol, and dicyclopentadiene-phenol addition resin.

As the active ester resin, specifically, an active ester resin containing a dicyclopentadiene-phenol addition structure, an active ester resin containing a naphthalene structures, an active ester resin that is an acetylated phenolic novolac, an active ester resin that is a benzoylated phenolic novolac, and the like are preferable, and among these, an active ester resin containing a dicyclopentadiene-phenol addition structure and an active ester resin containing a naphthalene structure are more preferable from the viewpoint of superior improvement in peel strength.

Furthermore, various novolac resins, addition polymerization resins of alicyclic diene compounds such as dicyclopentadiene and phenolic compounds, modified novolac resins of phenolic hydroxyl group-containing compounds and aromatic compounds containing alkoxy groups, phenolic aralkyl resins (zylock resin), naphthol aralkyl resins, trimethylolmethane resins, tetraphenylol ethane resins, biphenyl-modified phenolic resins, biphenyl-modified naphthol resins, aminotriazine-modified phenolic resins, and various vinyl polymers may be used in combination.

More specifically, examples of the various novolac resins include polymers obtained by reacting a phenol, a phenyl phenol, a resorcinol, a biphenyl, a bisphenol such as bisphenol A or bisphenol F, a phenolic hydroxyl group-containing compound such as naphthol or dihydroxynaphthalene, and an aldehyde compound under an acid catalyst condition.

Examples of the various types of vinyl polymers include homopolymers or copolymers of vinyl compounds such as poly hydroxy styrene, polystyrene, polyvinyl naphthalene, polyvinyl anthracene, polyvinyl carbazole, polyindene, polyacenaphthylene, polynorbornene, polycyclodecene, polytetracyclododecene, polynortricyclene, and poly (meth) acrylate.

The thermoplastic resin refers to a resin that can be melt molded by heating. Specific examples of the thermoplastic resin include a polyethylene resin, a polypropylene resin, a polystyrene resin, a rubber-modified polystyrene resin, an acrylonitrile-butadienestyrene (ABS) resin, an acrylonitrile-styrene (AS) resin, a polymethyl methacrylate resin, an acrylic resin, a polyvinyl chloride resin, a polyvinylidene chloride resin, a polyethylene terephthalate resin, an ethylene vinyl alcohol resin, an acetic acid cellulose resin, an ionomer resin, a polyacrylonitrile resin, a polyamide resin, a polyacetal resin, a polybutylene terephthalate resin, a polylactic acid resin, a polyphenylene ether resin, a modified polyphenylene ether resin, a polycarbonate resin, a polysulfone resin, a polyphenylene sulfide resin, a polyether imide resin, a polyether sulfone resin, a polyarylate resin, a thermoplastic polyimide resin, a polyamide imide resin, a polyether ether ketone resin, a polyketone resin, a liquid crystal polyester resin, a fluororesin, a syndiotactic polystyrene resin, and a cyclic polyolefin resin. These thermoplastic resins can be used alone or in combination of two or more kinds thereof.

When these other resins are used, the mixing ratio of the phenolic hydroxyl group-containing compound of the present invention and other resins can be set as desired depending on the application, but from the viewpoint of not interfering with the repairability and remoldability that the present invention achieves, the ratio is preferably 0.5 to 100 parts by mass of other resins to 100 parts by mass of the phenolic hydroxyl group-containing compound of the present invention.

Further, a curing accelerator may be used in combination with the curable resin composition of the present invention. Examples of the curing accelerator include tertiary amine compounds such as imidazole and dimethylamino pyridine; phosphorus compounds such as triphenyl phosphine; boron trifluoride and boron trifluoride amine complexes such as boron trifluoride monoethylamine complex; organic acid compounds such as thiodipropionic acid; benzoxazine compounds such as thiodiphenol benzoxazine and sulfonylbenzoxazine; and sulfonyl compounds. Each of these may be used alone, or two or more kinds thereof may be used in combination. The amount of these catalysts added is preferably in the range of 0.001 to 15 parts by mass in 100 parts by mass of the curable resin composition.

In addition, when the curable resin composition of the present invention is used for applications that require high flame retardancy, non-halogen flame retardants that contain virtually no halogen atoms may be blended.

Examples of the non- halogen flame retardant include phosphorus-based flame retardants, nitrogen-based flame retardants, silicone-based flame retardants, inorganic flame retardants, organic metal salt flame retardants, the use of these flame retardants is not limited in any way, and the flame retardant may be used alone or in combination with other flame retardants of the same system, or in a combination with flame retardants of different systems.

As the phosphorus-based flame retardant, either an inorganic or organic one can be used. Examples of the inorganic compound include red phosphorus, ammonium phosphate such as ammonium phosphate, diammonium phosphate, triammonium phosphate, and ammonium polyphosphate, and inorganic nitrogen-containing phosphorus compounds such as phosphoric acid amide.

In addition, the surface treatment is preferably performed for the red phosphorus for the purpose of preventing hydrolysis and the like, and examples of the surface treatment method include (i) a method for coating with an inorganic compound such as magnesium hydroxide, aluminum hydroxide, zinc hydroxide, titanium hydroxide, bismuth oxide, bismuth hydroxide, bismuth nitrate or mixtures thereof, (ii) a method for coating with a mixture of an inorganic compound such as magnesium hydroxide, aluminum hydroxide, zinc hydroxide, or titanium hydroxide, and a thermosetting resin such as phenol resin, and (iii) a method for double coating with a thermosetting resin such as phenol resin on top of a film of an inorganic compound such as magnesium hydroxide, aluminum hydroxide, zinc hydroxide, or titanium hydroxide.

Examples of the organophosphorus compounds include, in addition to general-purpose organophosphorus compounds such as phosphate compounds, phosphonic acid compounds, phosphinic acid compounds, phosphine oxide compounds, phosphorane compounds, and organic nitrogen-containing phosphorus compounds, cyclic organophosphorus compounds such as 9,10-dihydro-9-oxa-10-phosphaphenanthrene-10-oxide, 10-(2,5-dihydroxyphenyl)-10H-9-oxa-10-phosphaphenanthrene-10-oxide, and 10-(2,7-dihydroxynaphthyl)-10H-9-oxa-10-phosphaphenanthrene-10-oxide and derivatives made by reacting them with compounds such as epoxy and phenolic resins.

Although the blending amount of the phosphorus-based flame retardant is appropriately selected depending on the type of the phosphorus-based flame retardant, the other components of the resin composition, and the desired degree of flame retardancy, for example, when the red phosphorus is used as the non-halogen flame retardant in 100 parts by mass of the resin composition containing a non-halogen flame retardant and all other components such as a filler, an additive, and the like blended therein, the amount is preferably in the range of 0.1 to 2.0 parts by mass, and when an organophosphorus compound is used, the amount is preferably in the range of 0.1 to 10.0 parts by mass and is more preferably in the range of 0.5 to 6.0 parts by mass.

In addition, when using the phosphorus-based flame retardant, hydrotalcite, magnesium hydroxide, boron compound, zirconium oxide, black dye, calcium carbonate, zeolite, zinc molybdate, activated carbon, or the like may be used in combination with the phosphorus-based flame retardant.

Examples of the nitrogen-based flame retardant include a triazine compound, a cyanuric acid compound, an isocyanuric acid compound, and phenothiazine, and a triazine compound, a cyanuric acid compound, and an isocyanuric acid compound are preferable.

Examples of the triazine compound include, in addition to melamine, acetoguanamine, benzoguanamine, melon, melam, succinoguanamine, ethylene dimelamine, melamine polyphosphate, and triguanamine, for example, (1) aminotriazine sulfate compounds such as guanylmelamine sulfate, melem sulfate, and melam sulfate, (2) co-condensates of phenols such as phenol, cresol, xylenol, butylphenol, nonylphenol, etc., with melamines such as melamine, benzoguanamine, acetoguanamine, formoguanamine, and formaldehyde, (3) mixtures of the co-condensates of (2) above with phenol resins such as phenol-formaldehyde condensates, (4) a product obtained by further modifying (2) and (3) with paulownia oil, isomerized linseed oil, and the like.

Examples of the cyanuric acid compound include cyanuric acid and cyanuric acid melamine.

The amount of nitrogen-based flame retardant to be blended is appropriately selected according to the type of nitrogen-based flame retardant, other components of the resin composition, and the desired degree of flame retardancy, but for example, it is preferable to blend in the range of 0.05 to 10 parts by mass in 100 parts by mass of the resin composition containing a non-halogen flame retardant and all other components such as a filler, an additive, and the like blended therein, and it is more preferable to blend in the range of 0.1 to 5 parts by mass.

In addition, when using the nitrogen-based flame retardant, metal hydroxides, molybdenum compounds, or the like may be used in combination.

The silicone-based flame retardant can be used with no particular restrictions as long as the silicone-based flame retardant is an organic compound containing silicon atoms. Examples of the silicone-based flame retardant include silicone oil, silicone rubber, and silicone resin. The amount of the silicone-based flame retardant to be blended is selected according to the type of silicone-based flame retardant, other components of the resin composition, and the desired degree of flame retardancy, but for example, it is preferable to blend in the range of 0.05 to 20 parts by mass in 100 parts by mass of the resin composition containing a non-halogen flame retardant and all other components such as a filler, an additive, and the like blended therein. In addition, when using the silicone-based flame retardant, a molybdenum compound, alumina, or the like may be used in combination.

Examples of the inorganic flame retardant include a metal hydroxide, a metal oxide, a metal carbonate compound, a metal powder, a boron compound, and a low melting point glass.

Examples of the metal hydroxide include aluminum hydroxide, magnesium hydroxide, dromite, hydrotalcite, calcium hydroxide, barium hydroxide, and zirconium hydroxide.

Examples of the metal oxide include zinc molybdate, molybdenum trioxide, zinc stannate, tin oxide, aluminum oxide, iron oxide, titanium oxide, manganese oxide, zirconium oxide, zinc oxide, molybdenum oxide, cobalt oxide, bismuth oxide, chromium oxide, nickel oxide, copper oxide, and tungsten oxide.

Examples of the metal carbonate compound include zinc carbonate, magnesium carbonate, calcium carbonate, barium carbonate, basic magnesium carbonate, aluminum carbonate, iron carbonate, cobalt carbonate, and titanium carbonate.

Examples of the metal powder include aluminum, iron, titanium, manganese, zinc, molybdenum, cobalt, bismuth, chromium, nickel, copper, tungsten, and tin.

Examples of the boron compound include zinc borate, zinc metaborate, barium metaborate, boric acid, and borax.

Examples of the low melting point glass include glass-like compounds such as Ceepree (Bokusui Brown Co., Ltd.), hydrated glass SiO₂-MgO-H₂O, and PbO-B₂O₃ based, ZnO-P₂O₅-MgO based, P₂O₅-B₂O₃-PbO-MgO based, P-Sn-O-F based, PbO-V₂O₅-TeO₂ based, Al₂O₃-H₂O based, and lead borosilicate based ones.

The amount of the inorganic flame retardant to be blended is selected according to the type of inorganic flame retardant, other components of the resin composition, and the desired degree of flame retardancy, but for example, it is preferable to blend in the range of 0.05 to 20 parts by mass in 100 parts by mass of the resin composition containing a non-halogen flame retardant and all other components such as a filler, an additive, and the like blended therein, and it is more preferable to blend in the range of 0.5 to 15 parts by mass.

Examples of the organic metal salt flame retardant include ferrocene, acetylacetonate metal complexes, organometallic carbonyl compounds, organocobalt salt compounds, organosulfonic acid metal salts, compounds in which metal atoms and aromatic or heterocyclic compounds are ionically or coordinately bonded.

The amount of the organic metal salt flame retardant is selected according to the type of organic metal salt flame retardant, other components of the resin composition, and the desired degree of flame retardancy, but for example, it is preferable to blend in the range of 0.005 to 10 parts by mass in 100 parts by mass of the resin composition containing a non-halogen flame retardant and all other components such as a filler, an additive, and the like blended therein.

The curable resin composition of the present invention may contain a filler. Examples of the filler include an inorganic filler and an organic filler. Examples of the inorganic filler include inorganic fine particles.

As the inorganic fine particles, examples of those with excellent heat resistance include, for example, alumina, magnesia, titania, zirconia, silica (quartz, fumed silica, precipitated silica, silica anhydride, fused silica, crystalline silica, ultrafine amorphous silica, and the like), and the like; examples of those with excellent heat conduction include boron nitride, aluminum nitride, alumina oxide, titanium oxide, magnesium oxide, zinc oxide, silicon oxide, diamond, and the like; examples of those with excellent conductivity includes a metal filler and/or a metal-coated filler made of single metals or alloys (for example, iron, copper, magnesium, aluminum, gold, silver, platinum, zinc, manganese, stainless steel, and the like); examples of those with excellent barrier properties include minerals such as mica, clay, kaolin, talc, zeolite, wollastonite, smectite, and the like, potassium titanate, magnesium sulfate, sepiolite, zonolite, aluminum borate, calcium carbonate, titanium oxide, barium sulfate, zinc oxide, magnesium hydroxide; examples of those with high refractive index include barium titanate, zirconium oxide, titanium oxide, and the like; examples of those with photocatalytic properties includes photocatalytic metals such as titanium, cerium, zinc, copper, aluminum, tin, indium, phosphorus, carbon, sulfur, ruthenium, nickel, iron, cobalt, silver, molybdenum, strontium, chromium, barium, and lead, composites of the above metals, and oxides thereof; examples of those with excellent wear resistance include metals such as silica, alumina, zirconia, and magnesium oxide, and composites and oxides thereof; examples of those with excellent conductivity include metals such as silver and copper, tin oxide, indium oxide, and the like; examples of those with excellent insulating properties include silica and the like; examples of those with excellent UV shielding include titanium oxide, zinc oxide, and the like. These inorganic fine particles are only required to be selected depending on the application in a timely manner, and can be used alone or in combination of two or more kinds thereof. In addition to the properties listed in the examples, the above inorganic fine particles have various other properties and thus are only required to be selected depending on the application in a timely manner.

For example, when silica is used as inorganic fine particles, there are no particular limitations, and known silica fine particles such as powdered silica and colloidal silica can be used. Examples of commercially available powdered silica fine particles include Aerosil 50 and 200 (trade names, manufactured by Nippon Aerosil Co., Ltd.), Sildex H31, H32, H51, H52, H121, and H122 (trade names, manufactured by AGC Inc.), E220A and E220 (trade names, manufactured by Nippon Silica Industrial Co., Ltd.), SYLYSIA 470 (trade name, manufactured by Fuji Silysia Co., Ltd.), and SG-flake (trade name, manufactured by Nippon Sheet Glass Co., Ltd.).

Examples of commercially available colloidal silica include methanol-silica sol, IPA-ST, MEK-ST, NBA-ST, XBA-ST, DMAC-ST, ST-UP, ST-OUP, ST-20, ST-40, ST-C, ST-N, ST-O, ST-50, and ST-OL manufactured by Nissan Chemical Corporation.

Surface-modified silica fine particles may be used, for example, there are silica fine particles that are surface treated with a reactive silane coupling agent having hydrophobic groups or modified with a compound having (meth)acryloyl groups. Examples of commercially available powdered silica modified with a compound having (meth)acryloyl groups include Aerosil RM50 and R711 manufactured by Nippon Aerosil Co., Ltd., and examples of commercially available colloidal silica modified with compounds having (meth)acryloyl groups include MIBK-SD manufactured by Nissan Chemical Corporation.

The shape of the silica fine particles is not limited and spherical, hollow, porous, rod-shaped, plate-shaped, fibrous, or irregularly shaped ones can be used. The primary particle diameter is preferably in the range of 5 to 200 nm.

As titanium oxide fine particles, not only extender pigments but also ultraviolet light responsive photocatalysts can be used, for example, anatase titanium oxide, rutile titanium oxide, and brookite titanium oxide can be used. Furthermore, particles designed to respond to visible light by doping different elements into the crystal structure of titanium oxide can also be used. Anionic elements such as nitrogen, sulfur, carbon, fluorine, and phosphorus, and cationic elements such as chromium, iron, cobalt, and manganese are suitably used as elements to be doped into titanium oxide. In addition, as the form thereof, a powder, a sol or slurry dispersed in an organic solvent or in water can be used. Examples of commercially available powdered titanium oxide fine particles include Aerosil P-25 manufactured by Nippon Aerosil Co., Ltd., and ATM-100 manufactured by Tayca Corporation. In addition, examples of commercially available slurry-like titanium oxide fine particles include TKD-701 manufactured by Tayca Corporation.

The curable resin composition of the present invention may further contain a fibrous substrate. The fibrous substrate is not particularly limited, but is preferably one used for a fiber-reinforced resin, and examples thereof include inorganic fibers and organic fibers.

Examples of the inorganic fiber include, in addition to inorganic fibers such as carbon fibers, glass fibers, boron fibers, alumina fibers, and silicon carbide fibers, carbon fibers, activated carbon fibers, graphite fibers, tungsten carbide fibers, silicon carbide fibers (silicon carbide fibers), ceramic fibers, natural fibers, and fibers of minerals such as basalt, boron nitride fibers, boron carbide fibers, and metal fibers. Examples of the metal fibers include aluminum fibers, copper fibers, brass fibers, stainless steel fibers, and steel fibers.

Examples of the organic fiber include synthetic fibers made of a resin material such as polybenzazole, aramid, PBO (polyparaphenylene benzoxazole), polyphenylene sulfide, polyester, acrylic, polyamide, polyolefin, polyvinyl alcohol, and polyarylate, and natural fibers such as cellulose, pulp, cotton, wool, and silk, and regenerated fibers such as those made from proteins, polypeptides, and alginic acid.

Among these, carbon and glass fibers are particularly preferred because of the wide range of industrial applications. Among these, only one type may be used, or multiple types may be used at the same time.

The fibrous substrate may be an aggregate of fibers, and the fibers may be continuous or discontinuous, woven or non-woven. In addition, the fibrous substrate can also be a fiber bundle with fibers aligned in one direction or a sheet-shaped one obtained by arranging fiber bundles. Further, the fibrous substrate can also be a three-dimensional shape obtained by increasing the thickness of the aggregate of fibers.

The curable resin composition of the present invention may use a dispersion medium for the purpose of adjusting the solid content and viscosity of the resin composition. The dispersion medium can be any liquid medium that does not impair the effects of the present invention, and examples thereof include various organic solvents, liquid organic polymers, and the like.

Examples of the organic solvent include ketones such as acetone, methyl ethyl ketone (MEK), methyl isobutyl ketone (MIBK), cyclic ethers such as tetrahydrofuran (THF) and dioxolan, esters such as methyl acetate, ethyl acetate, butyl acetate, aromatics such as toluene and xylene, and alcohols such as carbitol, cellosolve, methanol, isopropanol, butanol, propylene glycol monomethyl ether, which may used alone or in combination, and among these, methyl ethyl ketone is preferable from the viewpoint of volatility during coating and solvent recovery.

The liquid organic polymer is a liquid organic polymer which does not directly contribute to a curing reaction, and examples thereof include an acrylic polymer (FLOWLEN WK-20: manufactured by Kyoeisha Chemical Co., Ltd.), an amine salt of a special modified phosphate ester (HIPLAAD ED-251: Kusumoto Chemicals, Ltd.), and a modified acrylic block copolymer (DISPERBYK 2000; manufactured by BYK Chemie).

The resin composition of the present invention may have other formulations. Examples of the formulations include a catalyst, a polymerization initiator, an inorganic pigment, an organic pigment, an extender pigment, a clay mineral, a wax, a surfactant, a stabilizer, a flow regulating agent, a coupling agent, a dye, a leveling agent, a rheology control agent, an ultraviolet light absorbent, an antioxidant, a flame retardant, a plasticizer, and a reactive diluent.

By curing the resin composition of the present invention, a cured product can be obtained. Curing can be performed at room temperature or by heating. When performing thermal curing, curing may be done in a single heating step or through a multi-step heating process.

In addition, the curable resin compositions of the present invention can also be cured with active energy rays. In this case, a photocationic polymerization initiator can be used as a polymerization initiator. Visible light, ultraviolet light, X-rays, electron beams, or the like can be used as active energy rays.

Examples of the photocationic polymerization initiator include aryl-sulfonium salts, aryl-iodonium salts, and specifically, arylsulfonium hexafluorophosphate, arylsulfonium hexafluoroantimonate, arylsulfonium tetrakis(pentafluoro)borate, tri(alkylphenyl)sulfonium hexafluorophosphate, and the like can be used. The photocationic polymerization initiator may be used alone or in combination with two or more kinds thereof.

The curable resin composition of the present invention may be prepared by uniformly mixing the above-described components, and the method for mixing is not particularly limited. For example, the curable resin composition can be prepared by performing uniform mixing using a pot mill, ball mill, bead mill, roll mill, homogenizer, super mill, homogenizing disper, universal mixer, Banbury mixer, kneader, and the like.

For the curable resin composition of the present invention, the phenolic hydroxyl group-containing compound of the present invention and a compound (I) having reactivity with the phenolic hydroxyl group-containing compound, as well as the curing agents that can be used in combination, fillers, fibrous substrates, dispersion medium, resins other than the various compounds mentioned above, as needed, are dissolved in the dispersion medium such as organic solvents mentioned above. After dissolution, the curable resin composition can be obtained by removing the solvent and performing decompression drying in a vacuum oven, and the like. The curable resin composition of the present invention may be in a state in which the above-described constituent materials are uniformly mixed. At this time, the mixture is preferably uniformly mixed with a mixer or the like. The mixing ratio of each of the constituent materials can be appropriately adjusted depending on the desired properties of the cured product, such as mechanical strength, heat resistance, repairability, and remoldability. In addition, in the production of the curable resin composition, the specific order of mixing of the constituent materials is not particularly limited.

The cured product of the present invention is obtained by curing the compound (I) having reactivity with the phenolic hydroxyl group-containing compound by the phenolic hydroxyl group-containing compound of the present invention. The curing method can be appropriately selected from known methods depending on the properties of the compound (I) having reactivity with the phenolic hydroxyl group-containing compound to be used.

Since the cured product of the present invention is cured by the phenolic hydroxyl group-containing compound of the present invention as described above, it is possible to develop an appropriate crosslinking density and thereby maintain good mechanical strength. In addition, when the cured product of the present invention is subjected to scratches or mechanical energy such as an external force, the reversible bond is broken and the material exhibits easy disassembly. Furthermore, since the equilibrium moves toward the binding direction, it is believed that the adduct is formed again, allowing damage repair and remolding.

The structure of the obtained cured product can be checked by infrared absorption (IR) spectroscopy using Fourier transform infrared spectroscopy (FT-IR) or the like, elemental analysis, X-ray scattering, or the like.

As described above, the cured product according to one embodiment of the present invention can be obtained by using the phenolic hydroxyl group-containing compound of the present invention as one component of the curable resin composition, but the intermediate of the conjugated diene or parent diene intermediate described above, which is an intermediate of phenolic hydroxyl group-containing compounds, can be used in combination with a compound addition-reactable by the Diels-Alder reaction to from a cured product while forming (while synthesizing in situ) the phenolic hydroxyl group-containing compound during the curing process.

For example, when a curing reaction is performed by using the formula (1)', maleimide having a phenolic hydroxyl group, and the compound (I) having reactivity with the above-mentioned phenolic hydroxyl group-containing compound as essential raw materials, in the process of the curing reaction, a phenolic hydroxyl group-containing compound represented by the above formula (1) can be obtained, and as the curing reaction progresses, a cured product can be obtained. The maleimide having a hydroxyl group that can be used in this process is the same as described above.

In addition, when a curing reaction is performed by using the formula (2)', furan having a phenolic hydroxyl group, and the compound (I) having reactivity with the above-mentioned phenolic hydroxyl group-containing compound as essential raw materials, in the process of the curing reaction, a phenolic hydroxyl group-containing compound represented by the above formula (2) can be obtained, and as the curing reaction progresses, a cured product can be obtained. The furan having hydroxyl groups that can be used in this process is the same as described above.

The curable resin composition of the present invention and a cured product produced from the curable resin composition have both excellent easy disassembly and repairability, as well as remoldability, and are useful for the following applications.

The curable resin cured product of the present invention can be made into a laminate by being laminated on a base material. Inorganic materials such as metal and glass, as well as organic materials such as plastic and wood, can be used as the base material for the laminate, depending on the application in a timely manner, and the materials may be in the form of a laminate, such as a flat plate, a sheet-shaped product, or may have a three-dimensional structure, or may be three-dimensional. The base material may be of any shape that suits the purpose, such as having curvature on all or part of the surface. The hardness and thickness of the base material are not limited. In addition, a multi-layered laminate in which a first base material, a layer consisting of a cured product of the curable resin composition of the present invention, and a second base material are laminated in this order may be used. Since the curable resin composition of the present embodiment has excellent adhesive properties, the curable resin composition can be suitably used as an adhesive to bond the first base material to the second base material. Further, the curable resin cured product of the present invention may be used as the base material, and the cured product of the present invention may be further laminated.

In addition, the curable resin cured product of the present invention is particularly suitable for bonding dissimilar materials because of the ability to relieve stress. For example, even in a case of a laminate of dissimilar materials in which the base material is a metal and/or metal oxide and the second base material is a plastic layer, the adhesive strength is maintained by the stress relaxation ability of the cured product of the present invention.

In the laminate obtained by laminating the cured product of the present invention and the base material, the layer containing the cured product may be formed by coating or molding directly onto the base material, or may be laminated with an already molded material. In the case of direct coating, examples of the coating method include, but not particularly limited to, a spray method, a spin coating method, a dipping method, a roll coating method, a blade coating method, a doctor roll method, a doctor blade method, a curtain coating method, a slit coating method, a screen printing method, and an inkjet method. Examples of the direct molding include in mold decorating, insert molding, vacuum molding, extrusion lamination molding, and press molding. When the molded composition is laminated, the uncured or semi-cured composition layer may be laminated and then cured, or a layer containing a cured product obtained by completely curing the composition may be laminated on the base material. On the other hand, the lamination may be performed by coating the cured product of the present invention with a precursor that can be a base material, followed by curing, or the curing may be performed after the precursor that can be a base material or the composition of the present invention is bonded in an uncured or semi-cured state. The precursor that can be a base material is not particularly limited, and examples thereof include various curable resin compositions.

The cured product obtained by using the curable resin composition of the present invention has particularly high adhesion to metals and/or metal oxides and thus can be used particularly well as primers for metals. Examples of metals include copper, aluminum, gold, silver, iron, platinum, chromium, nickel, tin, titanium, zinc, various alloys, and composite materials thereof, and examples of metal oxides include single oxides and/or composite oxides of these metals. In particular, the cured product has excellent adhesive strength to iron, copper, and aluminum, and can be used well as an adhesive for iron, copper, and aluminum.

The curable resin composition of the present invention can be suitably used as an adhesive for structural members in the fields of automobiles, trains, civil engineering and construction, electronics, aircraft, and the space industry. The adhesive can maintain high adhesiveness without being affected by changes in the temperature environment, even when used to bond dissimilar materials, such as between a metal and a non-metal, and peeling or the like is unlikely to occur. Further, in addition to structural member applications, the adhesive can also be used as an adhesive for general office use, medical use, carbon fiber, storage battery cells, modules and cases, and the like, and can be used as optical component bonding adhesives, optical disc bonding adhesives, printed wiring board mounting adhesives, die bonding adhesives, semiconductor adhesives such as underfill, BGA reinforcement underfill, and mounting adhesives for anisotropic conductive film, anisotropic conductive paste, and the like.

When the curable resin composition of the present invention has a fibrous substrate, and the fibrous substrate is a reinforcing fiber, the curable resin composition containing the fibrous substrate can be used as a fiber-reinforced resin. The method of incorporating the fibrous substrate into the composition is not particularly limited as long as the effects of the present invention are not impaired, and examples thereof include a method in which the fibrous substrate and the composition are combined by a method such as kneading, coating, impregnation, injection, and pressure bonding, which can be selected in a timely manner depending on the form of the fibers and the application of the fiber-reinforced resin.

The method for molding the fiber-reinforced resin is not particularly limited. When producing a plate-shaped product, an extrusion molding method is generally used, but flat press can also be used. In addition, an extrusion molding method, a blow molding method, a compression molding method, a vacuum molding method, an injection molding method, or the like can be used. Further, if a film-shaped products is to be produced, the solution casting method can be used in addition to the melt extrusion method, and when a melt molding method is used, the examples thereof include inflation film molding, cast molding, extrusion lamination molding, calender molding, sheet molding, fiber molding, blow molding, injection molding, rotational molding, and coating molding. **In** addition, in the case of a resin that is cured by active energy rays, a cured product can be produced by using various curing methods using active energy rays. In particular, when thermosetting resin is the main component of a matrix resin, there is a molding method in which a prepreg is made from the molding material and pressurized and heated by pressing or autoclaving, and other examples thereof include resin transfer molding (RTM), vacuum assist resin transfer molding (VaRTM), a laminated molding, and hand layup molding.

The curable resin composition of the present invention can be used as molding materials for large cases, motor housings, casting materials inside cases, gears and pulleys, and the like because the cured product made from the curable resin composition has both good heat resistance and repairability, and are remoldable. The cured product may be a cured product of resin alone or a cured product of fiber reinforced, such as a glass chip.

The fiber-reinforced resin can form a state called uncured or semi-cured prepreg. After distributing the product in a prepreg state, final curing may be performed to form a cured product. When forming a laminate, it is preferable to form the prepreg, then laminate the other layers, and then perform final curing, because a laminate in which each layer adheres closely to each other can be formed thereby. Although there is no particular limit to the mass ratio of the composition and the fibrous substrate used in this process, it is usually preferable to perform the preparation so that the resin content in the prepreg is 20 to 60% by mass.

The cured product of the present invention has good heat resistance and repairability, as well as remoldability, and can be used as a heat-resistant material and electronic material. In particular, the cured product can be suitably used for semiconductor encapsulation materials, circuit boards, build-up films, build-up substrates, or the like, as well as adhesives and resist materials. In addition, the cured product is also suitable for use as a matrix resin for fiber-reinforced resins, and is particularly suitable as a prepreg with high heat resistance. The heat resistant member and the electronic member obtained in this manner can be suitably used for various applications, and the examples thereof include, but are not limited to, industrial machinery parts, general machinery parts, parts for automobiles, railroads, vehicles, or the like, aerospace and aviation-related parts, electronic and electrical parts, construction materials, container and packaging parts, household goods, sports and leisure goods, and housing parts for wind power generation.

Among these, the cured product can be suitably used as an adhesive for structural members in the automobile, train, civil engineering and construction, electronics, aircraft, and space industries, taking advantage of the excellent flexibility in the cured product. The adhesive of the present invention can maintain high adhesiveness without being affected by changes in the temperature environment, even when used to bond dissimilar materials, such as between a metal and a non-metal, and peeling or the like is unlikely to occur. Further, in addition to structural member applications, the adhesive of the present invention can also be used as an adhesive for general office use, medical use, carbon fiber, storage battery cells, modules and cases, and the like, and the examples thereof include optical component bonding adhesives, optical disc bonding adhesives, printed wiring board mounting adhesives, die bonding adhesives, semiconductor adhesives such as underfill, BGA reinforcement underfill, and mounting adhesives for anisotropic conductive film, anisotropic conductive paste, and the like.

Hereinafter, a representative product will be described with an example.

### 1. Semiconductor Encapsulation Material

Examples of the method for obtaining a semiconductor encapsulation material from the resin composition of the present invention include a method for thoroughly melting and mixing the resin composition, a curing accelerator, and compounding agents such as inorganic fillers by using an extruder, a kneader, a roll, or the like, as necessary, until the material becomes uniform. In this case, fused silica is usually used as the inorganic filler, but when used as a high thermal conductivity semiconductor encapsulant for power transistors and power ICs, it is recommended to use crystalline silica, alumina, silicon nitride, or the like, which has higher thermal conductivity than fused silica. Regarding the filling rate of the inorganic filler per 100 parts by mass of the curable resin composition, the inorganic filler is preferably used in the range of 30 to 95% by mass, and among these, 70 or more parts by mass is more preferable, and 80 or more parts by mass is even more preferable, in order to improve flame retardancy, moisture resistance and solder cracking resistance, and to lower the coefficient of linear expansion.

### 2. Semiconductor Device

For semiconductor package molding to obtain a semiconductor device from the curable resin composition of the present invention, a method is exemplified in which the semiconductor encapsulation materials are molded by cast molding, or by using a transfer molding machine, injection molding machine, or the like, and in addition, are heated at 50 to 250°C for 2 to 10 hours.

### 3. Printed Circuit Board

As rhe method for obtaining a printed circuit board from the composition of the present invention, a method is exemplified in which the above prepreg is laminated in the usual manner, copper foil is overlapped as appropriate, and heat-pressing is performed at 170 to 300°C for 10 minutes to 3 hours under pressure of 1 to 10 MPa.

### 4. Flexible Substrate

The method for producing a flexible substrate from the crosslinkable resin composition of the present invention includes a method consisting of the following three processes. A first process is to apply the crosslinkable resin composition, which contains resin components organic solvents, and the like, to electrically insulating film using a coating machine such as a reverse roll coater or comma coater, a second process is to heat the electrically insulating film coated with the crosslinkable resin composition for 1 to 15 minutes at 60 to 170°C by using a heater to volatilize the solvent from the electrically insulating film, thereby bringing the crosslinkable resin composition into B-stage, and a third process is to thermo-compress metal foil onto the adhesive (compression pressure of 2 to 200 N/cm and compression temperature of 40 to 200°C are preferable) by using a heat roll or the like on the electrically insulating film in which the crosslinkable resin composition has been brought into B-stage. If sufficient adhesive performance is obtained by going through the above three processes, the process may be terminated here, but if complete adhesive performance is required, it is preferable to further perform post-curing of the composition under the condition of 100 to 200°C for 1 to 24 hours. The thickness of the resin composition layer after being finally cured is preferably in the range of 5 to 100 µm.

### 5. Build-up Substrate

The method for obtaining a build-up substrate from the composition of the present invention includes, for example, the following processes. First, the composition containing an appropriate amount of rubber, filler, and the like is applied to a circuit board having a circuit formed thereon by spray coating, curtain coating, or the like, and then cured (process 1). Thereafter, if necessary, holes of a predetermined through-hole portion and the like are drilled, then the surface is treated with a roughening agent, and the surface is washed with hot water to form irregularities, and then plated with a metal such as copper (process 2). This procedure is sequentially repeated as desired to alternately build up resin insulating layers and conductor layers of a predetermined circuit pattern (process 3). Drilling of the through-hole portion is performed after the formation of the outermost resin insulating layer. In addition, the build-up substrate of the present invention can also be produced by heat-pressing, on a wiring board having a circuit formed thereon, copper foil with resin obtained by semi-curing the resin composition on the copper foil at 170 to 300°C, to form a roughened surface, which eliminates the need for a plating process.

### 6. Build-up Film

A build-up film can be obtained from the composition of the present invention by applying the composition to the surface of a support film (Y) as a base material, and then drying the organic solvent by heating or blowing hot air or the like to form a layer (X) of the composition.

As the organic solvent used here, it is preferable to use, for example, ketones such as acetone, methyl ethyl ketone, and cyclohexanone, acetic acid esters such as ethyl acetate, butyl acetate, cellosolve acetate, propylene glycol monomethyl ether acetate, and carbitol acetate, carbitols such as cellosolve and butylcarbitol, aromatic hydrocarbons such as toluene and xylene, dimethylformamide, dimethylacetamide, and N-methylpyrrolidone, and it is preferable to use in a ratio in which a non-volatile content is 30 to 60% by mass.

The thickness of the layer (X) to be formed is generally equal to or larger than the thickness of the conductor layer. The thickness of the conductor layer of the circuit board is usually in the range of 5 to 70 µm, and thus the thickness of the resin composition layer is preferably 10 to 100 µm. The layer (X) of the composition of the present invention may be protected by a protective film to be described later. Protection with a protective film prevents dust and other debris from adhering to or scratching the surface of the resin composition layer.

Examples of the support film and the protective film described above include polyolefins such as polyethylene, polypropylene, and polyvinyl chloride, polyesters such as polyethylene terephthalate (hereinafter, abbreviated as "PET" in some cases), polyethylene naphthalate, polycarbonate, polyimide, release paper, and metal foils such as copper foil, and aluminum foil. The support film and the protective film may be subjected to a releasing treatment in addition to a mat treatment and a corona treatment. The thickness of the support film is not particularly limited, but is usually from 10 to 150 µm, preferably in the range of 25 to 50 µm. The thickness of the protective film is preferably 1 to 40 µm.

The above-described support film (Y) is peeled after being laminated on a circuit board or after the insulating layer is formed by performing heating and curing. When the support film (Y) is peeled after the curable resin composition layer constituting the build-up film is cured by heating, adhesion of dust or the like in the curing process can be prevented. In the case of peeling after curing, the support film is usually subjected to a releasing treatment in advance.

A multilayer printed circuit board can be produced by using the build-up film obtained as described above. For example, when the layer (X) is protected with a protective film, the film is peeled off and then the layer (X) is laminated onto one or both sides of the circuit board so as to be in direct contact with the circuit board, for example, by a vacuum lamination method. The lamination method may be batch or continuous on rolls. In addition, if necessary, the build-up film and the circuit board may be heated before lamination (preheated) if necessary. The conditions for lamination are as follows: the compression temperature (lamination temperature) is preferably 70 to 140°C, and the compression pressure is preferably 1 to 11 kgf/cm2 (9.8 × 10⁴ to 107.9 × 10⁴ N/m²), and it is preferable to perform lamination under reduced air pressure of 20 mmHg (26.7 hPa) or less.

### 7. Conductive Paste

Examples of the method for obtaining the conductive paste from the composition of the present invention include a method for dispersing conductive particles in the composition. The conductive paste can be a circuit connection paste resin composition or an anisotropic conductive adhesive depending on the type of conductive particles used.

### Examples

Hereinafter, the present invention will be described in detail by means of examples and comparative examples, and in the following, "part" and "%" are on a mass basis unless otherwise specified. The present invention is not limited thereto.

¹H and ¹³C-NMR, FD-MS spectra and GPC were measured under the following conditions.
¹H-NMR: "JNM-ECA600" manufactured by JEOL RESONANCE
Magnetic field intensity: 600 MHz
Number of times of integration: 32
Solvent: DMSO-d₆
Sample concentration: 30% by mass
¹³C-NMR: "JNM-ECA600" manufactured by JEOL RESONANCE
Magnetic field intensity: 150 MHz
Number of times of integration: 320
Solvent: DMSO-d₆
Sample concentration: 30% by mass
FD-MS: "JMS-T100GC AccuTOF" manufactured by JEOL Ltd.
Measurement range: m/z = 50.00 to 2000.00
Rate of change: 25.6 mA/min
Final current value: 40 mA
Cathode voltage: -10 kV
GPC: "HLC-8320GPC" manufactured by Tosoh Corporation
Column: "TSK-GEL G2000HXL" + "TSK-GEL G3000HXL" + "TSK-GEL G4000HXL" manufactured by Tosoh Corporation
Detector: RI (differential refractometer)
Measuring conditions: 40°C
Mobile phase: tetrahydrofuran
Flow rate: 1 ml/min
Standard: "PStQuick A", "PStQuick B", "PStQuick E", and "PStQuick F" manufactured by Tosoh Corporation

The epoxy equivalent weight of the synthesized epoxy resin was measured in accordance with JIS K7236, and the epoxy equivalent weight (g/eq) was calculated.

The number of repeating units can be calculated, for example, from the results of GPC molecular weight measurement, or various appropriate instrumental analyses such as FD-MS and NMR.

### Synthesis Example 1

Into a flask equipped with a thermometer, a condenser, and a stirrer, 420 g (2.0 equivalents) of diglycidyl ether of 1,12-dodecanediol (manufactured by Yokkaichi Chemical Company Limited: epoxy equivalent weight of 210 g/eq) and 456 g (4.0 equivalents) of bisphenol A (hydroxyl group equivalent weight 114 g/eq) were charged, and after temperature was raised to 140°C over 30 minutes, 4.0 g of 4% aqueous sodium hydroxide solution was charged. Thereafter, the temperature was increased to 150°C over 30 minutes, followed by reaction at 150°C for another 6 hours. Thereafter, a neutralizing amount of sodium phosphate was then added to obtain 858 g of the hydroxy compound (Ph-1). This hydroxy compound (Ph-1) was found to contain the target hydroxy compound because the mass spectrum showed a peak of M+ = 771, corresponding to the theoretical structure of m = 1 in the following structural formula (Ph-1). The hydroxyl group equivalent weight of this hydroxy compound (Ph-1) calculated by GPC was 388 g/eq, and the average value of the repeating unit m was 0.8.

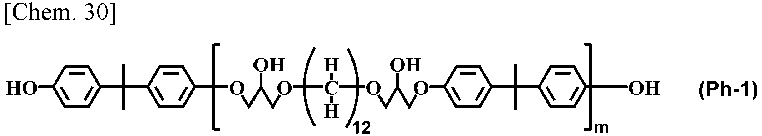

### Synthesis Example 2

The same reaction as in Synthesis Example 1 was carried out except that 420 g (2.0 equivalents) diglycidyl ether of 1,12-dodecanediol (epoxy equivalent weight of 210 g/eq) in Synthesis Example 1 was changed to 884 g (2.0 equivalents) of diglycidyl ether of polytetramethylene glycol (Denacol EX-991L manufactured by Nagase ChemteX Corporation: epoxy equivalent weight of 442 g/eq), to obtain 1,313 g of a hydroxy compound (Ph-2). This hydroxy compound (Ph-2) was found to contain the target hydroxy compound because the mass spectrum showed a peak of M+ = 1380, corresponding to the theoretical structure of m = 1 and n2 = 11 in the following structural formula (Ph-2). The hydroxyl group equivalent weight of this hydroxy compound (Ph-2) calculated by GPC was 600 g/eq, and the average value of the repeating unit m was 0.8.

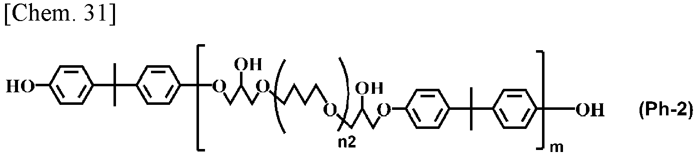

### Synthesis Example 3

The same reaction as in Synthesis Example 1 was carried out except that 420 g (2.0 equivalents) of diglycidyl ether of 1,12-dodecanediol (epoxy equivalent weight of 210 g/eq) in Synthesis Example 1 was changed to 962 g (2.0 equivalents) of diglycidyl ether of polypropylene glycol (Denacol EX-931 manufactured by Nagase ChemteX Corporation: epoxy equivalent weight of 481 g/eq), to obtain 1390 g of a hydroxy compound (Ph-3). This hydroxy compound (Ph-3) was found to contain the target hydroxy compound because the mass spectrum showed a peak of M+ = 1226, corresponding to the theoretical structure of m = 1 and n2 = 11 in the following structural formula (Ph-3). The hydroxyl group equivalent weight of this hydroxy compound (Ph-3) calculated by GPC was 593 g/eq, and the average value of the repeating unit m was 0.8.

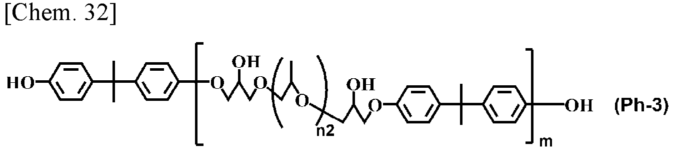

### Synthesis Example 4

Into a flask equipped with a thermometer, a dropping funnel, a condenser, and a stirrer, 388 g (hydroxyl group equivalent weight of 388 g/eq) of the hydroxy compound (Ph-1) obtained in Synthesis Example 1, 1,110 g (12.0 mol) of epichlorohydrin, and 300 g of n-butanol were added and dissolved while purging with nitrogen gas. Thereafter, the temperature was raised to 65°C, the pressure was reduced to an azeotropic pressure, and 122.4 g (1.5 mol) of a 49% aqueous sodium hydroxide solution was added dropwise over 5 hours.

Next, stirring was continued for 0.5 hours under the same conditions. The reaction was performed while separating the distillates from the azeotropic distillation with a Dean Stark trap, removing the water layer, and returning the oil layer to the reaction system. Thereafter, the unreacted epichlorohydrin was then distilled off by vacuum distillation. To the obtained crude epoxy resin, 1000 g of methyl isobutyl ketone and 110 g of n-butanol were added and dissolved.

Furthermore, 20.0 g of a 10% aqueous sodium hydroxide solution of was added to this solution, and the mixture was reacted at 80°C for 2 hours, after which washing with 300 g of water was repeated three times until the pH of the cleaning liquid became neutral.

Next, the system was dehydrated by azeotropy, and after undergoing microfiltration, the solvent was distilled off under reduced pressure to obtain 399 g of epoxy compound (Ep-1). The obtained epoxy compound (Ep-1) had an epoxy equivalent weight of 488 g/eq. The epoxy compound concerned was found to contain the target epoxy compound (Ep-1) because the mass spectrum showed a peak of M+ = 883, corresponding to the theoretical structure of m = 1, q = 1, p₁ = 0, and p₂ = 0 in the following structural formula (Ep-1). The obtained epoxy compound (Ep-1) contained a compound with q = 0, and when checked by GPC, the obtained epoxy compound contained 20.5% of the compound with q = 0 in the mixture, and the average value of the repeating unit q was 0.8.

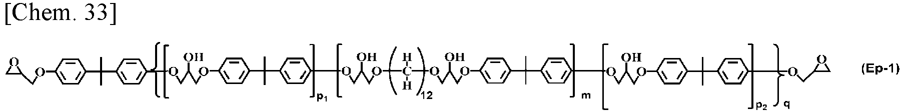

### Synthesis Example 5

The same reaction as in Synthesis Example 4 was carried out except that 388 g (hydroxyl group equivalent weight of 388 g/eq) of the hydroxy compound (Ph-1) in Synthesis Example 4 was changed to 600 g (hydroxyl group equivalent weight of 600 g/eq) of the hydroxy compound (Ph-2), to obtain 591 g of an epoxy compound (Ep-2). The obtained epoxy compound (Ep-2) had an epoxy equivalent weight of 722 g/eq. The epoxy resin was found to contain the target epoxy compound (Ep-2) because the mass spectrum showed a peak of M+ = 1492, corresponding to the theoretical structure of m = 1, n2 = 11, q = 1, p1 = 0, and p2 = 0 in the following structural formula (Ep-2). The obtained epoxy compound (Ep-2) contained a compound with q = 0, and when checked by GPC, the obtained epoxy compound contained 17.2% of the compound with q = 0 in the mixture, and the average value of the repeating unit q was 0.8.

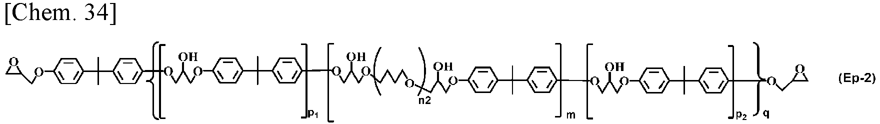

### Synthesis Example 6

The same reaction as in Synthesis Example 4 was carried out except that 388 g (hydroxyl group equivalent weight of 388 g/eq) of the hydroxy compound (Ph-1) in Synthesis Example 4 was changed to 593 g (hydroxyl group equivalent weight of 593 g/eq) of the hydroxy compound (Ph-3), to obtain 584 g of an epoxy compound (Ep-3). The obtained epoxy compound (Ep-3) had an epoxy equivalent weight of 714 g/eq. The epoxy compound was found to contain the target epoxy compound (Ep-3) because the mass spectrum showed a peak of M+ = 1336, corresponding to the theoretical structure of m = 1, n2 = 11, q = 1, p1 = 0, and p2 = 0 in the following structural formula (Ep-3). The obtained epoxy compound (Ep-3) contained a compound with q = 0, and when checked by GPC, the obtained epoxy compound contained 16.8% of the compound with q = 0 in the mixture, and the average value of the repeating unit q was 0.8.

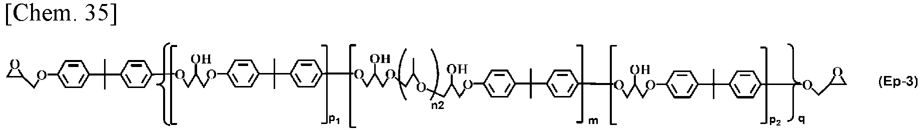

### Synthesis Example 7

An epoxy compound (Ep-4) was synthesized according to the method described in the literature of J. Network Polym., Jpn., Vol. 29, 208; 2008. The obtained epoxy compound (Ep-4) had an epoxy equivalent weight of 450 g/eq.

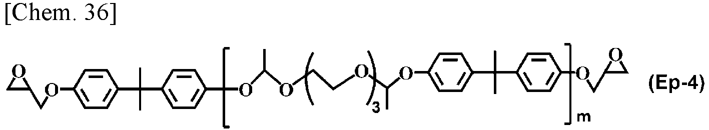

### Synthesis Example 8

According to the method described in the literature of Polymer Vol. 37 No. 16, 3721-3727; 1996, a phenolic hydroxyl group-containing maleimide compound having the following structure, 4-hydroxyphenylmaleimide (Ph-M-1), was synthesized.

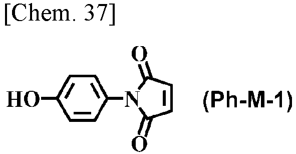

### Synthesis Example 9

According to the method described in the publication of International Application No. PCT/US2020/058107, a phenolic hydroxyl group-containing furan compound (Ph-F-1) having the following structure was synthesized.

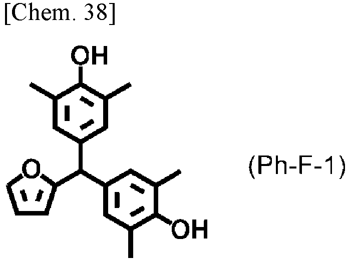

### Example 1

Into a flask equipped with a thermometer, a dropping funnel, a condenser, and a stirrer, 48.8 g (epoxy equivalent weight of 488 g/eq) of the epoxy resin (Ep-1) obtained in Synthesis Example 4 and 19.6 g (0.2 mol) of furfuryl alcohol were added and dissolved while purging with nitrogen gas. Thereafter, 0.7 g (0.007 mol) of triethyl amine was added, and the temperature was raised to 70°C over 30 minutes, the mixture was reacted at 70°C for another 9 hours. Thereafter, the temperature was raised to 150°C, and excess furfuryl alcohol was distilled off under reduced pressure to obtain 53 g of a furan compound (F-1). The molecular weight of this furan compound (F-1) measured by GPC was Mn = 1600 and Mw = 4900. The molecular weight of this furan compound (F-1) per mole of the furan structure calculated from ¹H-NMR was 575 g/eq. The obtained furan compound (F-1) contained a compound with q = 0, and when checked by GPC, the obtained furan compound contained 18.2% of the compound with q = 0 in the mixture, and the average value of the repeating unit q was 0.8.

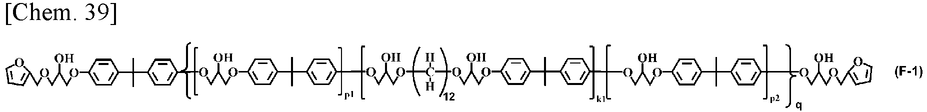

### Example 2

Into a flask equipped with a thermometer, a stirrer, and a condenser, 29 g of the furan compound (F-1, furan equivalent weight of 575 g/eq) obtained in Example 1, 5.3 g of 1,6'-bismaleimide-(2,2,4-trimethyl)hexane (BMI-THM, manufactured by Daiwakasei Industry Co., Ltd.), and 50 g of toluene were charged, and after replacement with nitrogen, the mixture was reacted at 60°C for 20 hours. Thereafter, toluene was distilled off under reduced pressure to obtain 34 g of a furan compound (F-2). The molecular weight measured by GPC was Mn = 2000 and Mw = 7900. In addition, the molecular weight per mole of the furan structure calculated from ¹H-NMR was 2043 g/eq. The obtained furan compound (F-2) contained a compound with n = 0 and q = 0, and when checked by GPC, the obtained furan compound contained 1.9% of the compound with n = 0 and q = 0 in the mixture, the average value of the repeating unit n was 1.1, and the average value of the repeating unit q was 0.8. In the chemical formula over the following two or more lines, * indicates a direct binding with the position of * in the next line.

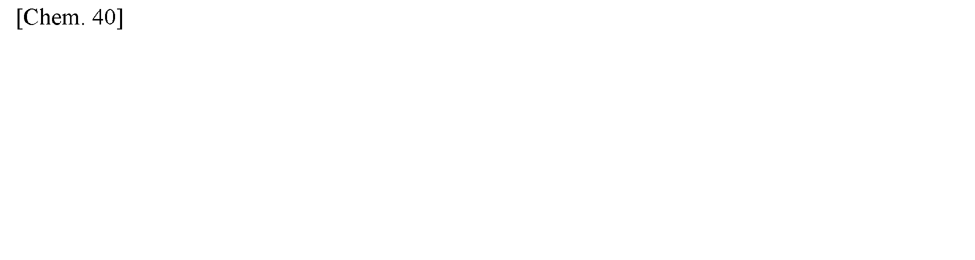

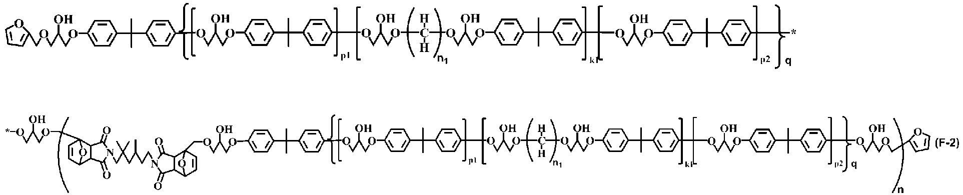

### Example 3

Into a flask equipped with a thermometer, a stirrer, and a condenser, 34 g of the furan compound (F-2, furan equivalent weight of 2043 g/eq) obtained in Example 2, 3.2 g of 4-hydroxyphenylmaleimide (Ph-M-1) obtained in Synthesis Example 8, and 50 g of toluene were charged, and after replacement with nitrogen, the mixture was reacted at 60°C for 12 hours. Thereafter, tetrahydrofuran was distilled off under reduced pressure to obtain 33 g of a phenolic hydroxyl group-containing compound (D-1). The molecular weight measured by GPC was Mn = 2200 and Mw = 8300. In addition, the hydroxyl equivalent weight calculated from ¹H-NMR was 2288 g/eq. The obtained phenolic hydroxyl group-containing compound (D-1) contained a compound with n = 0 and q = 0, and when checked by GPC, the obtained phenolic hydroxyl group-containing compound contained 1.1% of the compound with n = 0 and q = 0 in the mixture, the average value of the repeating unit n was 1.1, and the average value of the repeating unit q was 0.8.

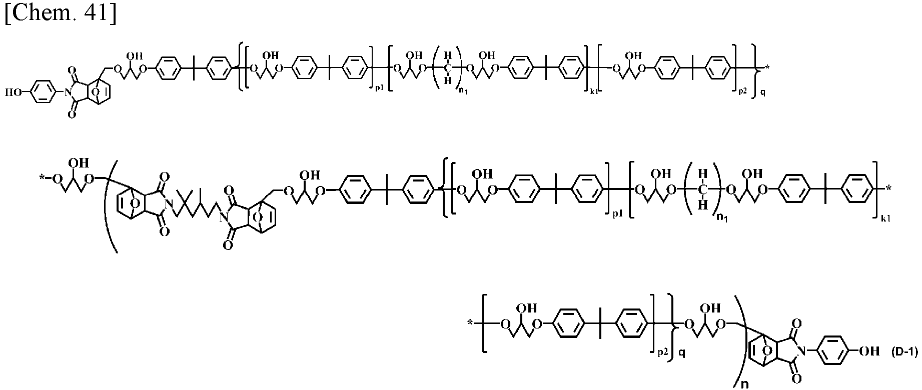

### Example 4

The same reaction as in Example 1 was carried out except that 48.8 g (epoxy equivalent weight of 488 g/eq) of the epoxy resin (Ep-1) in Example 1 was changed to 72.2 g (epoxy equivalent weight of 722 g/eq) of the epoxy resin (Ep-2), to obtain 74 g of a furan compound (F-3). The molecular weight of this furan compound (F-3) measured by GPC was Mn = 2400 and Mw = 8100. The molecular weight of this furan compound (F-3) per mole of the furan structure calculated from ¹H-NMR was 804 g/eq. The obtained furan compound (F-3) contained a compound with q = 0, and when checked by GPC, the obtained furan compound contained 16.5% of the compound with q = 0 in the mixture, and the average value of the repeating unit q was 0.8.

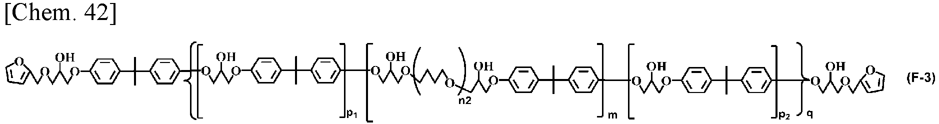

### Example 5

The same reaction as in Example 2 was carried out except that 29 g of the furan compound (F-1, furan equivalent weight of 575 g/eq) in Example 2 was changed to 40 g of a furan compound (F-3, furan equivalent weight of 804 g/eq), to obtain 45 g of a furan compound (F-4). The molecular weight measured by GPC was Mn = 2800 and Mw = 12100. In addition, the molecular weight per mole of the furan structure calculated from ¹H-NMR was 2730 g/eq. The obtained furan compound (F-4) contained a compound with n = 0 and q = 0, and when checked by GPC, the obtained furan compound contained 1.8% of the compound with n = 0 and q = 0 in the mixture, the average value of the repeating unit n was 1.1, and the average value of the repeating unit q was 0.8.

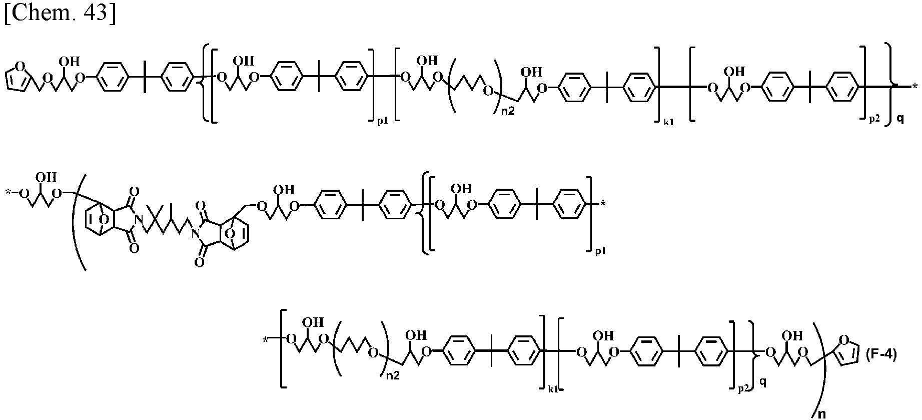

### Example 6

The same reaction as in Example 3 was carried out except that 34 g of the furan compound (F-2, furan equivalent weight of 2043 g/eq) in Example 3 was changed to 45 g of a furan compound (F-4, furan equivalent weight of 2730 g/eq), to obtain 43 g of a phenolic hydroxyl group-containing compound (D-2). The molecular weight measured by GPC was Mn = 3000 and Mw = 13000. In addition, the hydroxyl group equivalent weight calculated from ¹H-NMR was 2919 g/eq. The obtained phenolic hydroxyl group-containing compound (D-2) contained a compound with n = 0 and q = 0, and when checked by GPC, the obtained phenolic hydroxyl group-containing compound contained 1.5% of the compound with n = 0 and q = 0 in the mixture, the average value of the repeating unit n was 1.1, and the average value of the repeating unit q was 0.8.

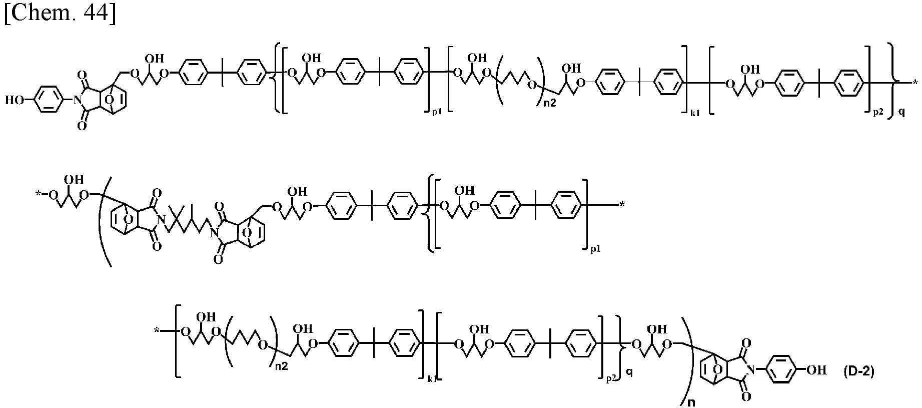

### Example 7

The same reaction as in Example 1 was carried out except that 48.8 g (epoxy equivalent weight of 488 g/eq) of the epoxy resin (Ep-1) in Example 1 was changed to 71.4 g (epoxy equivalent weight of 714 g/eq) of the epoxy resin (Ep-3), to obtain 73 g of a furan compound (F-5). The molecular weight of this furan compound (F-5) measured by GPC was Mn = 1900 and Mw = 5100. The molecular weight of this furan compound (F-5) per mole of furan structure calculated from ¹H-NMR was 796 g/eq. The obtained furan compound (F-5) contained a compound with q = 0, and when checked by GPC, the obtained furan compound contained 14.7% of the compound with q = 0 in the mixture, and the average value of the repeating unit q was 0.8.

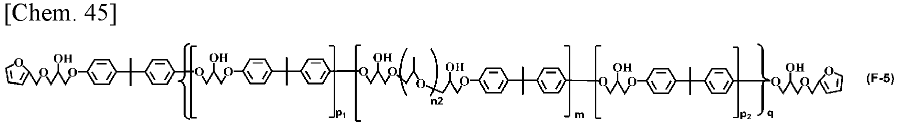

### Example 8

The same reaction as in Example 2 was carried out except that 29 g of the furan compound (F-1, furan equivalent weight of 575 g/eq) in Example 2 was changed to 40 g of a furan compound (F-5, furan equivalent weight of 796 g/eq), to obtain 46 g of a furan compound (F-6). The molecular weight measured by GPC was Mn = 2300 and Mw = 9000. In addition, the molecular weight per mole of the furan structure calculated from ¹H-NMR was 2706 g/eq. The obtained furan compound (F-6) contained a compound with n = 0 and q = 0, and when checked by GPC, the obtained furan compound contained 2.9% of the compound with n = 0 and q = 0 in the mixture, the average value of the repeating unit n was 1.1, and the average value of the repeating unit q was 0.8.

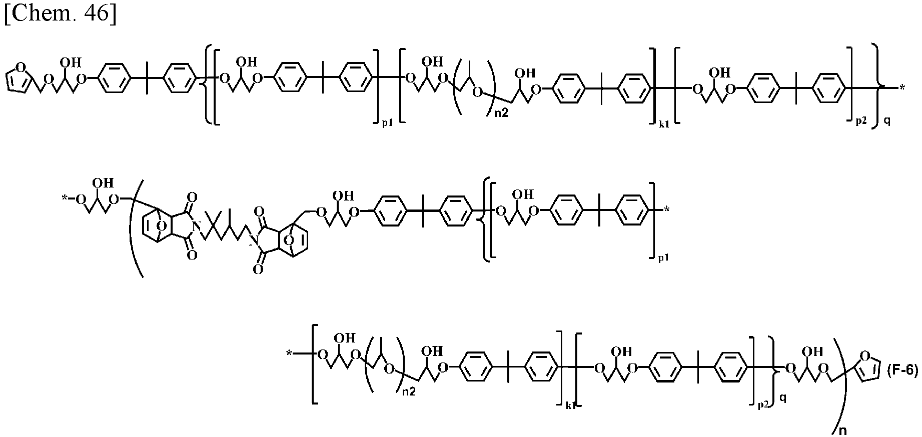

### Example 9

The same reaction as in Example 3 was carried out except that 34 g of the furan compound (F-2, furan equivalent weight of 2043 g/eq) in Example 3 was changed to 45 g of a furan compound (F-6, furan equivalent weight of 2706 g/eq), to obtain 44 g of a phenolic hydroxyl group-containing compound (D-3). The molecular weight measured by GPC was Mn = 2700 and Mw = 10100. In addition, the hydroxyl equivalent weight calculated from ¹H-NMR was 2895 g/eq. The obtained phenolic hydroxyl group-containing compound (D-3) contained a compound with n = 0 and q = 0, and when checked by GPC, the obtained phenolic hydroxyl group-containing compound contained 1.1% of the compound with n = 0 and q = 0 in the mixture, the average value of the repeating unit n was 1.1, and the average value of the repeating unit q was 0.8.

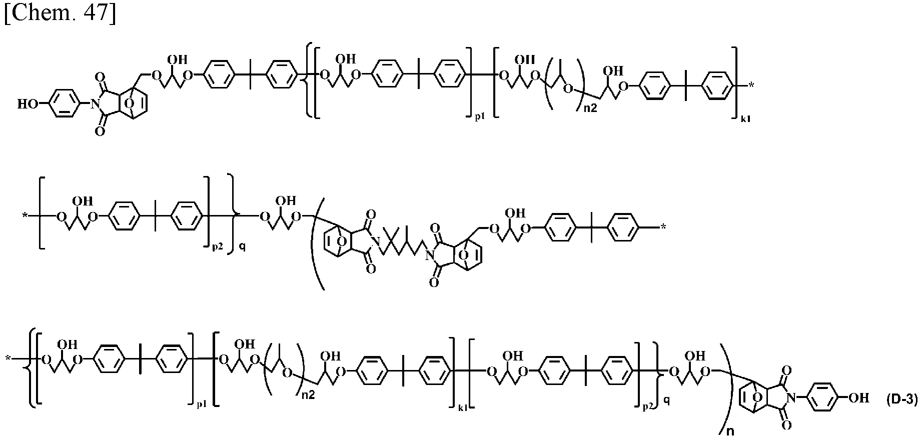

### Example 10

The same reaction as in Example 1 was carried out except that 48.8 g (epoxy equivalent weight of 488 g/eq) of the epoxy compound (Ep-1) in Example 1 was changed to 21 g of diglycidyl ether of 1,12-dodecanediol (manufactured by Yokkaichi Chemical Company Limited: epoxy equivalent weight of 210 g/eq), to obtain 29 g of a furan compound (F-7). The furan compound was found to contain the target epoxy compound (Ep-3) because the mass spectrum showed a peak at M+ = 510, corresponding to the theoretical structure of the following structural formula (F-7). The molecular weight of this furan compound (F-7) measured by GPC was Mn = 800 and Mw = 1300. The molecular weight of this furan compound (F-5) per mole of the furan structure calculated from ¹H-NMR was 302 g/eq.

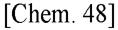

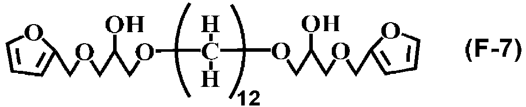

### Example 11

The same reaction as in Example 2 was carried out except that 29 g of the furan compound (F-1, furan equivalent weight of 575 g/eq) in Example 2 was changed to 15 g of a furan compound (F-7, furan equivalent weight of 302 g/eq), to obtain 20 g of a furan compound (F-8). The molecular weight measured by GPC was Mn = 1500 and Mw = 2100. In addition, the molecular weight per mole of the furan structure calculated from ¹H-NMR was 1224 g/eq. The obtained furan compound (F-8) contained a compound with n = 0, and when checked by GPC, the obtained furan compound contained 1.9% of the compound with n = 0 in the mixture, and the average value of the repeating unit n was 1.1.

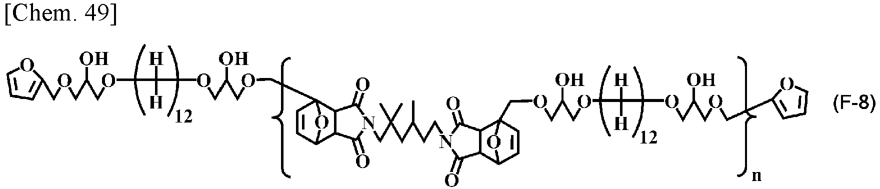

### Example 12

The same reaction as in Example 3 was carried out except that 34 g of the furan compound (F-2, furan equivalent weight of 2043 g/eq) in Example 3 was changed to 20 g of a furan compound (F-8, furan equivalent weight of 1224 g/eq), to obtain 22 g of a phenolic hydroxyl group-containing compound (D-4). The molecular weight measured by GPC was Mn = 1600 and Mw = 2300. In addition, the hydroxyl equivalent weight calculated from ¹H-NMR was 1413 g/eq. The obtained phenolic hydroxyl group-containing compound (D-3) contained a compound with n = 0, and when checked by GPC, the obtained phenolic hydroxyl group-containing compound contained 1.1% of the compound with n = 0 in the mixture, and the average value of the repeating unit n was 1.1.

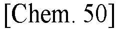

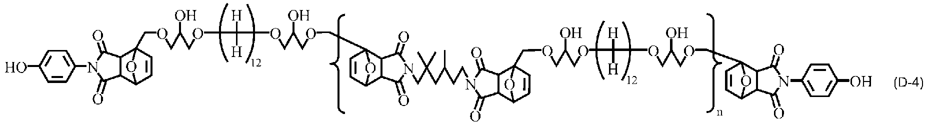

### Example 13

The same reaction as in Example 1 was carried out except that 48.8 g (epoxy equivalent weight of 488 g/eq) of the epoxy resin (Ep-1) in Example 1 was changed to 45.0 g (epoxy equivalent weight of 450 g/eq) of the epoxy resin (Ep-4), to obtain 49 g of a furan compound (F-9). The molecular weight of this furan compound (F-9) measured by GPC was Mn = 1100 and Mw = 1900. The molecular weight of this furan compound (F-9) per mole of the furan structure calculated from ¹H-NMR was 537 g/eq. The obtained furan compound (F-9) contained a compound with m = 0, and when checked by GPC, the obtained furan compound contained 20.1% of the compound with m = 0 in the mixture.

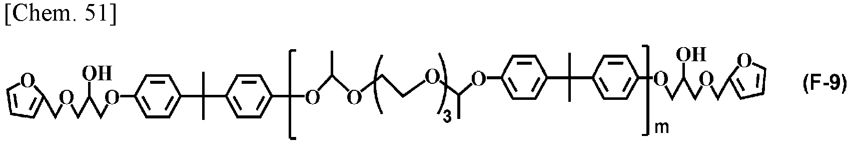

### Example 14

The same reaction as in Example 2 was carried out except that 29 g of the furan compound (F-1, furan equivalent weight 575 g/eq) in Example 2 was changed to 27 g of a furan compound (F-9, furan equivalent weight 537 g/eq), to obtain 32 g of a furan compound (F-10). The molecular weight measured by GPC was Mn = 2000 and Mw = 3900. In addition, the molecular weight per mole of the furan structure calculated from ¹H-NMR was 1929 g/eq. The obtained furan compound (F-10) contained a compound with m = 0 and n = 0, and when checked by GPC, the obtained furan compound contained 2.1% of the compound with m = 0 and n = 0 in the mixture, and the average value of the repeating unit n was 1.1.

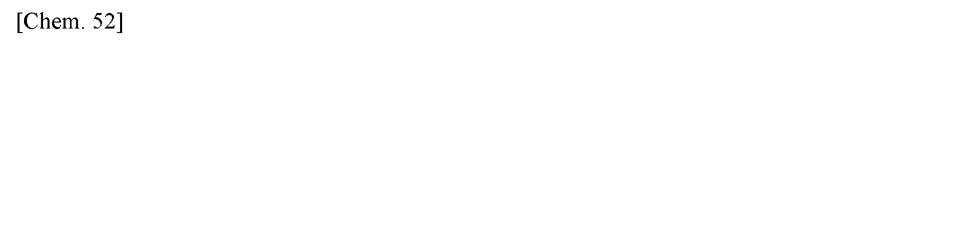

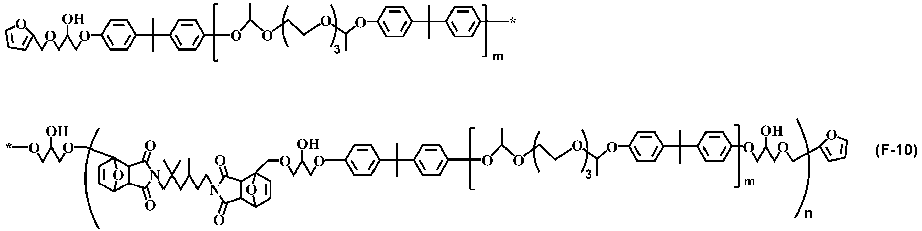

### Example 15

The same reaction as in Example 3 was carried out except that 34 g of the furan compound (F-2, furan equivalent weight of 2043 g/eq) in Example 3 was changed to 32 g of a furan compound (F-10, furan equivalent weight of 1929 g/eq), to obtain 32 g of a phenolic hydroxyl group-containing compound (D-5). The molecular weight measured by GPC was Mn = 2200 and Mw = 4500. In addition, the hydroxyl equivalent weight calculated from ¹H-NMR was 2119 g/eq. The obtained phenolic hydroxyl group-containing compound (D-5) contained a compound with m = 0 and n = 0, and when checked by GPC, the obtained phenolic hydroxyl group-containing compound contained 1.8% of the compound with m = 0 and n = 0 in the mixture, and the average value of the repeating unit n was 1.1.

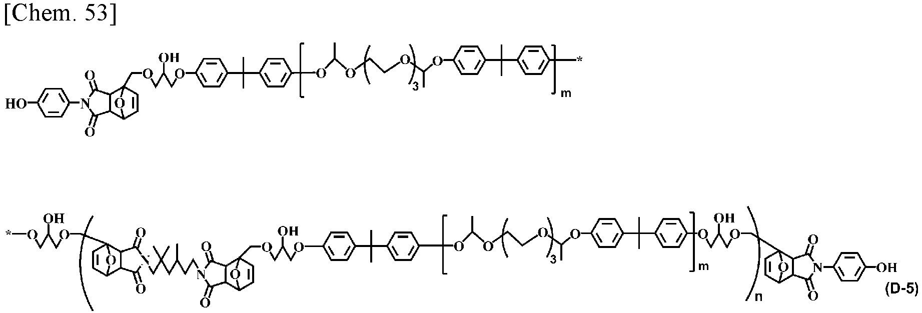

### Example 16

The same reaction as in Example 1 was carried out except that 48.8 g (epoxy equivalent weight of 488 g/eq) of the epoxy resin (Ep-1) in Example 1 was changed to 18.8 g (epoxy equivalent weight of 188 g/eq) of a bisphenol A type liquid epoxy resin "E-850S" (manufactured by DIC Corporation), to obtain 26 g of a furan compound (F-11). The molecular weight of this furan compound (F-11) measured by GPC was Mn = 800 and Mw = 1300. The molecular weight of this furan compound (F-11) per mole of the furan structure calculated from ¹H-NMR was 286 g/eq. The obtained furan compound (F-11) contained a compound of m = 0, and when checked by GPC, the obtained furan compound contained 66.1% of the compound with p1 = 0 in the mixture, and the average value of the repeating unit p1 was 0.1.

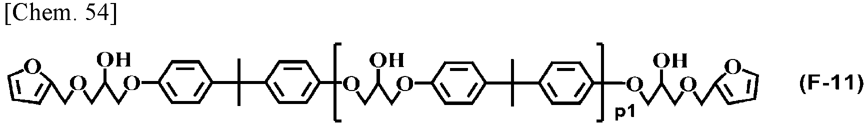

### Example 17

The same reaction as in Example 2 was carried out except that 29 g of the furan compound (F-1, furan equivalent weight of 575 g/eq) in Example 2 was changed to 14 g of a furan compound (F-11, furan equivalent weight of 286 g/eq), to obtain 19 g of a furan compound (F-12). The molecular weight measured by GPC was Mn = 1500 and Mw = 2400. In addition, the molecular weight per mole of the furan structure calculated from ¹H-NMR was 1158 g/eq. The obtained furan compound (F-12) contained a compound with p₁ = 0 and n = 0, and when checked by GPC, the obtained furan compound contained 7.0% of the compound with p1 = 0 and n = 0 in the mixture, the average value of the repeating unit n was 1. 1, and the average value of the repeating unit p1 was 0.1.

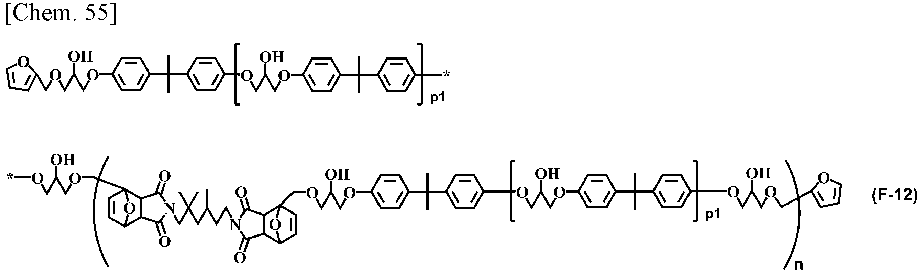

### Example 18

The same reaction as in Example 3 was carried out except that 34 g of the furan compound (F-2, furan equivalent weight of 2043 g/eq) in Example 3 was changed to 19 g of a furan compound (F-12, furan equivalent weight of 1158 g/eq), to obtain 20 g of a phenolic hydroxyl group-containing compound (D-6). The molecular weight measured by GPC was Mn = 1700 and Mw = 2600. In addition, the hydroxyl equivalent weight calculated from ¹H-NMR was 1347 g/eq. The obtained phenolic hydroxyl group-containing compound (D-6) contained a compound with m = 0 and n = 0, and when checked by GPC, the obtained phenolic hydroxyl group-containing compound contained 5.8% of the compound with p1 = 0 and n = 0 in the mixture, the average value of the repeating unit n was 1.1, and the average value of the repeating unit p1 was 0.1.

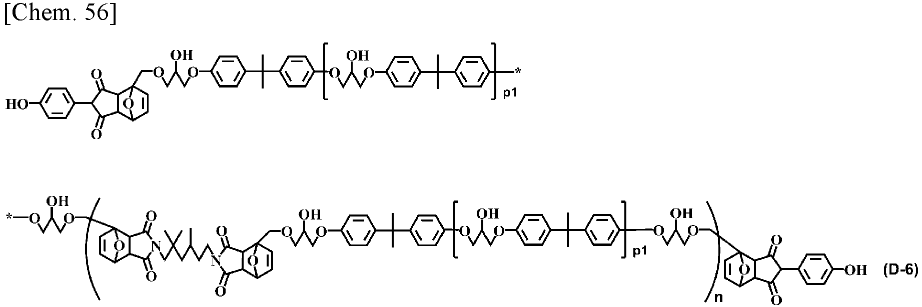

### Example 19

Into a flask equipped with a thermometer, a stirrer, and a condenser, 29 g of the furan compound obtained in Example 1 (F-1, furan equivalent weight of 575 g/eq), 11.9 g of 1,6'-bismaleimide-(2,2,4-trimethyl)hexane (BMI-THM, manufactured by Daiwakasei Industry Co., Ltd.), and 50 g of toluene were charged, and after replacement with nitrogen, the mixture was reacted at 60°C for 20 hours. Thereafter, toluene was distilled off under reduced pressure to obtain 41 g of a maleimide compound (M-1). The molecular weight measured by GPC was Mn = 2300 and Mw = 9000. In addition, the molecular weight per mole of the maleimide structure calculated from ¹H-NMR was 1627 g/eq. The obtained maleimide compound (M-1) contained a compound with n = 0, and when checked by GPC, the obtained maleimide compound contained 1.9% of the compound with n = 0 in the mixture, and the average value of the repeating unit n was 1.1.

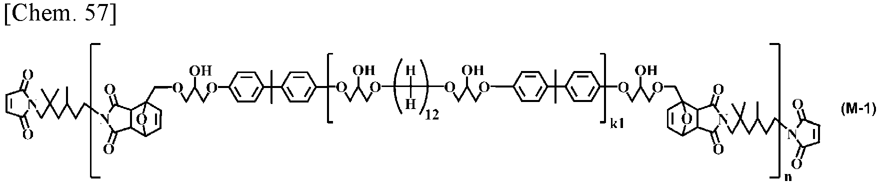

### Example 20

Into a flask equipped with a thermometer, a stirrer, and a condenser, 40 g of the maleimide compound (M-1, maleimide equivalent weight of 1627 g/eq) obtained in Example 19, 8.1 g of the phenolic hydroxyl group-containing compound (Ph-F-1) obtained in Synthesis Example 9, and 50 g of toluene were charged, and after replacement with nitrogen, the mixture was reacted at 60°C for 12 hours. Thereafter, toluene was distilled off under reduced pressure to obtain 43 g of a phenolic hydroxyl group-containing compound (D-7). The molecular weight measured by GPC was Mn = 3000 and Mw = 10500. In addition, the hydroxyl equivalent weight calculated from ¹H-NMR was 974 g/eq. The obtained phenolic hydroxyl group-containing compound (D-7) contained a compound with n = 0, and when checked by GPC, the obtained phenolic hydroxyl group-containing compound contained 1.1% of the compound with n = 0 in the mixture, and the average value of the repeating unit n was 1.1.

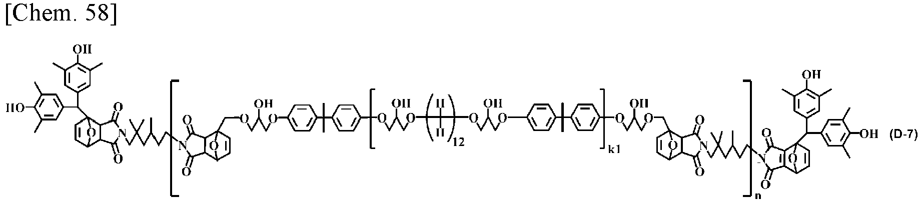

### Example 21

The same reaction as in Example 19 was carried out except that 29 g of the furan compound (F-1, furan equivalent weight of 575 g/eq) in Example 19 was changed to 167 g of a furan compound (F-7, furan equivalent weight of 302 g/eq), to obtain 28 g of a maleimide compound (M-2). The molecular weight measured by GPC was 2000, Mw = 3900. In addition, the molecular weight per mole of the maleimide structure calculated from ¹H-NMR was 1081 g/eq. The obtained maleimide compound (M-2) contained a compound with n = 0, and when checked by GPC, the obtained maleimide compound contained 2.1% of the compound with n = 0 in the mixture, and the average value of the repeating unit n was 1.1.

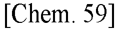

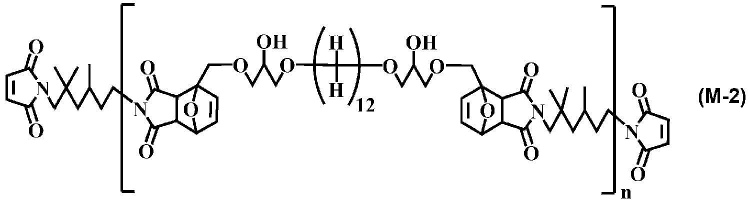

### Example 22

The same reaction as in Example 20 was carried out except that 40 g of the maleimide compound (M-1, maleimide equivalent weight of 1627 g/eq) in Example 20 was changed to 27 g of the maleimide compound (M-2, maleimide equivalent weight of 1081 g/eq), to obtain 32 g of a phenolic hydroxyl group-containing compound (D-8). The molecular weight measured by GPC was Mn = 3100 and Mw = 5200. In addition, the hydroxyl equivalent weight calculated from ¹H-NMR was 702 g/eq. The obtained phenolic hydroxyl group-containing compound (D-8) contained a compound with n = 0, and when checked by GPC, the obtained phenolic hydroxyl group-containing compound contained 1.2% of the compound with n = 0 in the mixture, and the average value of the repeating unit n was 1.1.

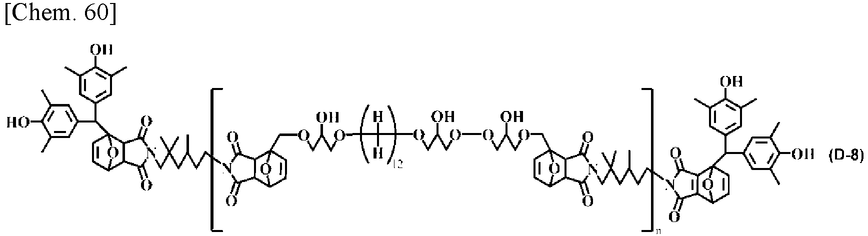

### Synthesis Example 10

The same reaction as in Synthesis Example 1 was carried out except that the amount of diglycidyl ether of 1,12-dodecanediol (epoxy equivalent weight of 210 g/eq) was changed from 420 g (2.0 equivalents) in Synthesis Example 1 to 798 g (3.8 equivalents), to obtain 1,207 g of a hydroxy compound (Ph-4). This hydroxy compound (Ph-4) was found to contain the target hydroxy compound because the mass spectrum showed a peak of M+ = 771, corresponding to the theoretical structure of m = 1 in the following structural formula (Ph-4). The hydroxyl group equivalent weight of this hydroxy compound (Ph-4) calculated by GPC was 2000 g/eq, and the average value of the repeating unit m was 6.9.

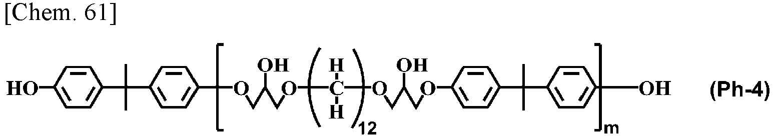

### Synthesis Example 11

The same reaction as in Synthesis Example 1 was carried out except that 420 g (2.0 equivalents) diglycidyl ether of 1,12-dodecanediol (epoxy equivalent weight 210 g/eq) in Synthesis Example 1 was changed to 1603 g (3.3 equivalents) of diglycidyl ether of polypropylene glycol ("Denacol EX-931" manufactured by Nagase ChemteX Corporation: epoxy equivalent weight of 481 g/eq), to obtain 2012 g of a hydroxy compound (Ph-5). The hydroxy compound (Ph-5) was found to contain the target hydroxy compound because the mass spectrum showed a peak of M+ = 1226, corresponding to the theoretical structure of m = 1 and n2 = 11 in the following structural formula (Ph-5). The hydroxyl equivalent weight of this hydroxy compound (Ph-5) calculated by GPC was 1802 g/eq, and the average value of the repeating unit m was 3.4.

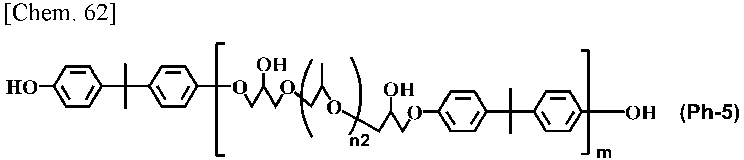

### Synthesis Example 12

The same reaction as in Synthesis Example 4 was carried out except that 388 g (hydroxyl group equivalent weight of 388 g/eq) of the hydroxy compound (Ph-1) in Synthesis Example 4 was changed to 2000 g (hydroxyl group equivalent weight of 2000 g/eq) of the hydroxy compound (Ph-4), to obtain 2120 g of an epoxy compound (Ep-5). The obtained epoxy compound (Ep-5) had an epoxy equivalent weight of 2320 g/eq. The epoxy resin was found to contain the target epoxy compound (Ep-5) because the mass spectrum showed a peak of M+ = 883, corresponding to the theoretical structure of m = 1, q = 1, p1 = 0, and p2 = 0 in the following structural formula (Ep-5). The obtained epoxy compound (Ep-1) contained a compound with q = 0, and when checked by GPC, the obtained epoxy compound contained 0.7% of the compound with q = 0 in the mixture, and the average value of the repeating unit q was 6.3.

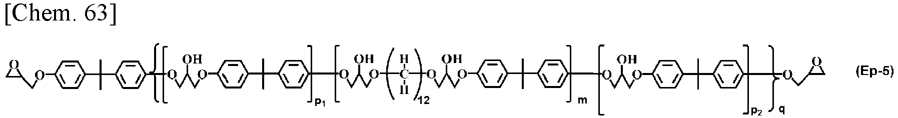

### Synthesis Example 13

The same reaction as in Synthesis Example 4 was carried out except that 388 g (hydroxyl equivalent weight of 388 g/eq) of the hydroxy compound (Ph-1) in Synthesis Example 4 was changed to 1802 g (hydroxyl equivalent weight of 1802 g/eq) of the hydroxy compound (Ph-5), to obtain 1851 g of an epoxy compound (Ep-6). The obtained epoxy compound (Ep-6) had an epoxy equivalent weight of 1895 g/eq. The epoxy compound was found to contain the target epoxy compound (Ep-6) because the mass spectrum showed a peak of M+ = 1336, corresponding to the theoretical structure of m = 1, n2 = 11, q = 1, p1 = 0, and p2 = 0 in the following structural formula (Ep-6). The obtained epoxy compound (Ep-6) contained a compound with q = 0, and when checked by GPC, the obtained epoxy compound contained 2.8% of the compound with q = 0 in the mixture, and the average value of the repeating unit q was 3.1.

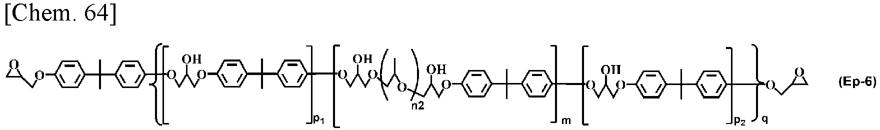

### Comparative Synthesis Example 1

Into a flask equipped with a thermometer, a stirrer, and a condenser, 31.8 g of 1,6'-bismaleimide-(2,2,4-trimethyl)hexane (BMI-THM, manufactured by Daiwakasei Industry Co., Ltd.), 19.6 g of furfuryl alcohol (manufactured by Tokyo Chemical Industry Co., Ltd.), and 50 g of toluene were charged, and after replacement with nitrogen, the mixture was reacted at 60°C for 12 hours. Thereafter, toluene was distilled off under reduced pressure to obtain 49g of an alcoholic hydroxyl group-containing compound (OH-1). The phenolic hydroxyl group-containing compound was found to contain the target alcoholic hydroxyl group-containing compound (OH-1) because the mass spectrum showed a peak of M+ = 514. In addition, the hydroxyl equivalent weight calculated from ¹H-NMR was 267 g/eq.

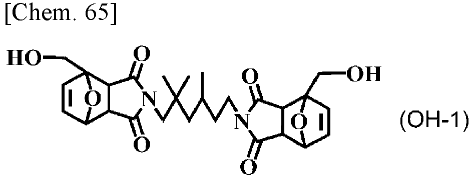

### Production of Composition and Cured Product

Each compound was used according to the formulations (the numerals in the tables are based on mass) shown in Tables 1 to 4 and mixed uniformly in a mixer ("Thinner Mixer ARV-200" manufactured by Thinky Corporation) to obtain a curable resin composition. This curable resin composition was sandwiched between mirror-finished aluminum plates ("JIS H 4000 A1050P" manufactured by Engineering Test Services Co., Ltd.) using a silicone tube as a spacer, and cured by heating under predetermined conditions to obtain a cured product having a thickness of 0.7 mm.

### <Tensile Elongation>

The obtained cured product was punched out into a dumbbell shape (JIS K 7161-2-1BA) using a punching blade, and this was used as a test piece. A tensile test of this test piece was performed in accordance with JIS K 7162-2 by using a tensile testing machine (Autograph AG-IS manufactured by Shimadzu Corporation), and the elongation rate at breakpoint was evaluated at 23°C in the measurement environment (test speed: 2 mm/min).

### <Remolding Test>

The produced cured product was frozen and pulverized. 0.07 g of the pulverized cured product was placed in a 10 mm square, 0.5 mm thick mold and vacuum pressed under conditions of 150°C/4 hours/10 MPa, and then aged at 60°C/24 hours. The appearance of the obtained cured product was visually observed. The criteria for determination are as follows.
A: Seams disappeared and cured product was integrated.
B: Seams are partially visible, but the cured product is integrated.
C: The shape was solid and fell apart when light force was applied.

### <Repair Test>

The prepared cured product was cut with a razor, and the resulting fractured surfaces were brought into contact with each other, and then aged in a dryer at 130°C for 30 minutes and at 60°C for 24 hours. After the cured product was taken out of the dryer, the presence or absence of bonding between the cross sections of the cured product was visually checked. The criteria for determination are as follows.
A: Bonding is observed and the bonded portion is not dissociated when the cured product is bent by 90°.
B: Bonding is observed and the bonded portion is dissociated when the cured product is bent.
C: No bonding was observed.

### <Disassembly Test>

Each compound was used according to the formulations (the numerals in the tables are based on mass) shown in Tables 1 to 4 and mixed uniformly in a mixer ("Thinner Mixer ARV-200" manufactured by Thinky Corporation) to obtain a curable resin composition. This resin composition was applied to one of two cold-rolled steel plates ("SPCC-SD" manufactured by TP Giken Co., Ltd., 1.0 mm x 25 mm x 100 mm), glass beads ("J-80" manufactured by Potters Ballotini Co., Ltd.) were added as a spacer, and the other SPCC-SD was attached (adhesive area: 25 mm x 12.5 mm). This was thermally cured at temperatures according to Tables 1 to 3 to obtain a test piece. The test piece was suspended in a dryer at 120°C, and with a weight of 500 g, a load was applied to one side of the base material. The test piece was allowed to stand in this state for 30 minutes to evaluate the adhesive condition of the base material. The criteria for determination are as follows.
A: The bonded portion shifted and the adhesive base material on the side where the load was applied fell.
B: The bonded portion shifted.
C: No change occurred in the base material.

**[Table 1]**

| | | Example 23 | Example 24 | Example 25 | Example 26 | Example 27 | Example 28 | Example 29 | Example 30 | Comparative Example 1 | Comparative Example 2 | Comparative Example 3 |
|---|---|---|---|---|---|---|---|---|---|---|---|---|
| Epoxy Resin | E-8508 | 50 | 50 | 50 | 50 | 50 | 50 | 50 | 50 | 100 | 50 | 50 |
| Hydroxyl Group-containing Compound | D-1 | 50 | | | | | | | | | | |
| | D-2 | | 50 | | | | | | | | | |
| | D-3 | | | 50 | | | | | | | | |
| | D-4 | | | | 50 | | | | | | | |
| | D-5 | | | | | 50 | | | | | | |
| | D-6 | | | | | | 50 | | | | | |
| | D-7 | | | | | | | 50 | | | | |
| | D-8 | | | | | | | | 50 | | | |
| | OH-1 | | | | | | | | | | 50 | |
| | Ph-1 | | | | | | | | | | | 50 |
| Curing Agent | DICY | 2.8 | 2.8 | 2.8 | 2.8 | 2.8 | 2.8 | 2.8 | 2.8 | 5.6 | 2.8 | 2.8 |
| Curing Accelerator | DCMU | 0.85 | 0.85 | 0.85 | 0.85 | 0.85 | 0.85 | 0.85 | 0.85 | 0.85 | 0.85 | 0.85 |
| Reversible Bond Concentration | mmol /*g* | 0.22 | 0.17 | 0.17 | 0.34 | 0.23 | 0.36 | 0.25 | 0.34 | 0.00 | 1.88 | 0.00 |
| Curing Conditions | | 170°C/60min + 60°C/24h | | | | | | | | | | |
| Tensile Elongation (%) | | 32 | 40 | 38 | 25 | 33 | 11 | 29 | 21 | <1 | <1 | 4 |
| Remolding Test | | B | A | A | A | A | B | A | B | C | - | C |
| Repair Rate | | A | A | A | A | A | B | A | A | C | - | C |
| Disassembly | | A | A | A | B | A | B | A | B | C | - | C |

**[Table 2]**

| | | Example 31 | Example 32 | Example 33 | Example 34 | Example 35 | Example 36 | Example 37 | Example 38 | Comparative Example 4 |
|---|---|---|---|---|---|---|---|---|---|---|
| Epoxy Resin | E-850S | 50 | 50 | 50 | 50 | 50 | 50 | 50 | 50 | 50 |
| Conjugated Diene Intermediate | F-1 | 38.6 | | | | | | | | 38.6 |
| | F-3 | | 41.3 | | | | | | | |
| | F-5 | | | 41.2 | | | | | | |
| | F-7 | | | | 32.1 | | | | | |
| | F-9 | | | | | 38.0 | | | | |
| | F-11 | | | | | | 31.2 | | | |
| Parent Diene Intermediate | M-1 | | | | | | | 41.7 | 38.5 | |
| | M-2 | | | | | | | | | |
| Bismaleimide Compound | BMI-TMH | 7.1 | 5.4 | 5.5 | 11.3 | 7.5 | 11.8 | | | 7.1 |
| Phenolic Hydroxyl Group-containing Maleimide Compound | Ph-M-1 | 4.2 | 3.2 | 3.3 | 6.7 | 4.5 | 7.0 | | | |
| Phenolic Hydroxyl Group-containing Furan Compound | Ph-F-1 | | | | | | | 8.3 | 11.5 | |
| Maleimide Compound | PMI | | | | | | | | | 4.2 |
| Curing Agent | DICY | 2.8 | 2.8 | 2.8 | 2.8 | 2.8 | 2.8 | 2.8 | 2.8 | 2.8 |
| Curing Accelerator | DCMU | 0.85 | 0.85 | 0.85 | 0.85 | 0.85 | 0.85 | 0.85 | 0.85 | 0.85 |
| Reversible Bond Concentration | mmol/g | 0.22 | 0.17 | 0.17 | 0.35 | 0.24 | 0.37 | 0.26 | 0.36 | 0.20 |
| Curing Conditions | | 170°C/60min + 60°C/24h | | | | | | | | |
| Tensile Elongation (%) | | 30 | 35 | 33 | 20 | 30 | 12 | 25 | 18 | - |
| Remolding Test | | B | A | A | A | A | B | A | B | - |
| Repair Rate | | A | A | A | A | A | B | A | A | - |
| Disassembly | | A | A | A | B | A | B | A | B | - |

**[Table 3]**

| | | Example 39 | Example 40 | Example 41 | Example 42 | Example 43 | Example 44 | Example 45 | Example 46 | Comparative Example 5 |
|---|---|---|---|---|---|---|---|---|---|---|
| Epoxy Resin | E-850S | 50 | 50 | 50 | 50 | 50 | 50 | 50 | 50 | 50 |
| Conjugated Diene Intermediate | F-1 | 38.6 | | | | | | | | 38.6 |
| | F-3 | | 41.3 | | | | | | | |
| | F-5 | | | 41.2 | | | | | | |
| | F-7 | | | | 32.1 | | | | | |
| | F-9 | | | | | 38.0 | | | | |
| | F-11 | | | | | | 31.2 | | | |
| Parent Diene Intermediate | M-1 | | | | | | | 41.7 | 38.5 | |
| | M-2 | | | | | | | | | |
| Bismaleimide Compound | BMI-TMH | 7.1 | 5.4 | 5.5 | 11.3 | 7.5 | 11.8 | | | 7.1 |
| Phenolic Hydroxyl Group-containing Maleimide Compound | Ph-M-1 | 4.2 | 3.2 | 3.3 | 6.7 | 4.5 | 7.0 | | | |
| Phenolic Hydroxyl Group-containing Furan Compound | Ph-F-1 | | | | | | | 8.3 | 11.5 | |
| Maleimide Compound | PMI | | | | | | | | | 4.2 |
| Curing Agent | DTA | 5.5 | 5.5 | 5.5 | 5.5 | 5.5 | 5.5 | 5.5 | 5.5 | 5.5 |
| Reversible Bond Concentration | mmol/g | 0.21 | 0.16 | 0.16 | 0.34 | 0.22 | 0.35 | 0.24 | 0.34 | 0.20 |
| Curing Conditions | | 60°C/24h | | | | | | | | |
| Tensile Elongation (%) | | 29 | 35 | 32 | 18 | 29 | 11 | 23 | 16 | |
| Remolding Test | | B | A | A | A | A | B | A | B | |
| Repair Rate | | A | A | A | A | A | B | A | A | |
| Disassembly | | A | A | A | B | A | B | A | B | |

**[Table 4]**

| | | Example 47 | Example 48 | Example 49 | Example 50 | Example 51 | Example 52 |
|---|---|---|---|---|---|---|---|
| Epoxy Resin | E-850S | 40 | 30 | 20 | 40 | 30 | 20 |
| | Ep-4 | 10 | 20 | 30 | | | |
| | Ep-5 | | | | 10 | 20 | 30 |
| Hydroxyl Group-containing Compound | D-1 | 50 | 50 | 50 | | | |
| | D-3 | | | | 50 | 50 | 50 |
| Curing Agent | DICY | 2.3 | 1.8 | 1.3 | 2.3 | 1.8 | 1.3 |
| Curing Accelerator | DCMU | 0.85 | 0.85 | 0.85 | 0.85 | 0.85 | 0.85 |
| Reversible Bond Concentration | mmol/g | 0.22 | 0.22 | 0.22 | 0.17 | 0.17 | 0.17 |
| Curing Conditions | | 170°C/60min + 60°C/24h | | | | | |
| Tensile Elongation (%) | | 35 | 37 | 40 | 40 | 42 | 45 |
| Remolding Test | | B | B | A | A | A | A |
| Repair Rate | | A | A | A | A | A | A |
| Disassembly | | A | A | A | A | A | A |

Each of the formulations listed in the tables is as follows.
E-850S: bisphenol A type liquid epoxy resin (manufactured by DIC Corporation, epoxy equivalent weight of 188 g/eq)
BMI-TMH: 1,6'-bismaleimide-(2,2,4-trimethyl)hexane
PMI: phenylmaleimide (manufactured by Kanto Chemical Co., Inc.)
DICY: dicyandiamide ("DICY7" manufactured by Mitsubishi Chemical Corporation)
DCMU: 3-(3,4-dichlorophenyl)-1,1-dimethylurea) ("B-605-IM" manufactured by DIC Corporation) DTA: diethylenetriamine (manufactured by Kanto Chemical Co., Inc.)

In Comparative Example 2, curing by heating could not be performed. This is believed to be because the hydroxyl group-containing compound OH-1 used is an alcoholic hydroxyl group, which has low reactivity during heat curing and no crosslinking was formed with the epoxy resin used in combination.

In Comparative Example 4 and Comparative Example 5, curing by heating could not be performed. It is believed that because the reversible bond-containing compound consisting of "conjugated diene intermediate + bismaleimide compound + maleimide compound" does not have a phenolic hydroxyl group, no crosslinking was formed with the epoxy resin used in combination.

## Claims

1. A phenolic hydroxyl group-containing compound in which a structural unit A having one or more phenolic hydroxyl groups and a structural unit B different from the structural unit A are linked in A-B-A, wherein
the structural unit A and the structural unit B are bonded by a reversible bond by a Diels-Alder reaction between a furan structure and a maleimide structure.

2. The phenolic hydroxyl group-containing compound according to Claim 1, wherein
the structural unit B has an alkylene chain or an alkylene ether chain.

3. The phenolic hydroxyl group-containing compound according to Claim 2, wherein
the alkylene chain has 4 to 16 carbon atoms.

4. The hydroxyl group-containing compound according to Claim 1, wherein
the structural unit B further has a reversible bond by the Diels-Alder reaction between the furan structure and the maleimide structure.

5. A phenolic hydroxyl group-containing compound represented by the following general formula: 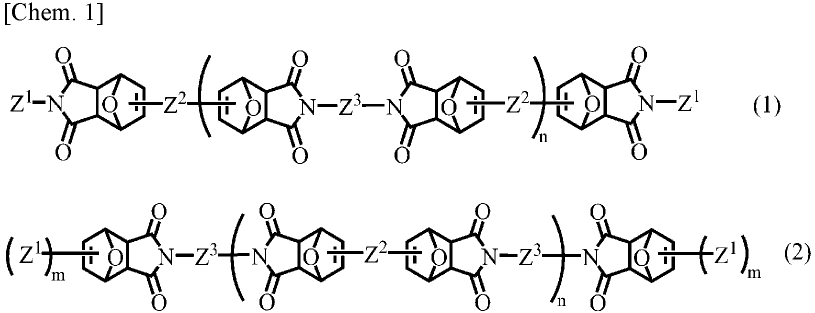
[the furan-derived structure in the formulas (1) and (2) may have a halogen atom, an alkoxy group, an aralkyloxy group, an aryloxy group, a nitro group, an amide group, an alkyloxycarbonyl group, an aryloxycarbonyl group, a cyano group, an alkyl group, a cycloalkyl group, an aralkyl group, or an aryl group as a substituent,
in the formulas,
m is an integer of 1 to 4,
n is an average value of repetition number of 0 to 10, and
Z¹ is any of structures represented by the following formula (3), Z² is any of structures represented by the following formula (4), and Z³ is any of structures represented by the following formula (5), and each of the multiple structures present in one molecule may be identical or different from each other, 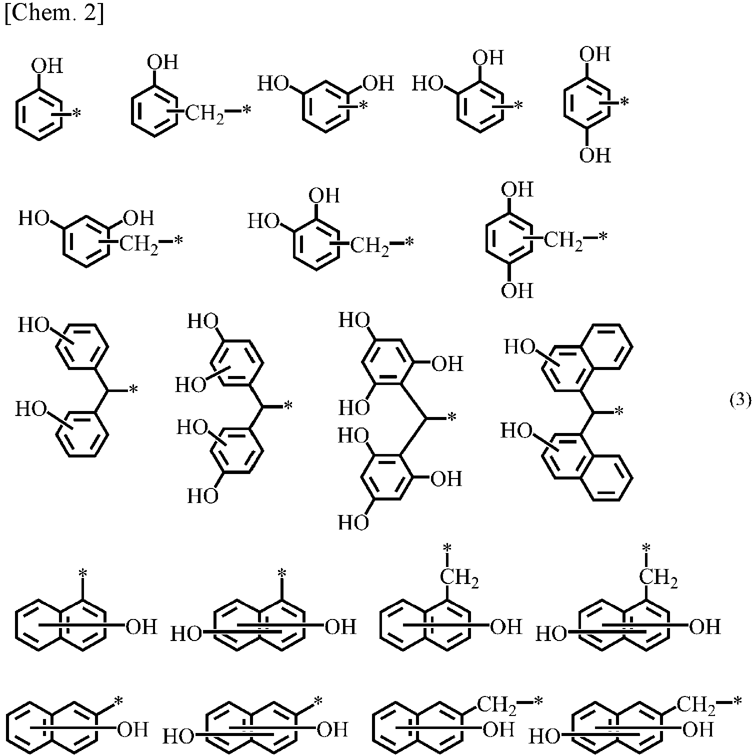
[an aromatic ring in the formula (3) may be substituted or unsubstituted, and * denotes a binding point, a hydroxyl group on a naphthalene ring in the formula indicates that the hydroxyl group may be bonded at any point], 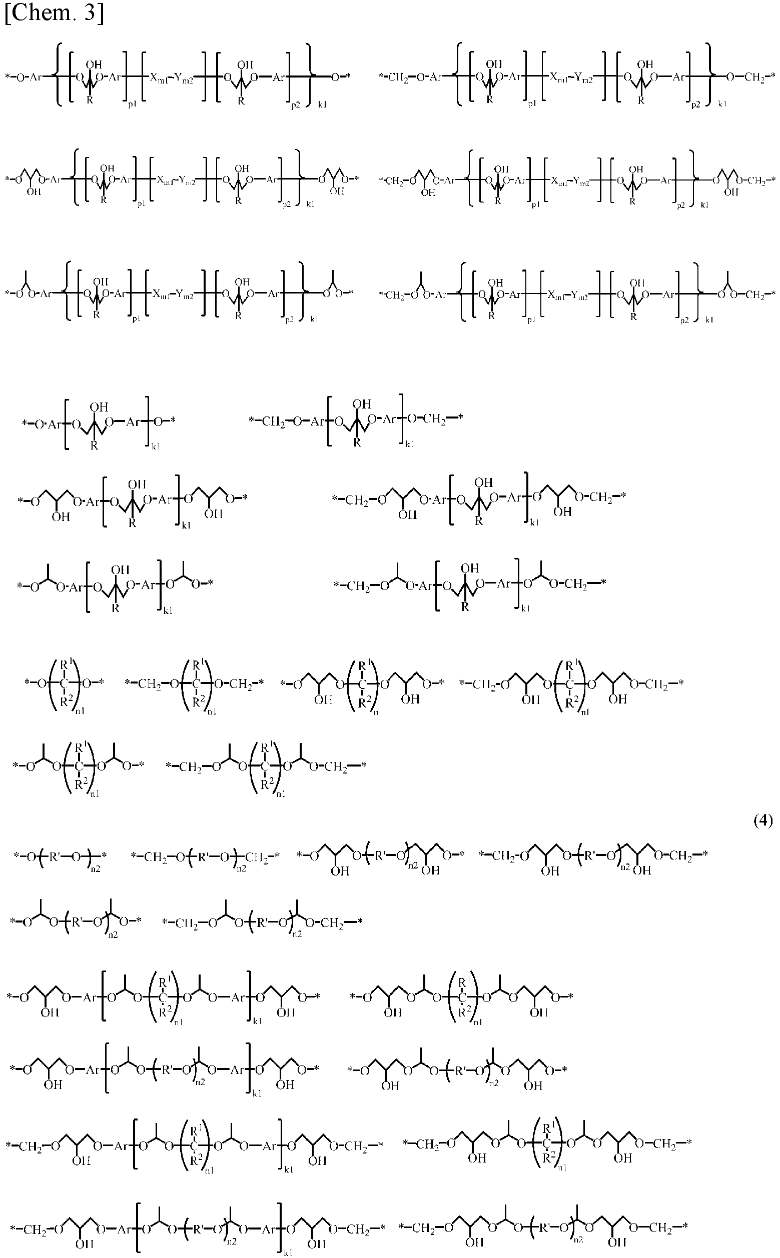
[in the formula (4),
Ar's are each independently a structure containing an unsubstituted aromatic ring or an aromatic ring having a substituent,
R¹ and R² are each independently a hydrogen atom, a methyl group or an ethyl group,
R is a hydrogen atom or a methyl group,
R' is a divalent hydrocarbon group having 2 to 12 carbon atoms,
n1 is an integer of 2 to 16, and n2 is an average value of repeating units of 2 to 30, k1 is an average value of repetition number, in a range of 0.5 to 10,
p1 and p2 are each independently 0 to 5, and
X is a structural unit represented by the following formula (4-1) and Y is a structural unit represented by the following formula (4-2), 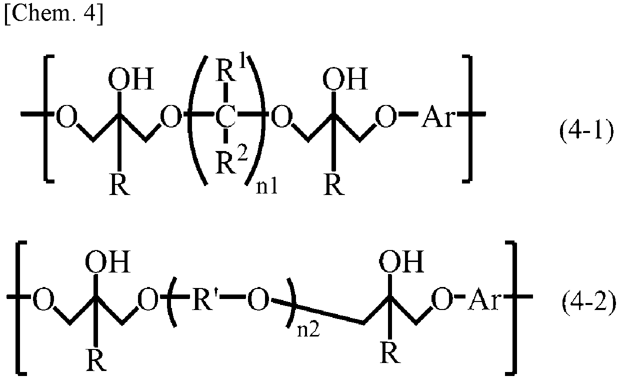
[in the formulas (4-1) and (4-2), Ar, R, R¹, R², R', n1, and n2 are the same as above],
m1 and m2 are an average value of repetition number, each independently 0 to 25, and m1 + m2 ≥ 1,
provided that a bond between the structural unit X represented by the formula (4-1) and the structural unit Y represented by the formula (4-2) may be random or blocked, and total numbers of respective structural units X and Y present in one molecule are m1 and m2, respectively], 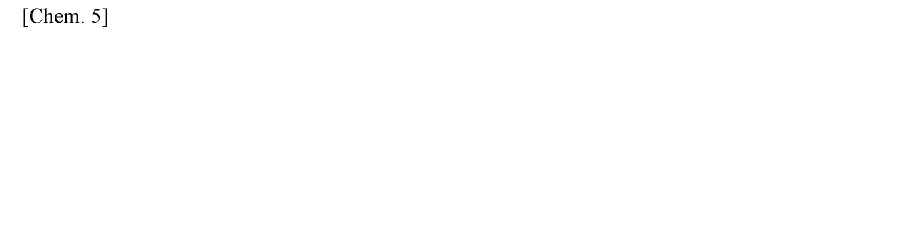 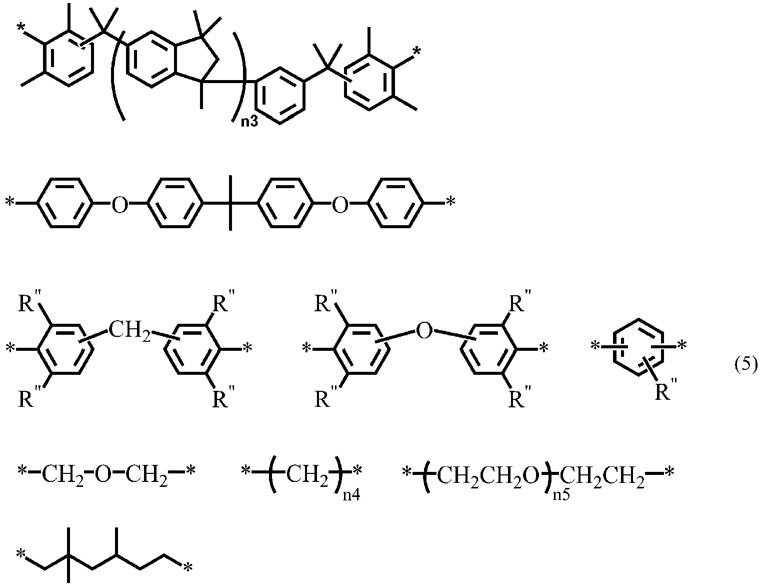
[in the formula (5), n3 and n5 are an average value of repetition number and are 0.5 to 10, respectively, n4 is an integer of 1 to 16, and R"'s are each independently a hydrogen atom, a methyl group or an ethyl group].

6. A curable resin composition comprising, as essential components:
the phenolic hydroxyl group-containing compound according to any one of Claims 1 to 5; and
a compound (I) having reactivity with a phenolic hydroxyl group-containing compound.

7. The curable resin composition according to Claim 6, wherein
a concentration of the reversible bond in the phenolic hydroxyl group-containing compound to a total mass of curable components in the curable resin composition is 0.10 mmol/g or more.

8. The curable resin composition according to Claim 6, wherein
the compound (I) having reactivity with the phenolic hydroxyl group-containing compound is an epoxy resin.

9. The curable resin composition according to Claim 8, further comprising:
a curing agent for an epoxy resin other than the phenolic hydroxyl group-containing compound.

10. The curable resin composition according to Claim 8, wherein
the epoxy resin is represented by the following formula (6) and has an epoxy equivalent weight of 500 to 10000 g/eq: 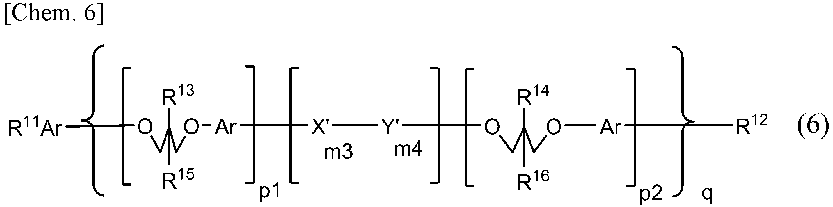
wherein Ar's are each independently a structure containing an unsubstituted aromatic ring or an aromatic ring having a substituent,
X ' is a structural unit represented by general formula (6-1) shown below, and Y' is a structural unit represented by general formula (6-2) shown below, 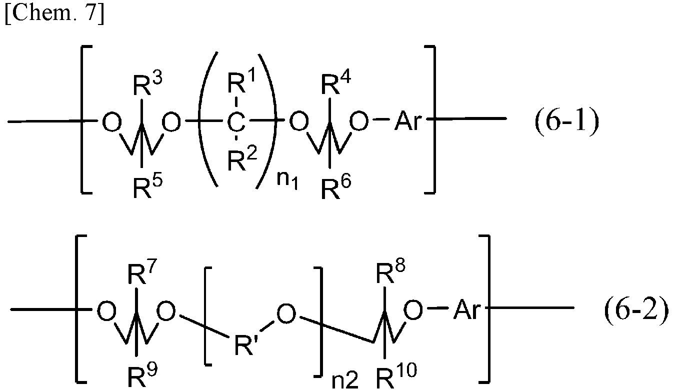
[in the formulas (6-1) and (6-2), Ar is the same as above,
R¹ and R² are each independently a hydrogen atom, a methyl group or an ethyl group,
R' is a divalent hydrocarbon group having 2 to 12 carbon atoms,
R³, R⁴, R⁷, and R⁸ are each independently a hydroxyl group, a glycidyl ether group, or a 2-methylglycidyl ether group,
R⁵, R⁶, R⁹, and R¹⁰ are each independently a hydrogen atom or a methyl group,
n1 is an integer of 4 to 16, and
n2 is an average value of repeating units of 2 to 30,]
R¹¹ and R¹² are each independently a glycidyl ether group or a 2-methylglycidyl ether group,
R¹³ and R¹⁴ are each independently a hydroxyl group, a glycidyl ether group or a 2-methylglycidyl ether group,
R¹⁵ and R¹⁶ are hydrogen atoms or methyl groups,
m3, m4, p1, p2, and q are an average value of repetition number,
m3 and m4 are each independently 0 to 25 and m3 + m4 ≥ 1,
p1 and p2 are each independently 0 to 5, and
q is 0.5 to 5,
provided that a bond between X' represented by the general formula (6-2) and Y' represented by the general formula (6-3) may be random or blocked, and total numbers of respective structural units X' and Y' present in one molecule are m3 and m4, respectively].

11. The curable resin composition according to Claim 6, wherein
the curable resin composition is any of a self-repairing composition, an easily disassembled composition, or a composition for a remolding material.

12. A cured product obtained by curing the curable resin composition according to Claim 6.

13. A laminate comprising:
a base material; and
a layer containing the cured product according to Claim 12.

14. A heat-resistant member comprising:
the cured product according to Claim 12.

15. An intermediate of a conjugated diene or a parent diene intermediate represented by the following general formulas (1)' and (2)': 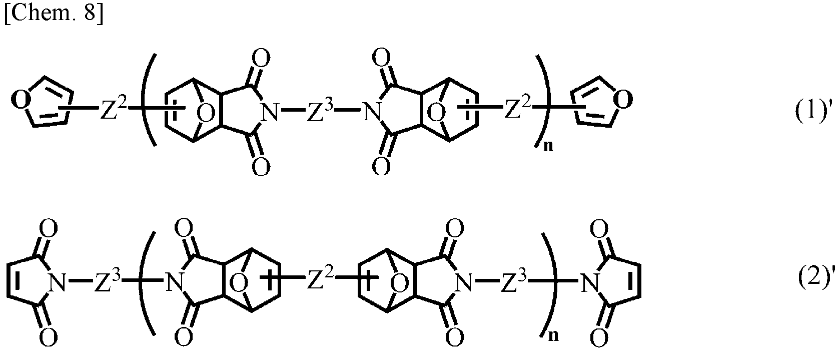 [in the formulas, n, Z², and Z³ are the same as above].

16. A method for producing a phenolic hydroxyl group-containing compound, the method comprising:
synthesizing a phenolic hydroxyl group-containing compound represented by the formulas (1) and (2) in situ during a process of curing thereof together with a compound (I) having reactivity with the phenolic hydroxyl group-containing compound, by using an intermediate of a conjugated diene or a parent diene intermediate represented by the general formulas (1)' and (2)'.

17. A cured product obtained by curing and reacting the formula (1)', a maleimide compound having a phenolic hydroxyl group, and a compound (I) having reactivity with the phenolic hydroxyl group-containing compound as essential raw materials.

18. A cured product obtained by curing and reacting the formula (2)', a furan compound having a phenolic hydroxyl group, and a compound (I) having reactivity with the phenolic hydroxyl group-containing compound as essential raw materials.
